(19) Europäisches Patentamt
European Patent Office
Office européen des brevets

(11) EP 1 616 557 A2

# (12) DEMANDE DE BREVET EUROPEEN

(43) Date de publication:
**18.01.2006 Bulletin 2006/03**

(51) Int Cl.:
*A61K 8/37* (2006.01) *A61K 8/81* (2006.01)
*A61K 8/898* (2006.01) *A61Q 1/06* (2006.01)

(21) Numéro de dépôt: **05300583.1**

(22) Date de dépôt: **13.07.2005**

(84) Etats contractants désignés:
**AT BE BG CH CY CZ DE DK EE ES FI FR GB GR HU IE IS IT LI LT LU LV MC NL PL PT RO SE SI SK TR**
Etats d'extension désignés:
**AL BA HR MK YU**

(30) Priorité: **16.07.2004 FR 0451559**

(71) Demandeur: **L'OREAL**
**75008 Paris (FR)**

(72) Inventeurs:
- **Shimuzu, Momoko**
**Takatsu-ku**
**KANAGAWA-KEN,**
**213-0012 (JP)**
- **Blin, Xavier**
**75015, PARIS (FR)**

(74) Mandataire: **Le Coupanec, Pascale A.M.P. et al**
**Nony & Associés,**
**3 rue de Penthièvre**
**75008 Paris (FR)**

## (54) Composition cosmétique comprenant un polymère de silicone defini et un agent filmogène

(57) La présente invention se rapporte à une composition cosmétique, de maquillage et/ou de soin destinée à être appliquée sur la peau, les lèvres et/ou les phanères, contenant un polymère de silicone particulier et un agent filmogène.

La composition, selon l'invention présente notamment une tenue améliorée, une brillance satisfaisante, voire améliorée, et un confort amélioré.

EP 1 616 557 A2

## Description

La présente invention se rapporte à une composition cosmétique, de maquillage et/ou de soin destinée à être appliquée sur la peau, les lèvres et/ou les phanères, contenant un polymère de silicone particulier et un agent filmogène.
La composition, selon l'invention présente notamment une tenue améliorée, une brillance satisfaisante, voire améliorée, et un confort amélioré.
Les compositions cosmétiques visées par la présente invention sont plus particulièrement des produits de maquillage et/ou de soin destinés à être appliqués sur la peau, les lèvres et/ou les phanères, notamment les rouges à lèvres, les baumes à lèvres, les crayons à lèvres, les fonds de teint liquides ou solides, notamment coulés en stick ou en coupelle, les produits anti-cernes et les produits de coloration de la peau, de tatouages éphémères, les produits de maquillage des yeux comme les eye-liners, en particulier sous forme de crayons, et les mascaras, notamment sous forme de pains ou encore de fards à paupière.
D'une manière générale, les compositions cosmétiques se doivent de conférer un effet esthétique lors de l'application, et de maintenir cet effet esthétique au cours du temps. Elles doivent notamment résister aux différents facteurs extérieurs susceptibles de modifier leur effet esthétique, comme la sueur ou les larmes pour un fond de teint, ou la salive pour un rouge à lèvres.
Ainsi, les compositions cosmétiques, telles que par exemple les rouges à lèvres, ne doivent pas migrer dans les rides ou ridules, ou transférer sur un tissu. Elles doivent également être agréables à appliquer et maintenir une sensation de confort au cours du temps, tout en conservant des propriétés esthétiques satisfaisantes.
Des compositions comprenant des dérivés alkylglycéryléther de silicone ont été décrites dans les demandes JP 6-305933 et JP 7-330547. Des compositions à base huileuse et comprenant des dérivés polyglycérylés de silicone ou fluoroalkylpolyglycérylés de silicone ont été proposés dans les demandes JP 6-157236, JP 9-071504 et JP 10-310504. Des compositions comprenant des dérivés alkylglycérols de silicone ont été également décrites dans le brevet EP 0 475 130 et dans les demandes JP 2-844453 et JP 2-587797. D'autres compositions comprenant des dérivés de silicone hydroxylés par des saccharides, du butylène glycol ou du glycérol, ont été décrites dans les demandes JP 5-186596 et JP 6-145023.
La présente invention a pour objet de proposer des compositions cosmétiques contenant un polymère de silicone polyglycérylé présentant une tenue sur les matières kératiniques améliorée, sans affectation de la brillance, voire avec une amélioration de la brillance, et un confort également susceptible d'être amélioré.
Au sens de la présente invention, on entend par les termes « matières kératiniques » couvrir la peau, les muqueuses, comme les lèvres, les ongles et les fibres kératiniques, à l'image des cils et des cheveux.
Les compositions cosmétiques conformes à la présente invention sont particulièrement avantageuses pour une utilisation sur la peau et les lèvres.
Les inventeurs ont découvert, de manière inattendue, que l'utilisation d'un polymère de silicone et de formule générale (I) :

$$R^1_a\, R^2_b\, R^3_c\, SiO_{(4-a-b-c)/2} \qquad (I)$$

dans laquelle $R^1$ est notamment un radical alkyle, $R^2$ est représenté par la formule générale (III) :

$$Q\text{-}O\text{-}X \qquad (III)$$

dans laquelle Q est un radical hydrocarboné bivalent et X un radical hydrocarboné polyhydroxylé,
et $R^3$ est un groupement organosiloxane de formule générale (IV) :

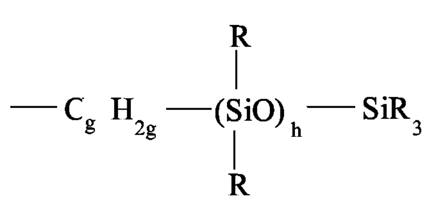
(IV)

dans laquelle chacun des radicaux R représente, indépendamment l'un de l'autre, un radical alkyle,
en association avec au moins un agent filmogène était particulièrement avantageuse pour la formulation de compositions cosmétiques présentant une tenue améliorée sans affectation de la brillance, voire avec une brillance moyenne également améliorée, et éventuellement susceptible de présenter un confort amélioré.
Ainsi, l'invention, selon un premier aspect, concerne une composition cosmétique anhydre comprenant dans

un milieu physiologiquement acceptable au moins un agent filmogène, présent dans un rapport pondéral, par rapport au poids du polymère de silicone de formule générale (I), inférieur à 5: 1, en particulier inférieur à 1:1, et plus particulièrement inférieur ou égal à 1:2, et au moins un polymère de silicone de formule générale (I) :

$$R^1_aR^2_bR^3_c\ SiO_{(4-a-b-c)/2} \qquad (I)$$

dans laquelle :

- a, b et c sont tels que a varie de 1 à 2,5, b et c, indépendamment l'un de l'autre, varient de 0,001 à 1,5,
- $R^1$ identique ou différent, est choisi parmi :
  - les radicaux alkyle en $C_1$ à $C_{30}$, le cas échéant substitués par un ou plusieurs atomes de fluor, groupements amino et/ou carboxy,
  - les radicaux aryle, aralkyle,
  - les radicaux de formule générale (II) :

    $$—C_dH_{2d}\text{-O-}(C_2H_4O)_e(C_3H_6O)_fR^4 \qquad (II)$$

    dans laquelle :

    - $R^4$ est un radical hydrocarboné en $C_1$ à $C_{30}$ ou un radical $R^{5-}$(CO)- avec $R^5$ étant un radical hydrocarboné en $C_1$ à $C_{30}$, et
    - d, e et f sont des entiers tels que d varie de 0 à 15, e et f, indépendamment l'un de l'autre, varient de 0 à 50, et

  - leurs combinaisons,

- $R^2$ est un radical représenté par la formule générale (III) :

  $$\text{-Q-O-X} \qquad (III)$$

  avec :

  - Q étant un radical hydrocarboné bivalent en $C_2$ à $C_{20}$ pouvant inclure au moins une liaison éther et/ou au moins une liaison ester et
  - X étant un radical hydrocarboné polyhydroxylé,

- $R^3$ est un organosiloxane de formule générale (IV) :

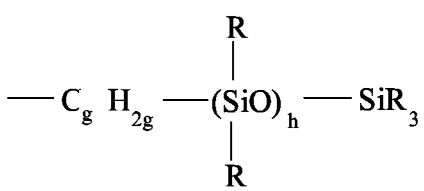

(IV)

  avec :

  - chacun des radicaux R représentant, indépendamment l'un de l'autre, un radical choisi parmi les radicaux alkyle en $C_1$ à $C_{30}$, le cas échéant substitué par un ou plusieurs atomes de fluor ; et les radicaux aryle, aralkyle,
  - g et h étant des entiers tels que g varie de 1 à 5 et h varie de 0 à 500.

Selon un second aspect, la présente invention a pour objet une composition cosmétique anhydre comprenant dans un milieu physiologiquement acceptable au moins au moins un agent filmogène non cristallin, un composé siliconé distinct de l'agent filmogène, et au moins un polymère de silicone de formule générale (I), telle que définie précédemment.

Selon un troisième aspect, la présente invention concerne également une composition cosmétique de maquillage des lèvres comprenant dans un milieu physiologiquement acceptable, au moins un agent filmogène, choisi parmi les agents filmogènes non siliconés et les agents filmogènes siliconés dépourvus de motif acrylique, et au moins

un polymère de silicone de formule générale (I), telle que définie ci-dessus.

Selon un quatrième aspect, la présente invention a également pour objet une composition cosmétique comprenant dans un milieu physiologiquement acceptable au moins un ester hydrocarboné comprenant moins de 40 atomes de carbone, au moins un agent filmogène, choisi parmi les agents filmogènes non siliconés et les agents filmogènes siliconés dépourvus de motif acrylique, et au moins un polymère de silicone de formule générale (I), telle que défmie ci-dessus.

Selon un cinquième aspect, la présente invention concerne encore une composition cosmétique anhydre comprenant dans un milieu physiologiquement acceptable, au moins un agent filmogène et au moins un polymère de silicone de formule générale (I), telle que défmie précédemment, sous réserve que lorsque $R^2$ est un groupement de formule générale (IIIA) :

$$-C_3H_6-O[CH_2CH(OH)CH_2O]_nH \qquad \text{(IIIA)}$$

dans laquelle n est un entier variant de 1 à 5, alors $R^1$ n'est pas un radical alkyl en $C_{12}$.

Selon un sixième aspect, la présente invention concerne une composition cosmétique telle que définie ci-dessus destinée au maquillage et/ou au soin des lèvres, et/ou de la peau, et en particulier concerne un rouge à lèvres.

Selon encore un autre de ses aspects, l'invention a pour objet un support synthétique sur lequel est présent, en tout ou partie de sa surface, au moins une couche d'une composition selon l'invention.

Selon encore un autre de ses aspects la présente invention a également pour objet un procédé de maquillage et/ou de soin des matières kératiniques, et notamment de la peau et/ou des lèvres, comprenant l'application sur celles-ci d'au moins une composition cosmétique conforme à la présente invention.

Selon un autre de ses aspects, l'invention a pour objet l'utilisation d'au moins un polymère de silicone de formule générale (I), telle que définie ci-dessus, en association avec au moins un agent filmogène pour la préparation d'une composition cosmétique présentant une tenue, une brillance, et un confort améliorés.

Par tenue améliorée on entend une tenue à l'eau et/ou une tenue à l'huile améliorée et/ou un transfert diminué et/ou une migration diminuée.

Les compositions cosmétiques selon l'invention peuvent se présenter sous forme de pâte, de liquide, de gel, de crème ou de solide. En particulier, les compositions cosmétiques selon l'invention sont sous forme coulée, et plus particulièrement elles sont sous la forme d'un stick. Elles peuvent être sous la forme d'une émulsion simple huile-dans-eau ou eau-dans-huile, ou multiple, d'un gel anhydre, solide ou souple.

En particulier, elles se présentent sous une forme anhydre.

Par le terme «composition sous forme coulée » on entend, au sens de la présente invention, une composition solide ou semi-solide obtenue à l'issue du refroidissement d'une composition introduite à l'état de fusion, dans un moule. Les compositions peuvent être coulées sous forme d'un stick ou d'un bâton, ou dans une coupelle.

Selon un mode particulier de réalisation, la composition cosmétique selon l'invention est sous forme coulée, c'est-à-dire solide ou semi-solide et plus particulièrement sous la forme d'un stick.

Pour déterminer la dureté d'une composition cosmétique conforme à l'invention, on prépare un stick de ladite composition ayant une section circulaire de 12,7 mm de diamètre. Le stick est coulé puis conservé à une température de 20 °C, 24 heures avant de faire la mesure.

La dureté peut être mesurée par la méthode dite « du fil à couper le beurre », qui consiste à couper transversalement le stick à l'aide d'un fil rigide de diamètre 250 μm en tungstène en faisant avancer le fil relativement au stick à une vitesse de 100 mm/min. La dureté correspond à la force maximale de cisaillement exercée par le fil sur le stick à 20 °C, cette force étant mesurée au moyen d'un dynamomètre DFGS2 commercialisé par la société INDELCO-CHATILLON. La dureté est exprimée en grammes.

Selon cette méthode la dureté d'une composition cosmétique conforme à l'invention présentée sous la forme d'un stick varie notamment de 50 à 300 g, en particulier de 70 à 250 g et plus particulièrement de 100 à 230 g.

Un des avantages des compositions cosmétiques selon la présente invention est de présenter une tenue de la couleur améliorée, se traduisant notamment par une migration ou un transfert de la couleur réduit(e) et/ou une tenue de la couleur à l'eau améliorée et/ou une tenue de la couleur à l'huile améliorée, et/ou une migration réduite lors du tracé du maquillage.

Un autre avantage des compositions cosmétiques selon la présente invention est le maintien d'une sensation confortable, d'une absence de sensation collante tout en présentant une bonne adhésion sur la peau.

Un troisième avantage des compositions cosmétiques selon la présente invention est le maintien de l'effet esthétique, notamment de l'effet de brillance, au cours du temps.

Selon un quatrième avantage, les compositions cosmétiques selon l'invention permettent de conférer une sensation lisse et douce, et de maintenir une bonne hydratation.

Selon un cinquième avantage, les compositions cosmétiques selon la présente invention présentent une bonne tenue face aux facteurs extérieurs susceptibles d'en modifier les propriétés esthétiques, comme la sueur ou un repas,

dans le cas d'un rouge à lèvres.

**POLYMERE DE SILICONE DE FORMULE GENERALE (I)**

Les polymères de silicone, conformes au polymère de silicone de formule générale (I) et utilisables dans les compositions cosmétiques selon la présente invention, sont décrits en détail dans la demande de brevet EP 1 213 316, incorporée dans la présente demande par référence.

Les polymères de silicone de formule générale (I) présentent l'avantage de pouvoir être utilisés comme agent tensioactif et/ou comme base huileuse.

Dans le cadre de la présente invention, les polymères de silicone de formule générale (I) convenant à la mise en oeuvre des compositions conformes à l'invention sont dénués de fonction d'agent de traitement de surface.

Introduits en quantité suffisante, ils présentent l'avantage de conférer une amélioration de la tenue, voire de la brillance et/ou du confort des compositions cosmétiques selon l'invention.

En particulier, les polymères de silicone utilisables dans les compositions cosmétiques selon la présente invention sont représentés par la formule générale (I) suivante :

$$R^1_aR^2_bR^3_c\,SiO_{(4-a-b-c)/2} \qquad (I)$$

dans laquelle :

a) a, b et c sont tels que a varie de 1 à 2,5 ; et b et c, indépendamment l'un de l'autre, varient de 0,001 à 1,5.

b) $R^1$, identique ou différent, est choisi parmi :

- les radicaux alkyle en $C_1$ à $C_{30}$, le cas échéant substitués par un ou plusieurs atomes de fluor, groupements amino et/ou carboxy,
- les radicaux aryle, aralkyle,
- les radicaux de formule générale (II) :

$$-C_dH_{2d}-O-(C_2H_4O)_e(C_3H_6O)_fR^4 \qquad (II)$$

dans laquelle :

- $R^4$ est un radical hydrocarboné en $C_1$ à $C_{30}$, ou un radical $R^{5}$-(CO)- avec $R^5$ étant un radical hydrocarboné en $C_1$ à $C_{30}$, et
- d, e et f sont des entiers tels que d varie de 0 à 15, e et f, indépendamment l'un de l'autre, varient de 0 à 50, et

- leurs combinaisons.

c) $R^2$ est représenté par la formule générale suivante (III) :

$$-Q-O-X \qquad (III)$$

dans laquelle :

- Q est un radical hydrocarboné bivalent en $C_2$ à $C_{20}$, pouvant inclure au moins une liaison éther et/ou au moins une liaison ester, et
- X est un radical hydrocarboné polyhydroxylé.

d) $R^3$ est un groupement organosiloxane de formule générale (IV) :

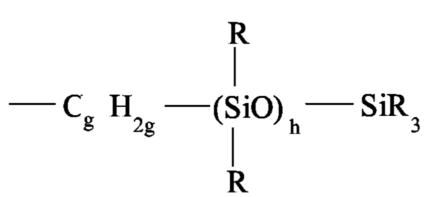

(IV)

avec :

- chacun des radicaux R représentant, indépendamment l'un de l'autre, un radical choisi parmi les radicaux alkyle en $C_1$ à $C_{30}$, le cas échéant substitué par un ou plusieurs atomes de fluor ; et les radicaux aryle et aralkyle,
- g et h étant des entiers tels que g varie de 1 à 5 et h varie de 0 à 500.

Lorsque les radicaux R représentent un radical choisi parmi les radicaux alkyle en $C_1$ à $C_{30}$, le cas échéant substitués par un ou plusieurs atomes de fluor, les radicaux aryle et les radicaux aralkyle, ils ont la même signification que le radical $R^1$ tel que défini précédemment.
Il est à noter que les radicaux $R^1$, $R^2$ et $R^3$ des polymères de silicone de formule générale (I), telle que définie ci-dessus, sont distribués de manière aléatoire ou statistique, c'est-à-dire qu'ils apparaissent dans la structure du polymère sans ordre déterminé. De même, $R^1$, $R^2$, et $R^3$ peuvent figure respectivement des radicaux de nature différente dans un composé de formule générale (I).
Selon un mode de réalisation particulier,
dans a) :

- de manière plus particulière, a varie de 1,2 à 2,3. Et, en particulier, b et c, indépendamment l'un de l'autre, varient de 0,05 à 1.

dans b) :

- lorsque $R^1$ est un radical alkyle, il peut être un radical alkyle en $C_1$ à $C_{30}$, en particulier un radical alkyle en $C_1$ à $C_{25}$, plus particulièrement un radical alkyle en $C_1$ à $C_{20}$, en particulier un radical alkyle en $C_1$ à $C_{10}$, et notamment un radical alkyle en $C_1$ à $C_6$, et en particulier un radical alkyle en $C_1$ à $C_4$. Plus particulièrement, il peut être un radical méthyle, éthyle, n- ou iso-propyle, n- ou iso- ou tert- butyle, pentyle, hexyle, heptyle, octyle, nonyle, décyle, undécyle ou lauryle. Il peut également être un radical cycloalkyle tel qu'un cyclopropyle, un cyclobutyle, cyclopentyle ou un cyclohexyle. Il peut également être un radical alkyle monoinsaturé ou polyinsaturé, linéaire ou ramifié. Il peut également être un radical alkyle substitué par un ou plusieurs atomes de fluor, tel que le trifluoropropyle ou l'heptadécafluorodécyle. Il peut également être un radical alkyle substitué par un ou plusieurs groupements amino, tels que l'amino-2 éthyle, l'amino-3 propyle, le (amino-2 éthyle)amino-3 propyle. Il peut également être un groupe alkyle substitué par un ou plusieurs groupements carboxy, tel que le carboxy-3 propyle.
- $R^1$ peut également être un radical aryle ou aralkyle tel que le radical phényle, le radical tolyle, le radical benzyle et le radical phénéthyle.
- $R^1$ peut également être un groupe organique représenté par la formule générale (II) :

$$— C_dH_{2d}\text{-O-}(C_2H_4O)_e(C_3H_6O)_fR^4 \qquad (II)$$

Selon un mode de réalisation particulier, $R^1$ peut être un radical hydroxylé ou un radical issu de la réaction d'addition d'un alkényléther, saturé ou insaturé, ramifié ou linéaire, dans lequel d = 0 et donc de formule :

$$\text{-O-}(C_2H_4O)_e(C_3H_6O)_fR^4$$

Dans ce cas, lorsque e et f égalent zéro, alors $R^1$ est un groupe alcoxy ayant de 4 à 30 atomes de carbone, par exemple un radical alcoxy inférieur en $C_4$ à $C_{10}$, tel que le butoxy ou le pentoxy ou un radical alcoxy supérieur, en $C_{11}$ à $C_{30}$, tel que l'oléoxy, le stéaroxy, à savoir par exemple, l'alcool cétylique, l'alcool oléique et l'alcool stéarylique, ou un radical issu d'un acide ou d'un acide gras, tel que l'acide acétique, l'acide lactique, l'acide butyrique, l'acide oléique, l'acide stéarique et l'acide béhénique.
Lorsque e et f sont supérieurs à 1, alors $R^1$ est un radical hydroxyle provenant de la réaction d'addition d'un alkylène oxyde.
Lorsque e et f égalent zéro, il est particulièrement avantageux que d soit égal à 3, 5 ou 11. Dans ce cas, $R^1$, selon la nature du substituant $R^4$, est un radical allyléther, penthényléther ou undécényléther, ou un radical allylstéaryléther, penthénylbéhényléther, ou undécényloléyléther.
Lorsque e ou f sont différents de zéro, un radical alcoxy et un radical ester sont présents *via* un groupement polyoxyalkylène.
Quelque soit e et f, il est particulièrement avantageux que d soit compris dans l'intervalle variant de 3 à 5.
Selon un mode de réalisation, le radical $R^1$ peut être l'un quelconque des radicaux précédemment définis, ou une combinaison de deux ou plusieurs de ces radicaux.

De manière avantageuse, $R^1$ est un radical alkyle choisi parmi le radical méthyle, le radical lauryle, et leurs combinaisons.

Par ailleurs, lorsque $R^1$ représente dans la même formule générale (I) deux ou plusieurs radicaux, par exemple un radical méthyle et un radical lauryle, ces réadicaux apparaissent dans la structure de manière aléatoire, et avec une fréquence qui leur est spécifique.

De manière particulière, au moins 50 % des radicaux $R^1$, en particulier au moins 70 % des radicaux $R^1$, et plus particulièrement 100 % des radicaux $R^1$ sont des radicaux méthyle.
dans c) :

- Q peut être en particulier un radical hydrocarboné bivalent choisi parmi : $-(CH_2)_2-$,$-(CH_2)_3-$, $-CH_2CH(CH_3)-CH_2$, $-(CH_2)_4-$, $-(CH_2)_5-$, $-(CH_2)_6-$, $- (CH_2)_7-$, $-(CH_2)_8-$, $-(CH_2)_9-$, $-(CH_2)_{10}-$, $-(CH_2)_{11}-$, $-(CH_2)_2-CH(CH_2CH_2CH_3)-$, $-CH_2-CH(CH_2CH_3)-$, $-(CH_2)_3-O-(CH_2)_2-$, $-(CH_2)_3-O-(CH_2)_2-O-(CH_2)_2-$, $-(CH_2)_3-O-CH_2CH(CH_3)-$ et $-CH_2-CH(CH_3)-COO(CH_2)_2-$.

De manière avantageuse, Q est un radical bivalent choisi parmi $-(CH_2)_2-$ et $- (CH_2)_3-$.

- X peut être de manière particulière un radical hydrocarboné polyhydroxylé comprenant au moins deux résidus hydroxyles, et en particulier un groupement hydrocarboné choisi parmi les dérivés glycérylés et les dérivés osidiques.

Les résidus glycérols peuvent être des composés ayant les formules suivantes, dans lesquelles Q a la même signification que dans la formule générale (III), et s et t sont des entiers compris dans l'intervalle variant de 1 à 20, en particulier de 1 à 15, en particulier de 1 à 10, et plus particulièrement de 1 à 5.

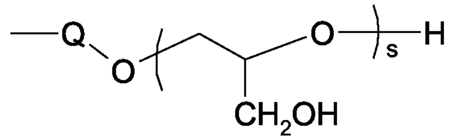

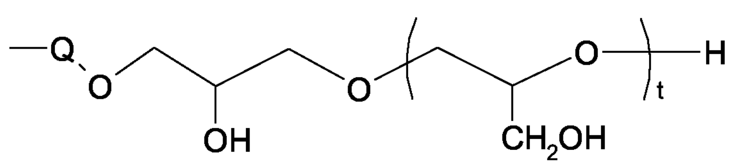

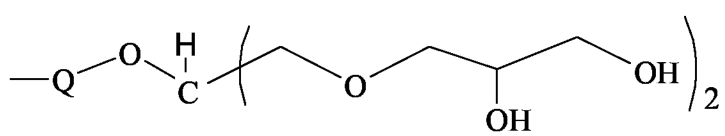

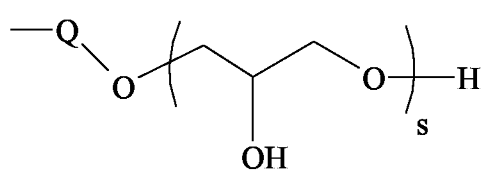

Dans les formules précédentes, un ou plusieurs groupes hydroxyles peuvent être remplacés par des groupes alcoxy ou des groupements esters.

Les radicaux osidiques utilisables dans la formule générale (III) peuvent être de type monosaccharidique, tel que les groupements glycosyle, mannosyle, galactosyle, ribosyle, arabinosyle, xylosyle ou fructosyle, de type oligosaccharidique tel que le maltosyle, le cellobiosyle, le lactosyle ou le maltotriosyle, ou de type polysaccharidique tel que la cellulose ou l'amidon.

De manière particulière, les groupements osidiques sont de type monosaccharidique ou oligosaccharidique.

dans d) :

- chacun des radicaux R peut représenter en particulier, indépendamment l'un de l'autre, un radical choisi parmi les radicaux alkyle en de $C_1$ à $C_{20}$, plus particulièrement en $C_1$ à $C_{10}$, en particulier en $C_1$ à $C_6$, le cas échéant substitué par un ou plusieurs atomes de fluor. Lorsque les radicaux R représentent un radical choisi parmi les radicaux alkyle tels que définis précédemment, le cas échéant substitués par un ou plusieurs atomes de fluor, ils ont la même signification que le radical $R^1$ tel que défini précédemment.
- g, selon un mode particulier de réalisation, est égal à 2
- h, selon un mode particulier de réalisation, est compris dans l'intervalle variant de 1 à 50.

Toutefois, selon certains des aspects particuliers de la présente invention, tels que définis précédemment, et notamment selon un cinquième aspect, le polymère de silicone de formule générale (I) est tel que lorsque le radical $R^2$ est représenté par la formule générale (IIIA) :

$$-C_3H_6O[CH_2CH(OH)CH_2O]_nH \qquad (IIIA)$$

dans laquelle n varie de 1 à 5, alors le radical $R^1$ est différent d'un radical alkyle en $C_{12}$.

Selon un mode particulier de réalisation, le polymère de silicone de formule générale (I) convenant à la mise en oeuvre de la présente invention est tel que :

- a varie de 1 à 1,4, b et c, indépendamment l'un de l'autre, varient de 0,02 à 0,03, et
- $R^1$ est un radical alkyle en $C_1$ à $C_{10}$, en particulier en $C_1$ à $C_6$, et plus particulièrement en $C_1$ à $C_4$.
- $R^2$ est représenté par la formule (IIIA) :

$$C_3H_6O[CH_2CH(OH)CH_2O]_n\,H \qquad (IIIA)$$

dans laquelle :

- n varie de 1 à 5, et
- $R^3$ est représenté par la formule (IVA) :

$$-C_2H_4(CH_3)_2SiO[(CH_3)_2SiO]_m\,Si(CH_3)_3 \qquad (IVA)$$

dans laquelle :

- m varie de 3 à 9.

Selon un autre mode particulier de réalisation, le polymère de silicone de formule générale (I), utilisable dans les compositions cosmétiques selon la présente invention, est tel que :

- a varie de 1 à 1,4, b et c, indépendamment l'un de l'autre, varient de 0,02 à 0,04,
- $R^1$ est un radical méthyle,
- $R^2$ est représenté par la formule (IIIA) dans laquelle n varie de 1 et 5, et
- $R^3$ est représenté par la formule (IVA) dans laquelle m varie de 3 à 9.

De manière avantageuse, le polymère de silicone de formule générale (I) utilisé dans la composition cosmétique conforme à l'invention, peut être choisi parmi la diméthicone de polyglycéryl-3 polyméthylsiloxyéthyle, la diméthicone de laurylpolyglycéryl-3 polyméthylsiloxyéthyle et la diméthicone de polyglycéryl-3 disiloxane, dont les formules sont respectivement :

- Diméthicone de polyglycéryl-3 polyméthylsiloxyéthyle (formule (V)) :

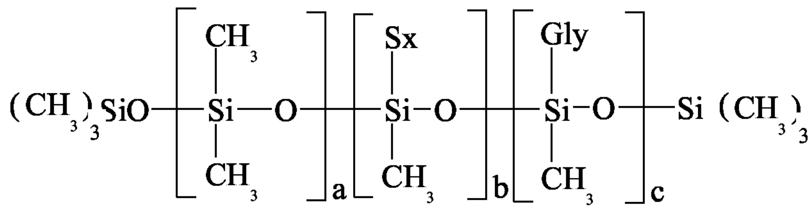 (V)

dans laquelle :

Sx : $-C_2H_4\ [(CH_3)_2SiO]_m si(CH_3)_3$
Gly : $-C_3H_6O[CH_2-CH(OH)CH_2O]_nH$

avec a = 1-1,4, b = 0,02-0,04, c = 0,02-0,04, m = 3-9, n = 1-5

- Diméthicone de lauryl polyglycéryl-3 polyméthylsiloxyéthyle (formule (VI)) :

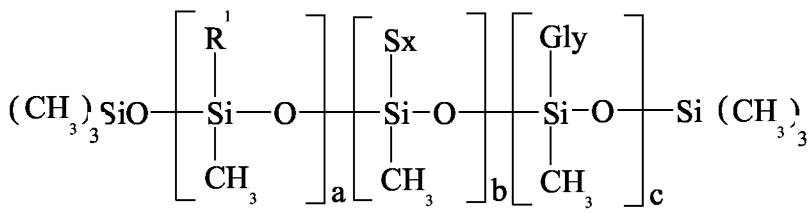 (VI)

dans laquelle Sx, Gly, a, b, c, m et n ont la même signification que précédemment et $R_1$ est, soit un radical méthyle, soit un radical lauryle.

- Diméthicone de polyglycéryl-3 disiloxane (formule (VII)) :

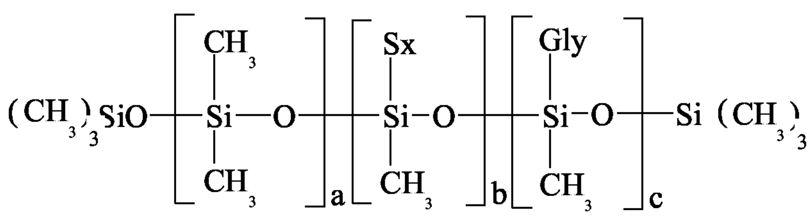 (VII)

dans laquelle Gly, a, b, c, m et n ont la même signification que précédemment, et

Sx : $-O(CH_3)_2SiO-Si(CH_3)_3$

Le polymère de silicone de formule générale (I) peut être présent dans les compositions cosmétiques conformes à la présente invention à raison de 0,1 à 40 % en poids, en particulier de 0,5 à 30 % en poids, plus particulièrement de 1 à 25 % en poids, en particulier de 5 à 20 % en poids, en particulier de 7 à 15 % en poids, par rapport au poids total de la composition.
Le polymère de silicone de formule générale (I) est, en particulier, mis en oeuvre dans la présente invention sous une forme libre.
Au sens de la présente invention, on entend désigner par "forme libre", une forme du polymère de silicone de formule générale (I) dans laquelle le polymère n'est pas mis en oeuvre sous une forme associée ou adsorbée à un autre matériau, comme décrit par exemple, dans les documents EP 1 416 016 et EP 1 424 373, où il est présent sous la forme d'un enrobage d'une poudre ou d'un agent de coloration afin de bloquer l'activité de surface des particules constituant la poudre correspondante.
Selon un mode particulier de réalisation, le polymère de silicone de formule générale (I) est avantageusement choisi parmi les polymères commercialisés par la société SHIN-ETSU sous les références KF6100®, KF6104® et

KF6105®.

Selon encore un autre mode de réalisation, le polymère commercialisé sous la référence KF6104® convient particulièrement à la préparation des compositions cosmétiques conformes à l'invention.

Selon encore un autre mode de réalisation, le composé désigné sous la référence KF6104®, commercialisé par la société SHIN-ETSU, convient particulièrement pour préparer des compositions cosmétiques conformes à l'invention et présentant une tenue sur les matières kératiniques améliorée, sans affectation de la brillance, voire avec une brillance moyenne également améliorée.

## MILIEU PHYSIOLOGIQUEMENT ACCEPTABLE

Par « milieu physiologiquement acceptable », on désigne un milieu non toxique et susceptible d'être appliqué sur la peau, les lèvres ou matières kératiniques d'êtres humains. Le milieu physiologiquement acceptable est généralement adapté à la nature du support sur lequel doit être appliquée la composition, ainsi qu'à l'aspect sous lequel la composition est destinée à être conditionnée.

Le milieu physiologiquement acceptable peut comprendre une phase aqueuse et/ou une phase grasse.

Selon un mode particulier de réalisation, la phase aqueuse ou la phase grasse peut former la phase continue de la composition.

Selon une variante, les compositions cosmétiques conformes à la présente invention peuvent être présentées sous la forme d'une émulsion, dans laquelle le polymère de silicone de formule générale (I), telle que définie précédemment, peut avoir la fonction de tensioactif.

Au sens de la présente invention, les émulsions contiennent une phase lipophile et une phase hydrophile, cette dernière n'étant pas systématiquement de l'eau.

Ainsi, les compositions cosmétiques conformes à l'invention peuvent être sous forme d'émulsion eau-dans-huile, huile-dans-eau, multiple ou anhydre.

Ainsi, les compositions cosmétiques conformes à l'invention peuvent être sous forme d'émulsion anhydre.

En particulier, la composition peut posséder, par exemple, une phase grasse continue, pouvant contenir moins de 10 % en poids d'eau, notamment moins de 5 % en poids d'eau par rapport au poids total de la composition.

Les compositions cosmétiques selon l'invention sont avantageusement anhydres, c'est-à-dire peuvent contenir moins de 5 %, en particulier moins de 3 %, en particulier moins de 2 %, et plus particulièrement moins de 1 % d'eau par rapport au poids total de la composition. Elles peuvent alors se présenter notamment sous forme de gels huileux, de liquides huileux, de pâtes ou de sticks ou encore sous forme d'une dispersion vésiculaire contenant des liquides ioniques et/ou non ioniques.

## AGENTS FILMOGENES

Par polymère "filmogène", on entend un polymère apte à former à lui seul ou en présence d'un agent auxiliaire de filmification, un film continu et adhérent sur un support, notamment sur les matières kératiniques, et de préférence un film cohésif et mieux encore, un film dont la cohésion et les propriétés mécaniques sont telles que ledit film peut être isolé dudit support.

Parmi les autres polymères filmogènes utilisables dans la composition de la présente invention, on peut citer les polymères synthétiques, de type radicalaire ou de type polycondensat, les polymères d'origine naturelle et leurs mélanges. Comme polymère filmogène, on peut citer en particulier les polymères acryliques, les polyuréthanes, les polyesters, les polyamides, les polyurées, les polymères cellulosiques comme la nitrocellulose.

Dans un mode de réalisation, le polymère filmogène est au moins un polymère choisi parmi le groupe comprenant :

- les polymères filmogènes solubles dans la phase grasse liquide, en particulier les polymères liposolubles, lorsque la phase grasse liquide comprend au moins une huile,
- les polymères filmogènes dispersibles dans la phase grasse liquide, en particulier les polymères sous la forme de dispersions non aqueuses de particules de polymères, en particulier des dispersions dans les huiles siliconées ou hydrocarbonées; dans un mode de réalisation, les dispersions non aqueuses de polymère comprennent des particules de polymères stabilisées sur leur surface mentionnées précédemment,
- les dispersions aqueuses de particules de polymères filmogènes, souvent appelées « latex » ; dans ce cas, la composition doit comprendre, outre la phase grasse liquide, une phase aqueuse,
- les polymères filmogènes hydrosolubles ; dans ce cas, la composition doit comprendre, outre la phase grasse liquide, une phase aqueuse.

**I. Filmogène dispersible dans la phase grasse liquide**

*1) Polymère greffé*

La composition selon l'invention peut comprendre, à titre d'agent filmogène, une dispersion de particules d'un polymère éthylénique greffé dans une phase grasse liquide.
Par polymère "éthylénique", on entend un polymère obtenu par polymérisation de monomères comprenant une insaturation éthylénique.
La dispersion de polymère éthylénique greffé est notamment exempte de polymère stabilisant distinct dudit polymère greffé, tels que ceux décrits dans EP749747 et décrits plus loin, et les particules de polymère éthylénique greffé ne sont donc pas stabilisées en surface par de tels polymères stabilisants additionnels. Le polymère greffé est donc dispersé dans la phase grasse liquide en l'absence de stabilisant additionnel en surface des particules.
Par polymère "greffé", on entend un polymère ayant un squelette comprenant au moins une chaîne latérale pendante ou située en bout de chaîne, et de préférence pendante.
Avantageusement, le polymère éthylénique greffé comprend un squelette éthylénique insoluble dans ladite phase grasse liquide, et des chaînes latérales liées de manière covalente audit squelette et solubles dans la phase grasse liquide.
Le polymère éthylénique greffé est notamment un polymère non réticulé. En particulier, le polymère est obtenu par polymérisation de monomères comprenant un seul groupement polymérisable.
Selon un mode de réalisation de l'invention, le polymère éthylénique greffé est un polymère acrylique greffé.
Le polymère éthylénique greffé est notamment susceptible d'être obtenu par polymérisation radicalaire dans un milieu organique de polymérisation :

- d'au moins un monomère éthylénique, en particulier d'au moins un monomère acrylique et éventuellement d'au moins un monomère additionnel vinylique non acrylique, pour former ledit squelette insoluble ; et
- d'au moins un macromonomère comportant un groupe terminal polymérisable pour former les chaînes latérales, ledit macromonomère ayant une masse moléculaire moyenne en poids supérieure ou égale à 200 et la teneur en macromonomère polymérisé représentant de 0,05 à 20 % en poids du polymère.

La phase grasse liquide peut contenir le milieu organique de polymérisation du polymère éthylénique greffé.
Le milieu organique liquide de dispersion, correspondant au milieu dans lequel est fourni le polymère greffé, peut être identique au milieu de polymérisation.
Toutefois, le milieu de polymérisation peut être substitué en tout ou partie par un autre milieu organique liquide. Cet autre milieu organique liquide peut être ajouté, après polymérisation, au milieu de polymérisation. Ce dernier est ensuite évaporé en tout ou partie.
La phase grasse liquide peut contenir des composés liquides organiques autres que ceux présents dans le milieu de dispersion. Ces autres composés sont choisis de manière à ce que le polymère greffé reste à l'état de dispersion dans la phase grasse liquide.
Le milieu liquide organique de dispersion est présent dans la phase grasse liquide de la composition selon l'invention du fait de l'introduction dans la composition de la dispersion de polymère greffé obtenue.
La phase grasse liquide comprend, de préférence majoritairement un ou plusieurs composés organiques liquides (ou huiles) tels que définis ci-après.
En particulier, la phase grasse liquide est une phase organique liquide non aqueuse et non miscible à l'eau à la température ambiante (25 °C).
On entend par "composé organique liquide" un composé non aqueux qui est à l'état liquide à la température ambiante (25 °C) et qui s'écoule donc de son propre poids.
On entend par "composé siliconé" un composé contenant au moins un atome de silicium.
Parmi les composés organiques liquides ou huiles pouvant être présents dans le milieu organique liquide de dispersion, on peut citer :

- les composés organiques liquides, notamment non siliconés ou siliconés, ayant un paramètre de solubilité global selon l'espace de solubilité de Hansen inférieur ou égal à 18 $(MPa)^{1/2}$, de préférence inférieur ou égal à 17 $(MPa)^{1/2}$,
- les monoalcools ayant un paramètre de solubilité global selon l'espace de solubilité de Hansen inférieur ou égal à 20 $(MPa)^{1/2}$; et
- leurs mélanges.

Le paramètre de solubilité global $\delta$ selon l'espace de solubilité de Hansen est défmi dans l'article « Solubility parameter values » de Eric A.Grulke de l'ouvrage « Polymer Handbook », 3$^{éme}$ édition, Chapitre VII, p.519-559 par la

relation :

$$\delta = (\delta_D{}^2 + \delta_P{}^2 + \delta_H{}^2)^{1/2}$$

dans laquelle :

- $d_D$ caractérise les forces de dispersion de LONDON issues de la formation de dipôles induits lors des chocs moléculaires,
- $d_P$ caractérise les forces d'interactions de DEBYE entre dipôles permanents, et
- $d_H$ caractérise les forces d'interactions spécifiques (type liaisons hydrogène, acide/base, donneur/accepteur, etc.).

La définition des solvants dans l'espace de solubilité selon Hansen est décrite dans l'article de C.M.Hansen « The three dimensional solubility parameters » J.Paint Technol. 39, 105 (1967).

Parmi les composés liquides organiques, notamment non siliconés ou siliconés, ayant un paramètre de solubilité global selon l'espace de solubilité de Hansen inférieur ou égal à 18 $(Mpa)^{1/2}$, on peut citer des corps gras liquides, notamment des huiles, qui peuvent être choisis parmi les huiles naturelles ou synthétiques, carbonées, hydrocarbonées, fluorées, siliconées, éventuellement ramifiées, seules ou en mélange.

Parmi ces huiles, on peut citer les huiles végétales formées par des esters d'acides gras et de polyols, en particulier les triglycérides, telles que l'huile de tournesol, de sésame ou de colza, ou les esters dérivés d'acides ou d'alcools à longue chaîne (c'est-à-dire ayant de 6 à 20 atomes de carbone), notamment les esters de formule RCOOR' dans laquelle R représente le reste d'un acide gras supérieur comportant de 7 à 19 atomes de carbone et R' représente une chaîne hydrocarbonée comportant de 3 à 20 atomes de carbone, tels que les palmitates, les adipates et les benzoates, notamment l'adipate de diisopropyle.

On peut également citer les alcanes linéaires, ramifiés et/ou cycliques éventuellement volatils et notamment des huiles de paraffme, de vaseline, ou le polyisobutylène hydrogéné, l'isododécane, ou encore les 'ISOPARS', les isoparaffines volatiles. On peut citer également les esters, les éthers, les cétones.

On peut encore citer les huiles siliconées telles que les polydiméthylsiloxanes et les polyméthylphénylsiloxanes, éventuellement substitués par des groupements aliphatiques et/ou aromatiques, éventuellement fluorés, ou par des groupements fonctionnels tels que des groupements hydroxyles, thiols et/ou amines, et les huiles siliconées volatiles, notamment cycliques.

En particulier, on peut citer les huiles de silicone, éventuellement ramifiées, volatiles et/ou non volatiles.

On peut citer, en particulier, comme composés organiques liquides non siliconés ayant un paramètre de solubilité global selon l'espace de solubilité de Hansen inférieur ou égal à 18 $(Mpa)^{1/2}$ :

- les esters linéaires, ramifiés ou cycliques, ayant au moins 6 atomes de carbone, notamment de 6 à 30 atomes de carbone ;
- les éthers ayant au moins 6 atomes de carbone, notamment de 6 à 30 atomes de carbone ; et
- les cétones ayant au moins 6 atomes de carbone, notamment de 6 à 30 atomes de carbone.

Par monoalcools liquides ayant un paramètre de solubilité global selon l'espace de solubilité de Hansen inférieur ou égal à 20 $(MPa)^{1/2}$, on entend les monoalcools liquides gras aliphatiques ayant de 6 à 30 atomes de carbone, la chaîne hydrocarbonée ne comportant pas de groupement de substitution. Comme monoalcools selon l'invention, on peut citer l'alcool oléique, le décanol, l'octyldodécanol et l'alcool linoléique.

Lorsque la phase grasse liquide de la composition cosmétique selon l'invention est une phase grasse liquide non siliconée, les macromonères présents dans le polymère greffé sont avantageusement des macromonomères carbonés tels que décrits ci-après.

En particulier, lorsque la phase grasse liquide de la composition cosmétique selon l'invention est une phase grasse liquide non siliconée, le polymère greffé présent dans la composition est avantageusement un polymère greffé non siliconé.

Par polymère greffé non siliconé, on entend un polymère greffé contenant majoritairement un macromonomère carboné et contenant éventuellement au plus 7 % en poids, de préférence au plus 5 % en poids, voire est exempt, de macromonomère siliconé.

Lorsque la phase grasse liquide de la composition cosmétique selon l'invention est une phase grasse liquide siliconée, les macromonomères présents dans le polymère greffé sont avantageusement des macromonomères siliconés tels que décrits ci-après.

En particulier, lorsque la phase grasse liquide est une phase grasse liquide siliconée, le polymère greffé présent

dans la composition est avantageusement un polymère greffé siliconé.

Par polymère greffé siliconé, on entend un polymère greffé contenant majoritairement un macromonomère siliconé et contenant éventuellement au plus 7 % en poids, de préférence au plus 5 % en poids, voire est exempt, de macromonomère carboné.

*a) Monomères*

Le choix des monomères constituant le squelette du polymère, des macromonomères, le poids moléculaire du polymère, la proportion des monomères et des macromonomères peut être fait en fonction du milieu organique liquide de dispersion de manière à obtenir avantageusement une dispersion de particules de polymères greffés en particulier une dispersion stable, ce choix pouvant être effectué par l'homme du métier.

Par "dispersion stable", on entend une dispersion qui n'est pas susceptible de former de dépôt solide ou de déphasage liquide/solide notamment après une centrifugation, par exemple, à 4000 tours/minute pendant 15 minutes.

Le polymère éthylénique greffé formant les particules en dispersion comprend donc un squelette insoluble dans ledit milieu de dispersion et une partie soluble dans ledit milieu de dispersion.

Le polymère éthylénique greffé peut être un polymère statistique.

Selon l'invention, on entend par "polymère éthylénique greffé "un polymère susceptible d'être obtenu par polymérisation radicalaire :

- d'un ou plusieurs monomère(s) éthylénique(s),
- avec un ou plusieurs macromonomère(s), dans un milieu organique de polymérisation.

Selon l'invention, on entend par "polymère acrylique greffé " un polymère susceptible d'être obtenu par polymérisation radicalaire :

- d'un ou plusieurs monomère(s) acrylique(s), et éventuellement d'un ou plusieurs monomère(s) additionnel(s) vinylique(s) non acrylique(s),
- avec un ou plusieurs macromonomère(s), dans un milieu organique de polymérisation.

Avantageusement, les monomères acryliques représentent de 50 à 100 % en poids, de préférence de 55 à 100 % en poids (notamment de 55 à 95 % en poids), préférentiellement de 60 à 100 % en poids (notamment de 60 à 90 % en poids) du mélange monomères acryliques + monomères vinyliques non acryliques éventuels.

En particulier, les monomères acryliques sont choisis parmi les monomères dont l'homopolymère est insoluble dans le milieu de dispersion considéré, c'est-à-dire que l'homopolymère est sous forme solide (ou non dissous) à une concentration supérieure ou égale à 5% en poids à température ambiante (20°C) dans ledit milieu de dispersion.

Selon l'invention, on entend par "macromonomère ayant un groupe terminal polymérisable" tout polymère comportant sur une seule de ses extrémités un groupe terminal polymérisable apte à réagir lors de la réaction de polymérisation avec les monomères acryliques et éventuellement les monomères vinyliques non acryliques additionnels constituant le squelette. Le macromonomère permet de former les chaînes latérales du polymère acrylique greffé. Le groupe polymérisable du macromonomère peut être avantageusement un groupe à insaturation éthylénique susceptible de se polymériser par voie radicalaire avec les monomères constituant le squelette.

Par "macromonomère carboné" on entend un macromonomère non siliconé, et notamment un macromonomère oligomère obtenu par polymérisation de monomère(s) non siliconé(s) à insaturation éthylénique, et principalement par polymérisation de monomères acryliques et/ou vinyliques non acryliques.

Par "macromonomère siliconé" on entend un macromonomère organopolysiloxane, et en particulier un macromonomère polydiméthylsiloxane.

En particulier, le macromonomère est choisi parmi les macromonomères dont l'homopolymère est soluble dans le milieu de dispersion considéré, c'est-à-dire complètement dissous à une concentration supérieure ou égale à 5 % en poids et à température ambiante dans ledit milieu de dispersion.

Ainsi, le polymère acrylique greffé comprend un squelette (ou chaîne principale) constitué par un enchaînement de motifs acryliques résultant de la polymérisation notamment d'un ou plusieurs monomères acryliques et des chaînes latérales (ou greffons) issus de la réaction des macromonomères, lesdites chaînes latérales étant liées de manière covalente à ladite chaîne principale.

Le squelette (ou chaîne principale) est insoluble dans le milieu de dispersion considéré alors que les chaînes latérales (ou greffons) sont solubles dans ledit milieu de dispersion.

Par "monomère acrylique", on entend dans la présente demande des monomères choisis parmi l'acide (méth)acrylique, les esters de l'acide (méth)acrylique (appelés également les (méth)acrylates), les amides de l'acide (méthacrylique) (appelés également les (méth)acrylamides).

Comme monomère acrylique susceptible d'être employé pour former le squelette insoluble du polymère, on peut citer, seul ou en mélange, les monomères suivants, ainsi que leurs sels :

- (i) les (méth)acrylates de formule (VIII) :

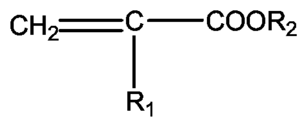

(VIII)

dans laquelle :

- $R_1$ désigne un atome d'hydrogène ou un groupe méthyle ;
- $R_2$ représente un groupe choisi parmi :

  - un groupe alkyle linéaire ou ramifié, comprenant de 1 à 6 atomes de carbone, ledit groupe pouvant comporter dans sa chaîne un ou plusieurs hétéroatomes choisis parmi O, N et S; et/ou pouvant comporter un ou plusieurs substituants choisis parmi -OH, les atomes d'halogène (F, Cl, Br, I) et -NR'R" avec R' et R" identiques ou différents choisis parmi les alkyles linéaires ou ramifiés en $C_1$-$C_4$; et/ou pouvant être substitué par au moins un groupe polyoxyalkylène, en particulier avec alkylène en $C_2$-$C_4$, notamment polyoxyéthylène et/ou polyoxypropylène, ledit groupe polyoxyalkylène étant constitué par la répétition de 5 à 30 motifs oxyalkylène;
  - un groupe alkyle cyclique comprenant de 3 à 6 atomes de carbone, ledit groupe pouvant comporter dans sa chaîne un ou plusieurs hétéroatomes choisis parmi O, N et S, et/ou pouvant comporter un ou plusieurs substituants choisis parmi OH et les atomes d'halogène (F, Cl, Br, I);

A titre d'exemples de $R_2$, on peut citer le groupe méthyle, éthyle, propyle, butyle, isobutyle, méthoxyéthyle, éthoxyéthyle, méthoxy-polyoxyéthylène 350 OE, trifluoroéthyle, 2-hydroxyéthyle, 2-hydroxypropyle, diméthylaminoéthyle, diéthylaminoéthyle, diméthylaminopropyle.

- (ii) les (méth)acrylamides de formule (IX) :

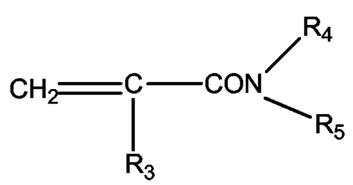

(IX)

dans laquelle :

- $R_3$ désigne un atome d'hydrogène ou un groupe méthyle ;
- $R_4$ et $R_5$, identiques ou différents, représentent un atome d'hydrogène ou un groupe alkyle, linéaire ou ramifié, comportant de 1 à 6 atomes de carbone, pouvant comporter un ou plusieurs substituants choisis parmi -OH, les atomes d'halogène (F, Cl, Br, I) et -NR'R" avec R' et R" identiques ou différents choisis parmi les alkyles linéaires ou ramifiés en $C_1$-$C_4$; ou
- $R_4$ représente un atome d'hydrogène et $R_5$ représente un groupe 1,1-diméthyl-3-oxobutyle.

A titre d'exemples de groupes alkyles pouvant constituer $R_4$ et $R_5$, on peut citer n-butyle, t-butyle, n-propyle, diméthylaminoéthyle, diéthylaminoéthyle, diméthylaminopropyle :

- (iii) les monomères (méth)acryliques comprenant au moins une fonction acide carboxylique, phosphorique ou sulfonique, tels que l'acide acrylique, l'acide méthacrylique, l'acide acrylamidopropanesulfonique.

Parmi ces monomères acryliques, on peut tout particulièrement citer les (méth)acrylates de méthyle, d'éthyle, de propyle, de butyle, d'isobutyle; les (méth)acrylates de méthoxyéthyle ou d'éthoxyéthyle; le méthacrylate de trifluoroéthyle; le méthacrylate de diméthylaminoéthyle, le méthacrylate de diéthylaminoéthyle, le méthacrylate de 2-hydroxypropyle, le méthacrylate de 2-hydroxyéthyle, l'acrylate de 2-hydroxypropyle, l'acrylate de 2-hydroxyéthyle ; le diméthylaminopropylméthacrylamide; et leurs sels ; et leurs mélanges.

En particulier, les monomères acryliques sont choisis parmi l'acrylate de méthyle, l'acrylate de méthoxyéthyle, le méthacrylate de méthyle, le méthacrylate de 2-hydroxyéthyle, l'acide acrylique, le méthacrylate de diméthylaminoéthyle, et leurs mélanges.

Parmi les monomères additionnels vinyliques non acryliques, on peut citer :

- les esters vinyliques de formule suivante :

$$R_6\text{-COO-CH=CH}_2$$

dans laquelle :

- $R_6$ représente un groupe alkyle linéaire ou ramifié, comprenant de 1 à 6 atomes, ou un groupe alkyle cyclique comportant de 3 à 6 atomes de carbone et/ou un groupe aromatique, par exemple de type benzénique, anthracénique, et naphtalénique ;
- les monomères vinyliques non acryliques comprenant au moins une fonction acide carboxylique, phosphorique ou sulfonique, tels que l'acide crotonique, l'anhydride maléique, l'acide itaconique, l'acide fumarique, l'acide maléique, l'acide styrènesulfonique, l'acide vinylbenzoïque, l'acide vinylphosphorique, et leurs sels ;
- les monomères vinyliques non acryliques comprenant au moins une fonction amine tertiaire, tels que la 2-vinylpyridine, la 4-vinylpyridine ;
- et leurs mélanges.

Avantageusement, les monomères acryliques présents dans le polymère greffés comprennent au moins l'acide (méth)acrylique et au moins un monomère choisi parmi les (méth)acrylates et les (méth)acrylamides décrits précédemment aux points (i) et (ii). De préférence, les monomères acryliques comprennent au moins l'acide (méth)acrylique et au moins un monomère choisi parmi les (méth)acrylates d'alkyle en $C_1$-$C_3$. L'acide (méth)acrylique peut être présent en une teneur d'au moins 5 % en poids, par rapport au poids total du polymère, notamment allant de 5 % à 80 % en poids, de préférence d'au moins 10 % en poids, notamment allant de 10 % en poids à 70 % en poids, préférentiellement d'au moins 15 % en poids, notamment allant de 15 % à 60 % en poids.

Parmi les sels, on peut citer ceux obtenus par neutralisation des groupements acides à l'aide de bases inorganiques telles que l'hydroxyde de sodium, l'hydroxyde de potassium, l'hydroxyde d'ammonium ou de bases organiques de type alkanols amines comme la monoéthanolamine, la diéthanolamine, la triéthanolamine, la 2-méthyl-2-amino-1-propanol.

On peut également citer les sels formés par neutralisation des motifs amine tertiaire, par exemple à l'aide d'acide minéral ou organique. Parmi les acides minéraux, on peut citer l'acide sulfurique ou l'acide chlorhydrique, l'acide bromhydrique, iodhydrique, l'acide phosphorique, l'acide borique. Parmi les acides organiques, on peut citer les acides comportant un ou plusieurs groupes carboxylique, sulfonique, ou phosphonique. Il peut s'agir d'acides aliphatiques linéaires, ramifiés ou cycliques ou encore d'acides aromatiques. Ces acides peuvent comporter, en outre, un ou plusieurs hétéroatomes choisis parmi O et N, par exemple sous la forme de groupes hydroxyles. On peut notamment citer l'acide acétique ou l'acide propionique, l'acide téréphtalique, ainsi que l'acide citrique et l'acide tartrique.

Selon un mode de réalisation de l'invention, le polymère éthylénique greffé ne contient pas de monomères vinyliques non acryliques additionnels tels que décrits précédemment. Dans ce mode de réalisation, le squelette insoluble du polymère éthylénique greffé est formé uniquement de monomères acryliques tels que décrits précédemment.

Il est entendu que ces monomères acryliques non polymérisés peuvent être solubles dans le milieu de dispersion considéré, mais le polymère formé avec ces monomères est insoluble dans le milieu de dispersion.

Selon un mode particulier de réalisation de l'invention, le polymère éthylénique greffé est susceptible d'être obtenu par polymérisation radicalaire dans un milieu organique de polymérisation :

- d'un monomère acrylique principal choisi parmi les (méth)acrylates d'alkyle en $C_1$-$C_3$, seul ou en mélange, et éventuellement d'un ou plusieurs monomères acryliques additionnels choisis parmi l'acide (méth)acrylique, l'acide méthacrylique et les (méth)acrylates d'alkyle de formule (X) définie ci-après, et leurs sels, pour former ledit squelette insoluble ; et
- et d'au moins un macromonomère siliconé comportant un groupe terminal polymérisable, tel que défini précédemment.

Comme monomère acrylique principal, on peut utiliser l'acrylate de méthyle, le méthacrylate de méthyle, l'acrylate d'éthyle, le méthacrylate d'éthyle, l'acrylate de n-propyle, le méthacrylate de n-propyle, l'acrylate d'iso-propyle et le méthacrylate d'iso-propyle, et leurs mélanges.

On peut citer tout particulièrement l'acrylate de méthyle, le méthacrylate de méthyle, le méthacrylate d'éthyle.

Les monomères acryliques additionnels peuvent être choisis parmi :

- l'acide (méth)acrylique et ses sels,
- les (méth)acrylates de formule (X) et leurs sels :

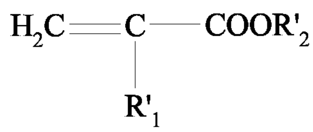

(X)

dans laquelle :

- $R'_1$ désigne un atome d'hydrogène ou un groupe méthyle ;
- $R'_2$ représente :
  - un groupe alkyle linéaire ou ramifié comprenant de 1 à 6 atomes de carbone, ledit groupe comportant dans sa chaîne un ou plusieurs atomes d'oxygène et/ou comportant un ou plusieurs substituants choisis parmi -OH, les atomes d'halogène (F, Cl, Br, I) et -NR'R" avec R' et R" identiques ou différents choisis parmi les alkyles linéaires ou ramifiés en $C_1$-$C_3$ ;
  - un groupe alkyle cyclique comprenant de 3 à 6 atomes de carbone, ledit groupe pouvant comporter dans sa chaîne un ou plusieurs atomes d'oxygène et/ou pouvant comporter un ou plusieurs substituants choisis parmi OH et les atomes d'halogène (F, Cl, Br, I) ;
  - et leurs mélanges.

A titre d'exemples de $R'_2$, on peut citer le groupe méthoxyéthyle, éthoxyéthyle, trifluoroéthyle; 2-hydroxyéthyle, 2-hydroxypropyle, diméthylaminoéthyle, diéthylaminoéthyle, diméthylaminopropyle.

Parmi ces monomères acryliques additionnels, on peut tout particulièrement citer l'acide (méth)acrylique, les (méth)acrylates de méthoxyéthyle ou d'éthoxyéthyle; le méthacrylate de trifluoroéthyle; le méthacrylate de diméthylaminoéthyle, le méthacrylate de diéthylaminoéthyle, le méthacrylate de 2-hydroxypropyle, le méthacrylate de 2-hydroxyéthyle, l'acrylate de 2-hydroxypropyle, l'acrylate de 2-hydroxyéthyle, leurs sels, et leurs mélanges.

On peut citer tout particulièrement l'acide acrylique et l'acide méthylacrylique.

*b) Macromonomères*

Les macromonomères comportent à une des extrémités de la chaîne un groupe terminal polymérisable apte à réagir au cours de la polymérisation avec les monomères acryliques et éventuellement les monomères vinyliques additionnels, pour former les chaînes latérales du polymère éthylénique greffé. Ledit groupe terminal polymérisable peut être en particulier un groupe vinyle ou (méth)acrylate (ou (méth)acryloxy), et de préférence un groupe (méth)acrylate.

Les macromonomères sont choisis préférentiellement parmi les macromonomères dont l'homopolymère a une température de transition vitreuse (Tg) inférieure ou égale à 25°C, notamment allant de - 100°C à 25°C, de préférence allant de - 80°C à 0°C.

Les macromonomères ont une masse moléculaire moyenne en poids supérieure ou égale à 200, de préférence supérieure ou égale à 300, préférentiellement supérieure ou égale à 500, et plus préférentiellement supérieure à 600.

De préférence, les macromonomères ont une masse moléculaire moyenne en poids (Mw) allant de 200 à 100 000, de préférence allant de 500 à 50 000, préférentiellement allant de 800 à 20 000, plus préférentiellement allant de 800 à 10000, et encore plus préférentiellement allant de 800 à 6000.

Dans la présente demande, les masses molaires moyennes en poids (Mw) et en nombre (Mn) sont déterminées par chromatographie liquide par perméation de gel (solvant THF, courbe d'étalonnage établie avec des étalons de polystyrène linéaire, détecteur réfractométrique).

Comme macromonomères carbonés, on peut en particulier citer :

- (i) les homopolymères et les copolymères (méth)acrylate d'alkyle linéaire ou ramifié en C8 à C22, présentant un groupe terminal polymérisable choisi parmi les groupes vinyle ou (méth)acrylate parmi lesquels on peut citer en particulier : les macromonomères de poly(acrylate d'éthyl-2 hexyle) à extrémité mono(méth)acrylate ; les macromonomères de poly(acrylate de dodécyle) ou de poly(méthacrylate de dodécyle) à extrémité mono(méth)acrylate ; les macromonomères de poly(acrylate de stéaryle) ou de poly (méthacrylate de stéaryle) à extrémité mono(méth)acrylate.
De tels macromonomères sont notamment décrits dans les brevets EP895467 et EP96459 et dans l'article Gillman K.F., Polymer Letters, Vol 5, page 477-481 (1967).
On peut en particulier citer les macromonomères à base de poly(acrylate d'éthyl-2-hexyle) ou de poly(acrylate de dodécyle) à extrémité mono(méth)acrylate.

- (ii) les polyoléfines ayant un groupe terminal à insaturation éthylénique , en particulier ayant un groupement terminal (méth)acrylate. Comme exemple de telles polyoléfmes, on peut citer en particulier les macromonomères suivants, étant entendu qu'ils ont un groupe terminal (méth)acrylate : les macromonomères de polyéthylène, les macromonomères de polypropylène, les macromonomères de copolymère polyéthylène/polypropylène, les macromonomères de copolymère polyéthylène/polybutylène, les macromonomères de polyisobutylène ; les macromonomères de polybutadiène; les macromonomères de polyisoprène ; les macromonomères de polybutadiène; les macromonomères de poly(éthylène/butylène)-polyisoprène.

De tels macromonomères sont en particulier décrits dans US5625005 qui mentionne des macromonomères éthylène/butylène et éthylène/propylène à groupement terminal réactif (méth)acrylate.

On peut en particulier citer le méthacrylate de poly(éthylène/butylène), tel que celui commercialisé sous la dénomination KRATON LIQUID L-1253 par KRATON POLYMERS.

Comme macromonomères siliconés, on peut en particulier citer les polydiméthylsiloxanes à groupement terminal mono (méth)acrylate, et notamment ceux de formule (XI) suivante :

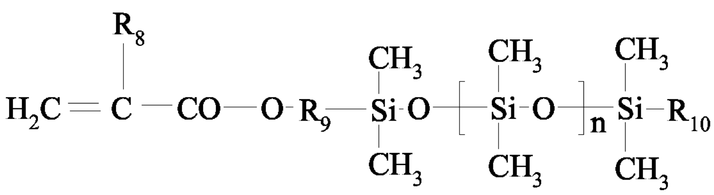

(XI)

dans laquelle :

- $R_8$ désigne un atome d'hydrogène ou un groupement méthyle ;
- $R_9$ désigne un groupe hydrocarboné divalent ayant de 1 à 10 atomes de carbone et contient éventuellement une ou deux liaisons éther -O- ;
- $R_{10}$ désigne un groupe alkyl ayant de 1 à 10 atomes de carbone, notamment ayant de 2 à 8 atomes de carbone ; et
- n désigne un nombre entier allant de 1 à 300, de préférence allant de 3 à 200, et préférentiellement allant de 5 à 100.

Comme macromonomères siliconés, on peut utiliser les monométhacryloxypropyl polydiméthylsiloxanes tels que ceux commercialisés sous la dénomination PS560-K6 par la société UNITED CHEMICAL TECHNOLOGIES INC. (UCT) ou sous la dénomination MCR-M17 par la société GELEST INC.

Plus particulièrement, le macromonomère polymérisé (constituant les chaînes latérales du polymère greffé) représente de 0,1 à 15 % en poids du poids total du polymère, préférentiellement de 0,2 à 10 % en poids, et plus préférentiellement de 0,3 à 8 % en poids.

Comme polymère éthylénique greffé particulièrement avantageux dispersé dans une phase grasse liquide non siliconée, on peut utiliser ceux obtenus par polymérisation :

- de l'acrylate de méthyle et du macromonomère polyéthylène/polybutylène à groupement terminal méthacrylate (notamment KRATON L-1253), en particulier dans un solvant choisi parmi l'isododécane, l'isononanoate d'isononyle, l'octyldodécanol , le malate de diisostéaryle, un benzoate d'alkyl $C_{12}$-$C_{15}$ (tel que Finsolv TN) ;
- de l'acrylate de méthoxyéthyle et du macromonomère polyéthylène/polybutylène à groupement terminal méthacrylate (notamment KRATON L-1253), en particulier dans l'isododécane ;
- des monomères acrylate de méthyle/méthacrylate de méthyle et du macromonomère polyéthylène/polybutylène à groupement terminal méthacrylate (notamment KRATON L-1253), en particulier dans l'isododécane ;
- des monomères acrylate de méthyle/acide acrylique et du macromonomère polyéthylène/polybutylène à groupement terminal méthacrylate (notamment KRATON L-1253), en particulier dans l'isododécane ;
- des monomères acrylate de méthyle/méthacrylate de diméthylaminoéthyle et du macromonomère polyéthylène/polybutylène à groupement terminal méthacrylate (notamment KRATON L-1253), en particulier dans l'isododécane ;

- des monomères acrylate de méthyle / méthacrylate de 2-hydroxyéthyle et du macromonomère polyéthylène/polybutylène à groupement terminal méthacrylate (notamment KRATON L-1253), en particulier dans l'isododécane.

Comme polymère acrylique greffé particulièrement envisagé dispersé dans une phase grasse liquide siliconée, on peut utiliser ceux obtenus par polymérisation :

- de l'acrylate de méthyle et du macromonomère monométhacrylolxypropylpolydiméthylsiloxane ayant un poids moléculaire moyen en poids allant de 800 à 6000, en particulier dans le décaméthylcyclopentasiloxane ou le phényltriméthicone ;
- de l'acrylate de méthyle, d'acide acrylique et du macromonomère monométhacryloxypropylpolydiméthylsiloxane ayant un poids moléculaire moyen en poids allant de 800 à 6000, en particulier dans le décaméthylcyclopentasiloxane ou le phényltriméthicone.

En particulier, le polymère greffé a une masse moléculaire moyenne en poids (Mw) comprise entre 10 000 et 300 000, notamment entre 20 000 et 200 000, mieux encore entre 25 000 et 150 000.

Grâce aux caractéristiques susmentionnées, dans un milieu organique de dispersion donné, les polymères ont la capacité de se replier sur eux-mêmes, formant ainsi des particules de forme sensiblement sphérique, avec sur le pourtour de ces particules les chaînes latérales déployées, qui assurent la stabilité de ces particules. De telles particules résultant des caractéristiques du polymère greffé ont la particularité de ne pas s'agglomérer dans ledit milieu et donc de s'autostabiliser et de former une dispersion de particules de polymère particulièrement stable.

En particulier, les polymères éthyléniques greffés de la dispersion peuvent former des particules nanométriques, de taille moyenne allant de 10 à 400 nm, de préférence de 20 à 200 nm.

Du fait de cette taille très faible, les particules de polymère greffé en dispersion sont particulièrement stables et donc peu susceptibles de former des agglomérats.

La dispersion de polymère greffé peut donc être une dispersion stable et ne forme pas de sédiments, lorsqu'elle est placée pendant une durée prolongée (par exemple 24 heures) à température ambiante (25 °C).

En particulier, la dispersion de particules de polymère greffé présente un taux de matière sèche (ou extrait sec) en polymère pouvant aller de 40 % à 70 % en poids de matière sèche, notamment allant de 45 % à 65 % en poids.

*c) Procédé d'obtention*

On peut préparer la dispersion de particules de polymère greffé par un procédé comprenant une étape de copolymérisation radicalaire, dans un milieu organique de polymérisation, d'un ou plusieurs monomères acryliques tels que définis précédemment avec un ou plusieurs macromonomères tels que définis précédemment.

Comme indiqué précédemment, le milieu organique liquide de dispersion peut être identique ou différent du milieu de polymérisation.

D'une manière classique, la copolymérisation peut être effectuée en présence d'un initiateur de polymérisation. Les initiateurs de polymérisation peuvent être des amorceurs radicalaires. De manière générale, un tel initiateur de polymérisation peut être choisi parmi les composés organiques peroxydés tels que le dilauroyl peroxyde, le dibenzoyl peroxyde, le tert-butyl peroxy-2-éthylhexanoate ; les composés diazotés tels que l'azobisisobutyronitrile, l'azobisdiméthylvalero-nitrile.

La réaction peut être également initiée à l'aide de photoinitiateurs ou par une radiation telle que des UV, des neutrons ou par plasma.

D'une manière générale, pour mettre en oeuvre ce procédé, on introduit, dans un réacteur de taille appropriée à la quantité de polymère que l'on va réaliser, au moins une partie du milieu organique de polymérisation, une partie des monomères acryliques et/ou vinyliques additionnels, qui constituera, après polymérisation, le squelette insoluble, la totalité du macromonomère (qui constituera les chaînes latérales du polymère) et une partie de l'initiateur de polymérisation. A ce stade d'introduction, le milieu réactionnel forme un milieu relativement homogène.

Le milieu réactionnel est ensuite agité et chauffé jusqu'à une température pour obtenir une polymérisation des monomères et macromonomères. Après un certain temps, le milieu initialement homogène et limpide conduit à une dispersion d'aspect laiteux. On ajoute ensuite un mélange constitué de la partie restante de monomères et de l'initiateur de polymérisation. Après un temps adéquat pendant lequel le mélange est chauffé sous agitation, le milieu se stabilise sous forme d'une dispersion laiteuse, la dispersion comprenant des particules de polymères stabilisés dans le milieu dans lequel elles ont été créées, ladite stabilisation étant due à la présence, dans le polymère, de chaînes latérales solubles dans ledit milieu de dispersion.

Le polymère greffé peut être présent dans la composition selon l'invention en une teneur en matière sèche (ou matière active) allant de 1 à 70 % en poids par rapport au poids total de la composition, mieux de 5 à 60% en poids, de préférence allant de 6 à 45% et mieux allant de 8 à 40% en poids.

*2) Polymère séquencé*

La composition selon l'invention peut contenir, à titre d'agent filmogène un polymère éthylénique séquencé linéaire, appelé par la suite "polymère séquencé", de structure particulière telle que décrite ci-après.

Par polymère "séquencé", on entend un polymère comprenant au moins 2 séquences distinctes, de préférence au moins 3 séquences distinctes.

Le polymère est un polymère à structure linéaire. Par opposition, un polymère de structure non linéaire est, par exemple, un polymère à structure ramifiée, en étoile, greffée, ou autre.

Avantageusement, le polymère séquencé est exempt de styrène. Par "polymère exempt de styrène", on entend un polymère contenant moins de 10 % en poids, par rapport au poids total du polymère, de préférence moins de 5 % en poids, mieux moins de 2 % en poids, mieux moins de 1 % en poids, voire ne contenant pas, de monomère styrénique comme le styrène, les dérivés de styrène tels que le méthylstyrène, le chlorostyrène ou le chlorométhylstyrène.

De manière particulière, le polymère séquencé comprend au moins une première séquence et au moins une deuxième séquence ayant des températures de transition vitreuse (Tg) différentes, lesdites première et deuxième séquences étant reliées entre elles par une séquence intermédiaire comprenant au moins un monomère constitutif de la première séquence et au moins un monomère constitutif de la deuxième séquence.

Par « au moins » une séquence, on entend une ou plusieurs séquences.

La séquence intermédiaire est une séquence comprenant au moins un monomère constitutif de la première séquence et au moins un monomère constitutif de la deuxième séquence du polymère permet de « compatibiliser » ces séquences.

On précise que dans ce qui précède et ce qui suit les termes "première" et « deuxième » séquences ne conditionnent nullement l'ordre desdites séquences (ou blocs) dans la structure du polymère séquencé.

Avantageusement, les première et deuxième séquences et du polymère séquencé sont incompatibles l'une avec l'autre.

Par "séquences incompatibles l'une avec l'autre", on entend que le mélange formé du polymère correspondant à la première séquence et du polymère correspondant à la deuxième séquence, n'est pas miscible dans le liquide organique majoritaire en poids du la phase grasse liquide, à température ambiante (25°C) et pression atmosphérique ($10^5$ Pa), pour une teneur du mélange de polymères supérieure ou égale à 5 % en poids, par rapport au poids total du mélange (polymères et solvant), étant entendu que :

- i) lesdits polymères sont présents dans le mélange en une teneur telle que le rapport pondéral respectif va de 10/90 à 90/10, et que
- ii) chacun des polymères correspondant au première et seconde séquences a une masse moléculaire moyenne (en poids ou en nombre) égale à celle du polymère séquencé +/- 15%.

Dans le cas où la phase grasse liquide comprend un mélange de liquide organiques, et dans l'hypothèse de deux ou plusieurs liquides organiques présents en proportions massiques identiques, ledit mélange de polymères est non miscible dans au moins l'un d'entre eux.

Bien entendu, dans le cas où la phase grasse liquide comprend un unique liquide organique, ce dernier est le liquide organique majoritaire.

De façon particulière, le polymère séquencé ne comprend pas d'atomes de silicium dans son squelette. Par "squelette", on entend la chaîne principale du polymère, par opposition aux chaînes latérales pendantes.

En particulier, le polymère séquencé n'est pas soluble dans l'eau ou dans un mélange d'eau et de monoalcools inférieurs linéaires ou ramifiés ayant de 2 à 5 atomes de carbone comme l'éthanol, l'isopropanol ou le n-propanol, sans modification de pH, à une teneur en matière active d'au moins 1% en poids, à température ambiante (25°C).

En particulier, le polymère séquencé n'est pas un élastomère.

Par "polymère non élastomère", on entend un polymère qui, lorsqu'il est soumis à une contrainte visant à l'étirer (par exemple de 30% relativement à sa longueur initiale), ne revient pas à une longueur sensiblement identique à sa longueur initiale lorsque cesse la contrainte.

*a) Test de recouvrance*

De manière plus spécifique, par "polymère non élastomère" on désigne un polymère ayant une recouvrance instantanée $R_i$ < à 50% et une recouvrance retardée $R_{2h}$ < 70% après avoir subi un allongement de 30%. De préférence, $R_i$ est < à 30 %, et $R_{2h}$ < 50.

Plus précisément, le caractère non élastomérique du polymère est déterminé selon le protocole suivant :

On prépare un film de polymère par coulage d'une solution du polymère dans une matrice téflonnée puis séchage

pendant 7 jours dans une ambiance contrôlée à 23±5°C et 50±10 % d'humidité relative.
On obtient alors un film d'environ 100 µm d'épaisseur dans lequel sont découpées des éprouvettes rectangulaires (par exemple à l'emporte-pièce) d'une largeur de 15 mm et d'une longueur de 80 mm.
On impose à cet échantillon une sollicitation de traction à l'aide d'un appareil commercialisé sous la référence Zwick, dans les mêmes conditions de température et d'humidité que pour le séchage.

Les éprouvettes sont étirées à une vitesse de 50 mm/min et la distance entre les mors est de 50 mm, ce qui correspond à la longueur initiale ($l_0$) de l'éprouvette.
On détermine la recouvrance instantanée Ri de la manière suivante :

- on étire l'éprouvette de 30 % ($\varepsilon_{max}$) c'est-à-dire environ 0,3 fois sa longueur initiale ($l_0$)
- on relâche la contrainte en imposant une vitesse de retour égale à la vitesse de traction, soit 50 mm/min et on mesure l'allongement résiduel de l'éprouvette en pourcentage, après retour à contrainte nulle ($\varepsilon_i$).

La recouvrance instantanée en % ($R_i$) est donnée par la formule ci-après :

$$R_i = (\varepsilon_{max} - \varepsilon_i)/ \varepsilon_{max}) \times 100$$

Pour déterminer la recouvrance retardée, on mesure l'allongement résiduel de l'éprouvette en pourcentage ($\varepsilon_{2h}$), 2 heures après retour à la contrainte nulle.
La recouvrance retardée en % ($R_{2h}$) est donnée par la formule ci-après:

$$R_{2h}= (\varepsilon_{max} - \varepsilon_{2h})/\varepsilon_{max}) \times 100$$

A titre purement indicatif, un polymère selon un mode de réalisation de l'invention possède une recouvrance instantanée $R_i$ de 10% et une recouvrance retardée $R_{2h}$ de 30%.
Avantageusement, le polymère séquencé a un indice de polydispersité I supérieur à 2, par exemple allant de 2 à 9, en particulier supérieur ou égal à 2,5, par exemple allant de 2,5 à 8, et plus particulièrement supérieur ou égal à 2,8 et notamment, allant de 2,8 à 6.
L'indice de polydispersité I du polymère séquencé est égal au rapport de la masse moyenne en poids Mw sur la masse moyenne en nombre Mn.
On détermine les masses molaires moyennes en poids (Mw) et en nombre (Mn) par chromatographie liquide par perméation de gel (solvant THF, courbe d'étalonnage établie avec des étalons de polystyrène linéaire, détecteur réfractométrique).
La masse moyenne en poids (Mw) du polymère séquencé est en particulier inférieure ou égale à 300 000, elle va par exemple de 35 000 à 200 000, et plus particulièrement de 45 000 à 150 000.
La masse moyenne en nombre (Mn) du polymère séquencé est en particulier inférieure ou égale à 70 000, elle va par exemple de 10 000 à 60 000, et plus particulièrement de 12 000 à 50 000.
Chaque séquence ou bloc du polymère séquencé est issue d'un type de monomère ou de plusieurs types de monomères différents.
Cela signifie que chaque séquence peut être constituée d'un homopolymère ou d'un copolymère ; ce copolymère constituant la séquence pouvant être à son tour statistique ou alterné.
Avantageusement, la séquence intermédiaire comprenant au moins un monomère constitutif de la première séquence et au moins un monomère constitutif de la deuxième séquence du polymère séquencé est un polymère statistique.
En particulier, la séquence intermédiaire est issue essentiellement de monomères constitutifs de la première séquence et de la deuxième séquence.
Par "essentiellement", on entend au moins à 85%, en particulier au moins à 90%, en particulier à 95% et encore plus particulièrement à 100%.
Avantageusement, la séquence intermédiaire a une température de transition vitreuse Tg comprise entre les températures de transition vitreuse des première et deuxième séquences.
Les températures de transition vitreuse indiquées des première et deuxième séquences peuvent être des Tg théoriques déterminées à partir des Tg théoriques des monomères constitutifs de chacune des séquences, que l'on peut trouver dans un manuel de référence tel que le Polymer Handbook, 3rd ed, 1989, John Wiley, selon la relation suivante, dite Loi de Fox :

$$1/Tg = \sum_i (\omega_i/Tg_i),$$

,
$\omega_i$ étant la fraction massique du monomère i dans la séquence considerée et $Tg_i$ étant la température de transition vitreuse de l'homopolymère du monomère i.

Sauf indication contraire, les Tg indiquées pour les première et deuxième séquences dans la présente demande sont des Tg théoriques.

L'écart entre les températures de transition vitreuse de la première et de la deuxième séquences est généralement supérieur à 10 ˚C, en particulier supérieur à 20 ˚C, et en particulier supérieur à 30 ˚C.

*b) Séquences du polymère*

En particulier, la première séquence du polymère séquencé peut être choisie parmi :

a) une séquence ayant une Tg supérieure ou égale à 40 ˚C,
b) une séquence ayant une Tg inférieure ou égale à 20 ˚C,
c) une séquence ayant une Tg comprise entre 20 et 40 ˚C,

et la deuxième séquence choisie dans une catégorie a), b) ou c) différente de la première séquence.

On entend désigner dans la présente invention, par l'expression "compris entre ... et ... ", un intervalle de valeurs dont les bornes mentionnées sont exclues, et "de ... à ..." et "allant de ... à ...", un intervalle de valeurs dont les bornes sont inclues.

**a) Séquence ayant une Tg supérieure ou égale à 40 ˚C**

La séquence ayant une Tg supérieure ou égale à 40 ˚C a par exemple une Tg allant de 40 à 150 ˚C, en particulier supérieure ou égale à 50 ˚C, allant par exemple de 50 ˚C à 120 ˚C, et en particulier supérieure ou égale à 60 ˚C, allant par exemple de 60 ˚C à 120 ˚C.

La séquence ayant une Tg supérieure ou égale à 40˚C peut être un homopolymère ou un copolymère.

Dans le cas où cette séquence est un homopolymère, elle est issue de monomères, qui sont tel(s) que les homopolymères préparés à partir de ces monomères ont des températures de transition vitreuse supérieures ou égales à 40 ˚C. Cette première séquence peut être un homopolymère, constitué par un seul type de monomère (dont la Tg de l'homopolymère correspondant est supérieure ou égale à 40 ˚C).

Dans le cas où la première séquence est un copolymère, elle peut être issue en totalité ou en partie de un ou de plusieurs monomères, dont la nature et la concentration sont choisies de façon que la Tg du copolymère résultant soit supérieure ou égale à 40˚C. Le copolymère peut par exemple comprendre :

- des monomères qui sont tel(s) que les homopolymères préparés à partir de ces monomères ont des Tg supérieures ou égales à 40 ˚C, par exemple une Tg allant de 40 à 150 ˚C, en particulier supérieure ou égale à 50 ˚C, allant par exemple de 50 ˚C à 120˚C, et en particulier supérieure ou égale à 60 ˚C, allant par exemple de 60 ˚C à 120˚C, et
- des monomères qui sont tel(s) que les homopolymères préparés à partir de ces monomères ont des Tg inférieures à 40 ˚C, choisis parmi les monomères ayant une Tg comprise entre 20 à 40 ˚C et/ou les monomères ayant une Tg inférieure ou égale à 20 ˚C, par exemple une Tg allant de -100 à 20 ˚C, en particulier inférieure à 15 ˚C, notamment allant de - 80 ˚C à 15˚C et en particulier inférieur à 10˚C, par exemple allant de -50˚C à 0 ˚C à, tels que décrits plus loin.

Les monomères dont les homopolymères ont une température de transition vitreuse supérieure ou égale à 40 ˚C sont, de préférence, choisis parmi les monomères suivants, appelés aussi monomères principaux :

- les méthacrylates de formule (XII) :

$$CH_2 = C(CH_3)\text{-}COOR_1 \qquad (XII)$$

dans laquelle $R_1$ représente un groupe alkyle non substitué, linéaire ou ramifié, contenant de 1 à 4 atomes de carbone, tel qu'un groupe méthyle, éthyle, propyle ou isobutyle ou $R_1$ représente un groupe cycloalkyle $C_4$ à $C_{12}$,

- les acrylates de formule (XIII) :

$$CH_2 = CH\text{-}COOR_2 \qquad (XIII)$$

dans laquelle $R_2$ représente un groupe cycloalkyle en $C_4$ à $C_{12}$ tel que l'acrylate d'isobornyle ou un groupe tertio butyle,

- les (méth)acrylamides de formule (XIV) :

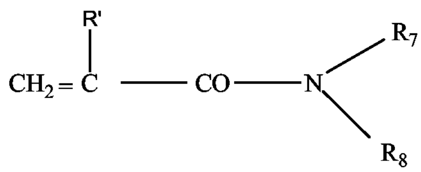

(XIV)

où :

- $R_7$ et $R_8$ identiques ou différents représentent chacun un atome d'hydrogène ou un groupe alkyle en $C_1$ à $C_{12}$ linéaire ou ramifié, tel qu'un groupe n-butyle, t-butyle, isopropyle, isohexyle, isooctyle, ou isononyle ; ou $R_7$ représente H et $R_8$ représente un groupement 1,1-diméthyl-3-oxobutyl, et
- R' désigne H ou méthyle,
- et leurs mélanges.

Comme exemple de monomères, on peut citer le N-butylacrylamide, le N-t-butylacrylamide, le N-isopropylacrylamide, le N,N-diméthylacrylamide et le N,N-dibutylacrylamide.
Des monomères principaux particulièrement avantageux sont le méthacrylate de méthyle, le (méth)acrylate d'isobutyle, le (méth)acrylate d'isobornyle et leurs mélanges.

**b) <u>Séquence avant une Tg inférieure ou égale à 20 °C</u>**

La séquence ayant une Tg inférieure ou égale à 20 °C a par exemple une Tg allant de -100 à 20 °C, de préférence inférieure ou égale à 15 °C, notamment allant de -80 °C à 15 °C et mieux inférieure ou égale à 10 °C, par exemple allant de -50 °C à 0 °C.
La séquence ayant une Tg inférieure ou égale à 20°C peut être un homopolymère ou un copolymère.
Dans le cas où cette séquence est un homopolymère, elle est issue de monomères, qui sont tel(s) que les homopolymères préparés à partir de ces monomères ont des températures de transition vitreuse inférieures ou égales à 20°C. Cette deuxième séquence peut être un homopolymère, constitué par un seul type de monomère (dont la Tg de l'homopolymère correspondant est inférieure ou égale à 20°C).
Dans le cas où la séquence ayant une Tg inférieure ou égale à 20°C est un copolymère, elle peut être issue en totalité ou en partie de un ou de plusieurs monomères, dont la nature et la concentration sont choisis de façon que la Tg du copolymère résultant soit inférieure ou égale à 20°C.
Elle peut par exemple comprendre

- un ou plusieurs monomères dont l'homopolymère correspondant a une Tg inférieure ou égale à 20 °C, par exemple une Tg allant de -100 °C à 20 °C, en particulier inférieure à 15 °C, notamment allant de -80 °C à 15 °C et en particulier inférieur à 10 °C, par exemple allant de -50 °C à 0 °C et
- un ou plusieurs monomères dont l'homopolymère correspondant a une Tg supérieure à 20 °C, tels que les monomères ayant une Tg supérieure ou égale à 40 °C, par exemple une Tg allant de 40 à 150 °C, en particulier supérieure ou égale à 50 °C, allant par exemple de 50 °C à 120 °C, et en particulier supérieure ou égale à 60 °C, allant par exemple de 60 °C à 120 °C et /ou les monomère ayant une Tg comprise entre 20 et 40 °C, tels que décrits plus haut.

En particulier, la séquence ayant une Tg inférieure ou égale à 20 °C est un homopolymère.
Les monomères dont l'homopolymère a une Tg inférieure ou égale à 20 °C sont, de préférence, choisis parmi les monomères suivants, ou monomère principaux :

- les acrylates de formule (XV) :

$$CH_2 = CHCOOR_3 \qquad (XV)$$

$R_3$ représentant un groupe alkyle non substitué en $C_1$ à $C_{12}$, linéaire ou ramifié, à l'exception du groupe tertiobutyle, dans lequel se trouve(nt) éventuellement intercalé(s) un ou plusieurs hétéroatomes choisis parmi O, N, S,
- les méthacrylates de formule (XVI) :

$$CH_2 = C(CH_3)\text{-}COOR_4 \qquad (XVI)$$

$R_4$ représentant un groupe alkyle non substitué en $C_6$ à $C_{12}$ linéaire ou ramifié, dans lequel se trouve(nt) éventuellement intercalé(s) un ou plusieurs hétéroatomes choisis parmi 0, N et S;
- les esters de vinyle de formule (XVII) :

$$R_5\text{-CO-O-CH} = CH_2 \qquad (XVII)$$

où $R_5$ représente un groupe alkyle en $C_4$ à $C_{12}$ linéaire ou ramifié ;
- les éthers d'alcool vinylique et d'alcool en $C_4$ à $C_{12}$,
- les N-alkyl en $C_4$ à $C_{12}$ acrylamides, tels que le N-octylacrylamide,
- et leurs mélanges.

Les monomères principaux particulièrement préférés pour la séquence ayant une Tg inférieure ou égale à 20 ˚C sont les acrylates d'alkyles dont la chaîne alkyle comprend de 1 à 10 atomes de carbone, à l'exception du groupe tertiobutyle, tels que l'acrylate de méthyle, l'acrylate d'isobutyle, l'acrylate d'éthyl-2 hexyle et leurs mélanges.

**c) Séquence ayant une Tg comprise entre 20 et 40˚C**

La séquence qui a une Tg comprise entre 20 et 40 ˚C peut être un homopolymère ou un copolymère.
Dans le cas où cette séquence est un homopolymère, elle est issue de monomères (ou monomère principaux), qui sont tel(s) que les homopolymères préparés à partir de ces monomères ont des températures de transition vitreuse comprises entre 20 et 40 ˚C. Cette première séquence peut être un homopolymère, constitué par un seul type de monomère (dont la Tg de l'homopolymère correspondant va de 20 ˚C à 40 ˚C).
Les monomères dont l'homopolymère a une température de transition vitreuse comprise entre 20 et 40 ˚C sont, de préférence, choisis parmi le méthacrylate de n-butyle, l'acrylate de cyclodécyle, l'acrylate de néopentyle, l'isodécylacrylamide et leurs mélanges.
Dans le cas où la séquence ayant une Tg comprise entre 20 et 40˚C est un copolymère, elle est issue en totalité ou en partie de un ou de plusieurs monomères (ou monomère principaux), dont la nature et la concentration sont choisis de telle sorte que la Tg du copolymère résultant soit comprise entre 20 et 40 ˚C.
Avantageusement, la séquence ayant une Tg comprise entre 20 et 40 ˚C est un copolymère issu en totalité ou en partie :

- de monomères principaux dont l'homopolymère correspondant a une Tg supérieure ou égale à 40 ˚C, par exemple une Tg allant de 40 ˚C à 150 ˚C, en particulier supérieure ou égale à 50 ˚C, allant par exemple de 50 à 120 ˚C, et mieux supérieure ou égale à 60 ˚C, allant par exemple de 60 ˚C à 120 ˚C, tels que décrits plus haut, et/ou
- de monomères principaux dont l'homopolymère correspondant a une Tg inférieure ou égale à 20 ˚C, par exemple une Tg allant de -100 à 20 ˚C, en particulier inférieure ou égale à 15 ˚C, notamment allant de -80 ˚C à 15 ˚C et en particulier inférieure ou égale à 10 ˚C, par exemple allant de -50 ˚C à 0 ˚C, tels que décrits plus haut,
  lesdits monomères étant choisis de telle sorte que la Tg du copolymère formant la première séquence est comprise entre 20 et 40 ˚C.

De tels monomères principaux sont par exemple choisis parmi le méthacrylate de méthyle, l'acrylate et le méthacrylate d'isobornyle, l'acrylate de butyle, l'acrylate d'éthyl-2 hexyle et leurs mélanges.
Plus particulièrement, la proportion de la deuxième séquence ayant une Tg inférieure ou égale à 20 ˚C va de 10 à 85 % en poids du polymère, mieux de 20 à 70 % et encore mieux de 20 à 50 %.
Chacune des séquences peut néanmoins contenir en proportion minoritaire au moins un monomère constitutif de l'autre séquence.
Ainsi la première séquence peut contenir au moins un monomère constitutif de la deuxième séquence et inversement.

Chacune des première et/ou deuxième séquence du polymère séquencé peu(ven)t comprendre, outre les monomères indiqués ci-dessus, un ou plusieurs autres monomères appelés monomères additionnels, différents des monomères principaux cités précédemment.

La nature et la quantité de ce ou ces monomères additionnels sont choisies de manière à ce que la séquence dans laquelle ils se trouvent ait la température de transition vitreuse désirée.

*c) Monomère additionnel*

Ce monomère additionnel est par exemple choisi parmi :

- les monomères hydrophiles tels que :
  - les monomères à insaturation(s) éthylénique(s) comprenant au moins une fonction acide carboxylique ou sulfonique comme par exemple :
    - l'acide acrylique, l'acide méthacrylique, l'acide crotonique, l'anhydride maléique, l'acide itaconique, l'acide fumarique, l'acide maléique, l'acide acrylamidopropanesulfonique, l'acide vinylbenzoïque, l'acide vinylphosphorique et les sels de ceux-ci,
  - les monomères à insaturation(s) éthylénique(s) comprenant au moins une fonction amine tertiaire comme :
    - la 2-vinylpyridine, la 4-vinylpyridine, le méthacrylate de diméthylaminoéthyle, le méthacrylate de diéthylaminoéthyle, le diméthylaminopropyl méthacrylamide et les sels de ceux-ci,
    - les méthacrylates de formule (XVIII) :

$$CH_2 = C(CH_3)\text{-}COOR_6 \qquad (XVIII)$$

dans laquelle $R_6$ représente un groupe alkyle linéaire ou ramifié, contenant de 1 à 4 atomes de carbone, tel qu'un groupe méthyle, éthyle, propyle ou isobutyle, ledit groupe alkyle étant substitué par un ou plusieurs substituants choisis parmi les groupes hydroxyle (comme le méthacrylate de 2-hydroxypropyle, le méthacrylate de 2-hydroxyéthyle) et les atomes d'halogènes (Cl, Br, I, F), tel que le méthacrylate de trifluoroéthyle,

    - les méthacrylates de formule (XIX) :

$$CH_2 = C(CH_3)\text{-}COOR_9 \qquad (XIX)$$

dans laquelle $R_9$ représentant un groupe alkyle en $C_6$ à $C_{12}$ linéaire ou ramifié, dans lequel se trouve(nt) éventuellement intercalé(s) un ou plusieurs hétéroatomes choisis parmi 0, N et S, ledit groupe alkyle étant substitué par un ou plusieurs substituants choisis parmi les groupes hydroxyle et les atomes d'halogènes (Cl, Br, I, F) ;

    - les acrylates de formule (XX) :

$$CH_2 = CHCOOR_{10} \qquad (XX)$$

dans laquelle $R_{10}$ représentant un groupe alkyle en $C_1$ à $C_{12}$ linéaire ou ramifié substitué par un ou plusieurs substituants choisis parmi les groupes hydroxyle et les atomes d'halogène (Cl, Br, 1 et F), tel que l'acrylate de 2-hydroxypropyle et l'acrylate de 2-hydroxyéthyle, ou $R_{10}$ représente un alkyle en $C_1$ à $C_{12}$-O-POE (polyoxyéthylène) avec répétition du motif oxyéthylène de 5 à 30 fois, par exemple méthoxy-POE, ou $R_8$ représente un groupement polyoxyéthylèné comprenant de 5 à 30 motifs d'oxyde d'éthylène.

  - les monomères à insaturation éthylénique comprenant un ou plusieurs atomes de silicium tels que le méthacryloxypropyl triméthoxy silane, le méthacryloxypropyl tris (triméthylsiloxy ) silane,
  - et leurs mélanges.

Des monomères additionnels particulièrement préférés sont l'acide acrylique, l'acide méthacrylique, le méthacrylate de trifluoroéthyle et leurs mélanges.

Selon un mode particulier de réalisation, le polymère séquencé est un polymère non siliconé, c'est à dire un polymère exempt d'atome de silicium.

Ce ou ces monomères additionnels représente(nt) généralement une quantité inférieure ou égale à 30% en poids, par exemple de 1 à 30% en poids, de préférence de 5 à 20% en poids et, de préférence encore, de 7 à 15% en poids du poids total des première et/ou deuxième séquences.

En particulier, chacune des première et deuxième séquences comprend au moins un monomère choisi parmi les esters d'acide (méth)acrylique, et éventuellement au moins un monomère choisi parmi l'acide (méth)acrylique, et leurs mélanges.

Avantageusement, chacune des première et deuxième séquences du polymère séquencé est issue en totalité d'au moins un monomère choisi parmi l'acide acrylique, les esters d'acide (méth)acrylique, et éventuellement d'au moins un monomère choisi parmi l'acide (méth)acrylique, et leurs mélanges.

*d) Procédé d'obtention*

Le polymère séquencé peut être obtenu par polymérisation radicalaire en solution selon le procédé de préparation suivant :

- une partie du solvant de polymérisation est introduite dans un réacteur adapté et chauffée jusqu'à atteindre la température adéquate pour la polymérisation (typiquement entre 60 et 120 °C),
- une fois cette température atteinte, les monomères constitutifs de la première séquence sont introduits en présence d'une partie de l'initiateur de polymérisation,
- au bout d'un temps T correspondant à un taux de conversion maximum de 90 %, les monomères constitutifs de la deuxième séquence et l'autre partie de l'initiateur sont introduits,
- on laisse réagir le mélange pendant un temps T' (allant de 3 à 6 h) au bout duquel le mélange est ramené à température ambiante,
- on obtient le polymère en solution dans le solvant de polymérisation.

Par solvant de polymérisation, on entend un solvant ou un mélange de solvants. Le solvant de polymérisation peut être choisis notamment parmi l'acétate d'éthyle, l'acétate de butyle, les alcools tels que l'isopropanol, l'éthanol, les alcanes aliphatiques tels que l'isododécane et leurs mélanges. De manière particulière, le solvant de polymérisation est un mélange acétate de butyle et isopropanol ou l'isododécane.

Selon un mode particulier de réalisation, le polymère séquencé comprend une première séquence ayant une Tg supérieure ou égale à 40 °C, telle que décrite plus haut au a) et une deuxième séquence ayant une Tg inférieure ou égale à 20 °C, telle que décrite plus haut au b).

En particulier, la première séquence ayant une Tg supérieure ou égale à 40 °C est un copolymère issu de monomères qui sont tels que l'homopolymère préparé à partir de ces monomères a une température de transition vitreuse supérieure ou égale à 40 °C, tels que les monomère décrits plus haut.

Avantageusement, la deuxième séquence ayant une Tg inférieure ou égale à 20 °C est un homopolymère issu de monomères qui sont tel(s) que l'homopolymère préparé à partir de ces monomères a une température de transition vitreuse inférieure ou égale à 20 °C, tels que les monomères décrits plus haut.

En particulier, la proportion de la séquence ayant une Tg supérieure ou égale à 40 °C va de 20 à 90 % en poids du polymère, mieux de 30 à 80 % et encore mieux de 50 à 70%.

En particulier, la proportion de la séquence ayant une Tg inférieure ou égale à 20 °C va de 5 à 75 % en poids du polymère, de préférence de 15 à 50 % et mieux de 25 à 45 %.

Avantageusement, le polymère séquencé peut comprendre :

- une première séquence de Tg supérieure ou égale à 40 °C, par exemple allant de 85 à 115 °C, qui est un copolymère acrylate d'isobornyle/méthacrylate d'isobutyle,
- une deuxième séquence de Tg inférieure ou égale à 20 °C, par exemple allant de -85 à -55 °C, qui est un homopolymère d'acrylate d'éthyl-2 hexyle et
- une séquence intermédiaire qui est un copolymère statistique acrylate d'isobornyle/méthacrylate d'isobutyle/acrylate d'éthyl-2 hexyle.

Selon un autre mode de réalisation, le polymère séquencé comprend une première séquence ayant une température de transition vitreuse (Tg) comprise entre 20 et 40 °C, conforme aux séquences décrites c) et une deuxième séquence ayant une température de transition vitreuse inférieure ou égale à 20 °C, telle que décrite plus haut au b) ou une température de transition vitreuse supérieure ou égale à 40 °C, telle que décrite ci-dessus.

En particulier, la proportion de la première séquence ayant une Tg comprise entre 20 et 40 °C va de 10 à 85 % en poids du polymère, en particulier de 30 à 80 % et encore mieux de 50 à 70 %.

Lorsque la deuxième séquence est une séquence ayant une Tg supérieure ou égale à 40 °C, elle est en particulier présente en une proportion allant de 10 à 85 % en poids du polymère, en particulier de 20 à 70 % et plus particulièrement de 30 à 70 %.

Lorsque la deuxième séquence est une séquence ayant une Tg inférieure ou égale à 20 °C, elle est en particulier présente en une proportion allant de 10 à 85 % en poids du polymère, en particulier de 20 à 70 % et plus particulièrement de 20 à 50 %.

En particulier, la première séquence ayant une Tg comprise entre 20 et 40 °C est un copolymère issu de monomères qui sont tel(s) que l'homopolymère correspondant a une Tg supérieure ou égale à 40 °C et de monomères qui sont tel(s) que l'homopolymère correspondant a une Tg inférieure ou égale à 20 °C.

Avantageusement, la deuxième séquence ayant une Tg inférieure ou égale à 20 °C ou ayant une Tg supérieure ou égale à 40 °C est un homopolymère.

Selon une première variante, le polymère séquencé comprend :

- une première séquence de Tg comprise entre 20 et 40 °C, par exemple ayant une Tg de 21 à 39 °C, qui est un copolymère comprenant acrylate d'isobornyle/méthacrylate d'isobutyle/acrylate d'éthyl-2 hexyle,
- une deuxième séquence de Tg inférieure ou égale à 20 °C, par exemple allant de -65 à -35 °C, qui est un homopolymère de méthacrylate de méthyle et
- une séquence intermédiaire qui est un copolymère statistique acrylate d'isobornyle /méthacrylate d'isobutyle/acrylate d'éthyl-2 hexyle.

Selon une autre variante, le polymère séquencé peut comprendre :

- une première séquence de Tg supérieure ou égale à 40 °C, par exemple allant de 85 à 115 °C, qui est un copolymère méthacrylate d'isobornyle/méthacrylate d'isobutyle,
- une deuxième séquence de Tg inférieure ou égale à 20 °C, par exemple allant de -35 à -5 °C, qui est un homopolymère d'acrylate d'isobutyle et
- une séquence intermédiaire qui est un copolymère statistique méthacrylate d'isobornyle/méthacrylate d'isobutyle/acrylate d'isobutyle.

Selon encore une autre variante, le polymère séquencé peut comprendre :

- une première séquence de Tg supérieure ou égale à 40 °C, par exemple allant de 60 à 90 °C, qui est un copolymère acrylate d'isobornyle/méthacrylate d'isobutyle,
- une deuxième séquence de Tg inférieure ou égale à 20 °C, par exemple allant de -35 à -5 °C, qui est un homopolymère d'acrylate d'isobutyle et
- une séquence intermédiaire qui est un copolymère statistique acrylate d'isobornyle/méthacrylate d'isobutyle/acrylate d'isobutyle.

## II. <u>Filmogène soluble dans la phase grasse liquide</u>

Dans un mode de réalisation, l'agent filmogène est un polymère organique filmogène soluble dans la phase grasse liquide.

Lorsque la phase grasse liquide de la composition comprend au moins une huile, l'agent filmogène peut être un polymère soluble dans ladite huile. Dans ce cas, on parle de polymère liposoluble. Le polymère liposoluble peut être d'un type chimique quelconque et peut être notamment choisi parmi :

a) les homopolymères et les copolymères liposolubles et amorphes des oléfines, des cyclooléfines, du butadiène, de l'isoprène, du styrène, des éthers, des esters ou amides vinyliques, des esters ou amides de l'acide (méth)acrylique contenant un groupement alkyle en $C_{4-50}$ linéaire, ramifié ou cyclique, et en particulier amorphes. Les homopolymères et les copolymères liposolubles préférés sont obtenus à partir de monomères choisis parmi le groupe constitué par le (méth)acrylate d'isooctyle, le (méth)acrylate d'isononyle, le (méth)acrylate de 2-éthylhexyle, le (méth)acrylate de lauryle, le (méth)acrylate d'isopentyle, le (méth)acrylate de n-butyle, le (méth)acrylate d'isobutyle, le (méth)acrylate de méthyle, le (méth)acrylate de tertio-butyle, le (méth)acrylate de tridécyle, le (méth)acrylate de stéaryle, ou des mélanges de ceux-ci. On citera, par exemple, le copolymère d'acrylate d'alkyle/acrylate de cycloalkyle commercialisé par PHOENIX CHEM. sous la dénomination GIOVAREZ AC-5099 ML, et les copolymères de vinylpyrrolidone, tels que les copolymères d'un alkène en $C_2$ à $C_{30}$, tel qu'en $C_3$ à $C_{22}$, et des associations de

ceux-ci, peuvent être utilisés. Comme exemples de copolymères de VP pouvant être utilisés dans l'invention, on peut citer le copolymère de VP/laurate de vinyle, de VP/stéarate de vinyle, la polyvinylpyrrolidone (PVP) butylée, de VP/hexadécène, de VP/triacontène ou de VP/acide acrylique/méthacrylate de lauryle.

Comme copolymères liposolubles particuliers, on peut citer :

- i) les polymères de silicone-acrylique greffés ayant un squelette siliconé, des greffons acryliques ou ayant un squelette acrylique, les greffons de silicone tels que le produit commercialisé sous la dénomination SA 70.5 par 3M et décrit dans les brevets US 5 725 882, US 5 209 924, US 4 972 037, US 4 981 903, US 4 981 902, US 5 468 477, et dans les brevets US 5 219 560 et EP 0 388 582,
- ii) les polymères liposolubles portant des groupements fluorés appartenant à l'une des classes décrites dans le texte ci-dessus, en particulier FOMBLIN, ceux décrits dans le brevet US 5 948 393, les copolymères de (méth)acrylate d'alkyle/(méth)acrylate de perfluoroalkyle décrits dans les brevets EP 0 815 836 et US 5 849 318,
- iii) les polymères ou copolymères résultant de la polymérisation ou la copolymérisation d'un monomère éthylénique, comprenant une ou plusieurs liaisons éthyléniques, de préférence conjuguées (ou diènes). Comme polymères ou copolymères résultant de la polymérisation ou la copolymérisation d'un monomère éthylénique, on peut utiliser des copolymères vinyliques, acryliques ou méthacryliques.

Dans un mode de réalisation, l'agent filmogène est un copolymère bloc comprenant au moins un bloc constitué de motifs styrène ou dérivés du styrène (par exemple le méthylstyrène, le chlorostyrène ou le chlorométhylstyrène). Le copolymère comprenant au moins un bloc styrène peut être un copolymère dibloc ou tribloc, voire un copolymère multibloc, en étoile ou radial. Le copolymère comprenant au moins un bloc styrène peut comprendre en outre, par exemple, un bloc alkylstyrène (AS), un bloc éthylène/butylène (EB) un bloc éthylène/ propylène (EP), un bloc butadiène (B), un bloc isoprène (I), un bloc acrylate (A), un bloc méthacrylate (MA) ou une association de ces blocs. Le copolymère comprenant au moins un bloc constitué de motifs styrène ou dérivés du styrène peut être un copolymère dibloc ou tribloc, et en particulier du type polystyrène/polyisoprène ou polystyrène/polybutadiène, tels que ceux commercialisés ou fabriqués sous la dénomination « LUVITOL HSB » par BASF et ceux du type polystyrène/copoly(éthylène-propylène) ou de manière alternative du type polystyrène/copoly(éthylène/butylène), tels que ceux commercialisés ou fabriqués sous la marque de fabrique « KRATON » par SHELL CHEMICAL CO. ou GELLED PERMETHYL 99A par PENRECO, peuvent être utilisés.

On peut citer par exemple le KRATON G1650 (SEBS), le KRATON G1651 (SEBS), le KRATON G1652 (SEBS), le KRATON G1657X (SEBS), le KRATON G1701X (SEP), le KRATON G1702X (SEP), le KRATON G1726X (SEB), le KRATON D-1101 (SBS), le KRATON D-1102 (SBS), le KRATON D-1107 (SIS), le GELLED PERMETHYL 99A-750, le GELLED PERMETHYL 99A-753-58 (mélange de polymère bloc en étoile et de polymère tribloc), le GELLED PERMETHYL 99A-753-59 (mélange de polymère bloc en étoile et de polymère tribloc), le VERSAGEL 5970 et le VERSAGEL 5960 de chez PENRECO (mélange de polymère en étoile et de polymère tribloc dans l'isododécane).

Des copolymères de styrène-méthacrylate peuvent également être utilisés tels que les polymère commercialisés sous les référenceq OS 129880, OS 129881 et OS 84383 de chez LUBRIZOL (copolymère de styrène-méthacrylate).

Dans un mode de réalisation, l'agent filmogène est choisi parmi les copolymères d'ester vinylique (le groupe vinylique étant directement relié à l'atome d'oxygène du groupe ester et l'ester vinylique ayant un radical hydrocarboné saturé, linéaire ou ramifié, de 1 à 19 atomes de carbone, lié au carbonyle du groupe ester) et d'au moins un autre monomère qui peut être un ester vinylique (différent de l'ester vinylique déjà présent), une $\alpha$-oléfine (ayant de 8 à 28 atomes de carbone), un alkylvinyléther (dont le groupe alkyl comporte de 2 à 18 atomes de carbone), ou un ester allylique ou méthallylique (ayant un radical hydrocarboné saturé, linéaire ou ramifié, de 1 à 19 atomes de carbone, lié au carbonyle du groupe ester).

Ces copolymères peuvent être partiellement réticulés à l'aide de réticulants qui peuvent être soit du type vinylique, soit du type allylique ou méthallylique, tels que le tétraallyloxyéthane, le divinylbenzène, l'octanedioate de divinyle, le dodécanedioate de divinyle, et l'octadécanedioate de divinyle.

Comme exemples de ces copolymères, on peut citer les copolymères : acétate de vinyle/stéarate d'allyle, l'acétate de vinyle/laurate de vinyle, acétate de vinyle/stéarate de vinyle, acétate de vinyle/octadécène, acétate de vinyle/octadécylvinyléther, propionate de vinyle/laurate d'allyle, propionate de vinyle/laurate de vinyle, stéarate de vinyle/octadécène-1, acétate de vinyle/dodécène-1, stéarate de vinyle/éthylvinyléther, propionate de vinyle/cétyl vinyle éther, stéarate de vinyle/acétate d'allyle, diméthyl-2, 2 octanoate de vinyle/laurate de vinyle, diméthyl-2, 2 pentanoate d'allyle/laurate de vinyle, diméthyl propionate de vinyle/stéarate de vinyle, diméthyl propionate d'allyle/stéarate de vinyle, propionate de vinyle/stéarate de vinyle, réticulé avec 0,2 % de divinyl benzène, diméthyl propionate de vinyle/laurate de vinyle, réticulé avec 0,2 % de divinyl benzène, acétate de vinyle/octadécyl vinyl éther, réticulé avec 0,2 % de tétraallyloxyéthane, acétate de vinyle/stéarate d'allyle, réticulé avec 0,2 % de divinyl benzène, acétate de vinyle/octadécène-1 réticulé avec 0,2 % de divinyl benzène et propionate d'allyle/stéarate d'allyle réticulé avec 0,2 % de divinyl benzène.

Comme polymères filmogènes liposolubles, on peut également citer les copolymères liposolubles, et en particulier ceux résultant de copolymérisation d'esters vinyliques ayant de 9 à 22 atomes de carbone ou d'acrylates ou de méthacrylates d'alkyle, les radicaux alkyles ayant de 10 à 20 atomes de carbone.

De tels copolymères liposolubles peuvent être choisis parmi les copolymères de polystéarate de vinyle, de polystéarate de vinyle réticulé à l'aide de divinylbenzène, de diallyléther ou de phtalate de diallyle, les copolymères de poly(méth)acrylate de stéaryle, de polylaurate de vinyle, de poly(méth)acrylate de lauryle, ces poly(méth)acrylates pouvant être réticulés à l'aide de diméthacrylate de l'éthylène glycol ou de tétraéthylène glycol.

Les copolymères liposolubles définis précédemment sont connus et notamment décrits dans la demande FR-A-2232303 ; ils peuvent avoir un poids moléculaire moyen en poids allant de 2000 à 500 000 et en particulier de 4000 à 200 000.

Comme exemples de polymères liposolubles pouvant être utilisés dans l'invention, on peut citer les polyalkylènes, les copolymères d'alcènes en $C_2$-$C_{20}$, en particulier le polybutène.

b) les polycondensats amorphes et liposolubles, en particulier ne comprenant pas de groupements donneurs d'interactions hydrogène, en particulier les polyesters aliphatiques ayant des chaînes latérales alkyle en $C_{4-50}$ ou bien les polyesters résultant de la condensation de dimères d'acides gras, voire les polyesters comprenant un segment siliconé sous la forme d'une séquence, greffon ou groupement terminal, tel que défini dans la demande de brevet FR 0 113 920, et

c) les polysaccharides amorphes et liposolubles comprenant des chaînes latérales alkyl (éther ou ester), en particulier les alkylcelluloses ayant un radical alkyle en $C_1$ à $C_8$ saturé ou insaturé, linéaire ou ramifié, tel que l'éthylcellulose et la propylcellulose.

Le polymère filmogène peut être choisi en particulier parmi les polymères cellulosiques tels que la nitrocellulose, l'acétate de cellulose, l'acétobutyrate de cellulose, l'acétopropionate de cellulose, l'éthyl cellulose, ou bien encore les polyuréthanes, les polymères acryliques, les polymères vinyliques, les polyvinylbutyrals, les résines alkydes, les résines issues des produits de condensation d'aldéhyde tels que les résines arylsulfonamide formaldéhyde comme la résine toluène sulfonamide formaldéhyde, les résines aryl-sulfonamide époxy.

Comme polymère filmogène, on peut notamment utiliser la nitrocellulose RS 1/8 sec ; RS × sec. ; ½ sec. ; RS 5 sec. ; RS 15 sec. ; RS 35 sec. ; RS 75 sec.; RS 150 sec ; AS × sec. ; AS ½sec. ; SS × sec. ; SS ½ sec. ; SS 5 sec., notamment commercialisée par la société HERCULES ; les résine toluène sulfonamide formaldéhyde "KETJENTFLEX MS80" de la société AKZO ou "SANTOLITE MHP", "SANTOLITE MS 80" de la société FACONNIER ou "RESIMPOL 80" de la société PAN AMERICANA, la résine alkyde "BECKOSOL ODE 230-70-E" de la société DAINIPPON, la résine acrylique "ACRYLOID B66" de la société ROHM & HAAS, la résine polyuréthane "TRIXENE PR 4127" de la société BAXENDEN.

d) les résines de silicone, généralement solubles ou gonflables dans les huiles de silicone. Ces résines sont des polymères de polyorganosiloxanes réticulés.

La nomenclature des résines de silicone est connue sous le nom de "MDTQ", la résine étant décrite en fonction des différentes unités monomèriques siloxane qu'elle comprend, chacune des lettres "MDTQ" caractérisant un type d'unité.

La lettre M représente l'unité monofonctionelle de formule $(CH_3)_3SiO_{½}$ , l'atome de silicium étant relié à un seul atome d'oxygène dans le polymère comprenant cette unité.

La lettre D signifie une unité difonctionnelle $(CH_3)_2SiO_{2/2}$ dans laquelle l'atome de silicium est relié à deux atomes d'oxygène

La lettre T représente une unité trifonctionnelle de formule $(CH_3)SiO_{3/2}$.

Dans les motifs M, D, T définis précédemment, au moins un des groupes méthyles peut être substitués par un groupe R différent du groupe méthyle tel qu'un radical hydrocarboné (notamment alkyle) ayant de 2 à 10 atomes de carbone ou un groupe phényl ou bien encore un groupe hydroxyle.

Enfin, la lettre Q signifie une unité tetrafonctionnelle $SiO_{4/2}$ dans laquelle l'atome de silicium est lié à quatre atomes d'hydrogène eux mêmes liés au reste du polymère.

Divers résines de propriétés différentes peuvent être obtenues à partir de ces différentes unités, les propriétés des ces polymères variant en fonction du type de monomères (ou unités), du type et du nombre de radicaux subsitués, de la longueur de la chaîne polymérique, du degré de ramification et de la taille des chaines pendantes.

A titre d'exemple de ces résines silicones, on peut citer :

- les siloxysilicates qui peuvent être des triméthylsiloxysilicate de formule (XXI) :

$$[(CH_3)_3\text{-Si-O}]_x\text{-}(SiO_{4/2})_y \qquad (XXI)$$

(unités MQ) dans laquelle x et y sont des entiers allant de 50 à 80,

- les polysilesquioxanes de formule $(CH_3SiO_{3/2})_{.x}$ (unités T) dans laquelle x est supérieur à 100 et dont au moins un des radicaux méthyle peut être substitué par un groupement R tel que défini plus haut,
- les polymethylsilsesquioxanes qui sont des polysilsesquioxanes dans lesquels aucun des radicaux méthyle n'est substitué par un autre groupement. De tels polymethylsilsesquioxanes sont décrits dans le document US 5,246,694 dont le contenu est incorporé par référence.

A titre d'exemples de résines polymethylsilsesquioxanes commercialement disponibles, on peut citer celles qui sont commercialisés :

- par la société WACKER sous la référence RESIN MK tels que la BELSIL PMS MK : polymère comprenant des unités répétitives $CH_3SiO_{3/2}$ (unités T), pouvant aussi comprendre jusqu'à 1% en poids d'unités $(CH_3)_2SiO_{2/2}$ (unités D) et présentant un poids moléculaire moyen d'environ 10000,
- par la société SHIN-ETSU sous les références KR-220L qui sont composé d'unités T de formule $CH_3SiO_{3/2}$ et ont des groupes terminaux Si-OH (silanol), sous la référence KR-242A qui comprennent 98% d'unités T et 2% d'unités diméthyle D et ont des groupes terminaux Si-OH ou encore sous la référence KR-251 comprenant 88 % d'unités T et 12 % d'unités dimethyl D et ont des groupes terminaux Si-OH.

Comme résines siloxysilicates, on peut citer les résines trimethylsiloxysilicate (TMS) éventuellement sous forme de poudres. De telles résines sont commercialisées sous la référence SR1000 par la société GENERAL ELECTRIC ou sous la référence TMS 803 par la société WACKER. On peut encore citer les résines timéthylsiloxysilicate commercialisées dans un solvant tel que la cyclomethicone, vendues sous la dénomination "KF-7312J" par la société SHIN-ETSU, "DC 749", "DC 593" par la société DOW CORNING.

e) Les polyamides siliconés du type polyorganosiloxane tels que ceux décrits dans les documents US-A-5 874 069, US-A-5,919,441, US-A-6,051,216 et US-A-5,981,680.

Selon l'invention, ces polymères siliconés peuvent appartenir aux deux familles suivantes :

1) des polyorganosiloxanes comportant au moins deux groupes capables d'établir des interactions hydrogène, ces deux groupes étant situés dans la chaîne du polymère, et/ou
2) des polyorganosiloxanes comportant au moins deux groupes capables d'établir des interactions hydrogène, ces deux groupes étant situés sur des greffons ou ramifications.

Les polymères comportant deux groupes capables d'établir des interactions hydrogène dans la chaîne du polymère peuvent être des polymères comprenant au moins un motif répondant à la formule (XXII) :

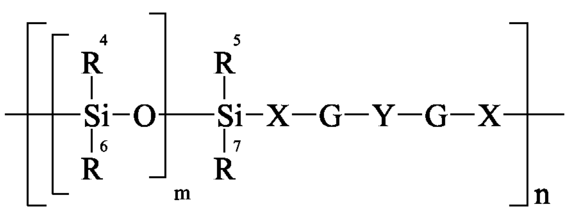

(XXII)

dans laquelle :

1) $R^4$, $R^5$, $R^6$ et $R^7$, identiques ou différents, représentent un groupe choisi parmi :

- les groupes hydrocarbonés, linéaires, ramifiés ou cycliques, en $C_1$ à $C_{40}$, saturés ou insaturés, pouvant contenir dans leur chaîne un ou plusieurs atomes d'oxygène, de soufre et/ou d'azote, et pouvant être substitués en partie ou totalement par des atomes de fluor,
- les groupes aryles en $C_6$ à $C_{10}$, éventuellement substitués par un ou plusieurs groupes alkyles en $C_1$ à $C_4$,
- les chaînes polyorganosiloxanes contenant ou non un ou plusieurs atomes d'oxygène, de soufre et/ou d'azote,

2) les X, identiques ou différents, représentent un groupe alkylène di-yle, linéaire ou ramifié en $C_1$ à $C_{30}$, pouvant contenir dans sa chaîne un ou plusieurs atomes d'oxygène et/ou d'azote,

3) Y est un groupe divalent alkylène linéaire ou ramifié, arylène, cycloalkylène, alkylarylène ou arylalkylène, saturé ou insaturé, en $C_1$ à $C_{50}$, pouvant comporter un ou plusieurs atomes d'oxygène, de soufre et/ou d'azote, et/ou porter comme substituant l'un des atomes ou groupes d'atomes suivants : fluor, hydroxy, cycloalkyle en $C_3$ à $C_8$, alkyle en $C_1$ à $C_{40}$, aryle en $C_5$ à $C_{10}$, phényle éventuellement substitué par 1 à 3 groupes alkyle en $C_1$ à $C_3$, hydroxyalkyle en $C_1$ à $C_3$ et amino alkyle en $C_1$ à $C_6$, ou

4) Y représente un groupe répondant à la formule (XXIII) :

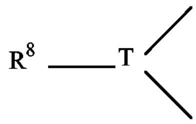
(XXIII)

dans laquelle

- T représente un groupe hydrocarboné trivalent ou tétravalent, linéaire ou ramifié, saturé ou insaturé, en $C_3$ à $C_{24}$ éventuellement substitué par une chaîne polyorganosiloxane, et pouvant contenir un ou plusieurs atomes choisis parmi O, N et S, ou T représente un atome trivalent choisi parmi N, P et Al, et
- $R^8$ représente un groupe alkyle en $C_1$ à $C_{50}$, linéaire ou ramifié, ou une chaîne polyorganosiloxane, pouvant comporter un ou plusieurs groupes ester, amide, uréthane, thiocarbamate, urée, thiourée et/ou sulfonamide qui peut être lié ou non à une autre chaîne du polymère,

5) les G, identiques ou différents, représentent les groupes divalents choisis parmi :

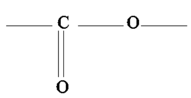 ; 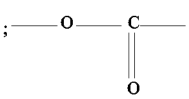 ; 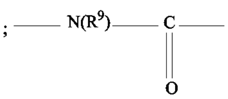 ;

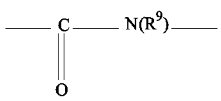 ; 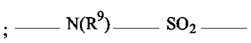 ; 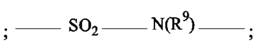 ;

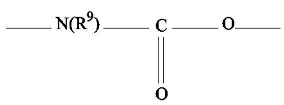 ; 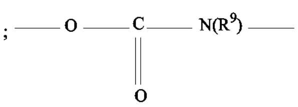 ; 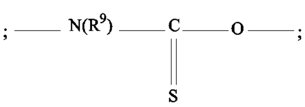 ;

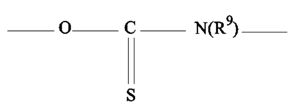 ; 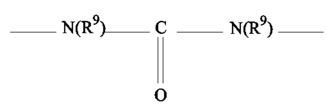 ,

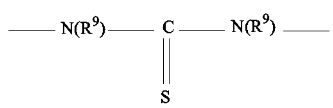 ,

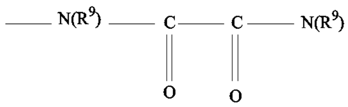 ; 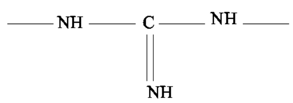 ; et

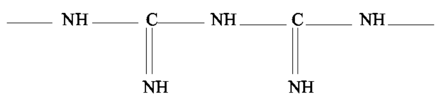

où $R^9$ représente un atome d'hydrogène ou un groupe alkyle, linéaire ou ramifié, en $C_1$ à $C_{20}$, à condition qu'au moins 50% des $R^9$ du polymère représente un atome d'hydrogène et qu'au moins deux des groupes G du polymère soient un autre groupe que :

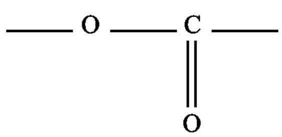 et 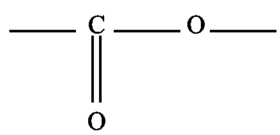 ;

6) n est un nombre entier allant de 2 à 500, en particulier de 2 à 200, et m est un nombre entier allant de 1 à 1000, en particulier de 1 à 700 et mieux encore de 6 à 200.

Selon l'invention, 80 % des $R^4$, $R^5$, $R^6$ et $R^7$, du polymère sont choisis de préférence parmi les groupes méthyle, éthyle, phényle et 3,3,3-trifluoropropyle.

Selon l'invention, Y peut représenter divers groupes divalents, comportant éventuellement de plus une ou deux valences libres pour établir des liaisons avec d'autres motifs du polymère ou copolymère. En particulier, Y représente un groupe choisi parmi :

a) les groupes alkylène linéaires en $C_1$ à $C_{20}$, de préférence en $C_1$ à $C_{10}$,
b) les groupes alkylène ramifiés pouvant comporter des cycles et des insaturations non conjuguées, en $C_{30}$ à $C_{56}$,
c) les groupes cycloalkylène en $C_5$-$C_6$,
d) les groupes phénylène éventuellement substitués par un ou plusieurs groupes alkyle en $C_1$ à $C_{40}$,
e) les groupes alkylène en $C_1$ à $C_{20}$, comportant de 1 à 5 groupes amides,
f) les groupes alkylène en $C_1$ à $C_{20}$, comportant un ou plusieurs substituants, choisis parmi les groupes hydroxyle, cycloalcane en $C_3$ à $C_8$, hydroxyalkyle en $C_1$ à $C_3$ et alkylamines en $C_1$ à $C_6$,
g) les chaînes polyorganosiloxane de formule (XXIV) :

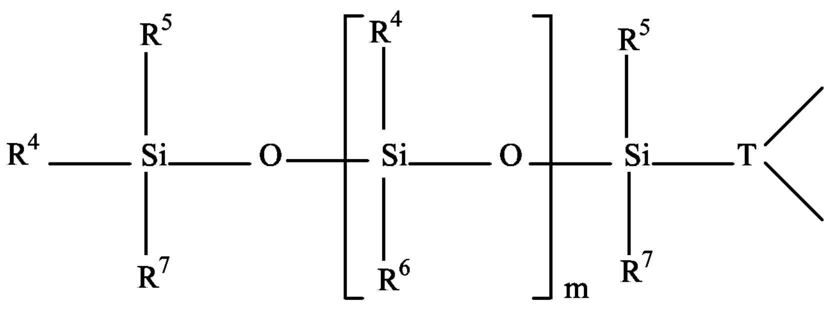

(XXIV)

dans laquelle $R^4$, $R^5$, $R^6$, $R^7$, T et m sont tels que définis ci-dessus, et
h) les chaînes polyorganosiloxanes de formule (XXV) :

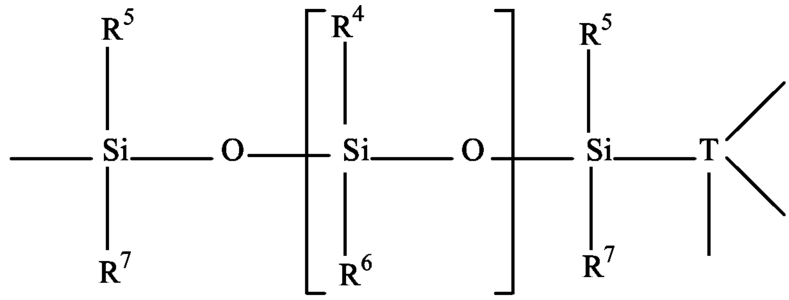

(XXV)

Les polyorganosiloxanes de la seconde famille peuvent être des polymères comprenant au moins un motif répondant à la formule (XXVI) :

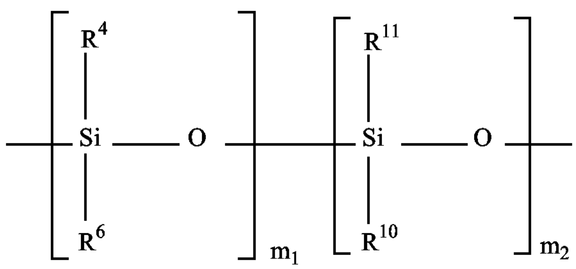

(XXVI)

dans laquelle :

- $R^4$ et $R^6$, identiques ou différents, sont tels que définis ci-dessus pour la formule (XXII),
- $R^{10}$ représente un groupe tel que défini ci-dessus pour $R^4$ et $R^6$, ou représente le groupe de formule $-X-G-R^{12}$ dans laquelle X et G sont tels que définis ci-dessus pour la formule (XXII) et $R^{12}$ représente un atome d'hydrogène ou un groupe hydrocarboné, linéaire, ramifié ou cyclique, saturé ou insaturé, en $C_1$ à $C_{50}$ comportant éventuellement dans sa chaîne un ou plusieurs atomes choisis parmi O, S et N, éventuellement substitué par un ou plusieurs atomes de fluor et/ou un ou plusieurs groupes hydroxyle, ou un groupe phényle éventuellement substitué par un ou plusieurs groupes alkyle en $C_1$ à $C_4$,
- $R^{11}$ représente le groupe de formule $-X-G-R^{12}$ dans laquelle X, G et $R^{12}$ sont tels que définis ci-dessus,
- $m_1$ est un nombre entier allant de 1 à 998, et
- $m_2$ est un nombre entier allant de 2 à 500.

Selon l'invention, le polymère utilisé, peut être un homopolymère, c'est-à-dire un polymère comportant plusieurs motifs identiques, en particulier des motifs de formule (XXII) ou de formule (XXVI).

Selon l'invention, on peut aussi utiliser un polymère constitué par un copolymère comportant plusieurs motifs de formule (XXII) différents, c'est-à-dire un polymère dans lequel l'un au moins des $R^4$, $R^5$, $R^6$, $R^7$, X, G, Y, m et n est différent dans l'un des motifs. Le copolymère peut être aussi formé de plusieurs motifs de formule (XXVI), dans lequel l'un au moins des $R^4$, $R^6$, $R^{10}$, $R^{11}$, $m_1$ et $m_2$ est différent dans l'un au moins des motifs.

On peut encore utiliser un copolymère comportant au moins un motif de formule (XXII) et au moins un motif de formule (XXVI), les motifs de formule (XXII) et les motifs de formule (XXVI) pouvant être identiques ou différents les uns des autres.

Selon une variante, on peut encore utiliser un copolymère comprenant de plus au moins un motif hydrocarboné comportant deux groupes capables d'établir des interactions hydrogènes choisis parmi les groupes ester, amide, sulfonamide, carbamate, thiocarbamate, urée, uréthane, thiourée, oxamido, guanidino, biguanidino et leurs combinaisons.

Ces copolymères peuvent être des copolymères blocs, des copolymères séquencés ou des copolymères greffés.

## II. Filmogène insoluble dans la phase grasse

Selon l'invention le polymère filmogène peut être un solide insoluble dans la phase grasse de la composition

à une température ambiante, par exemple, d'environ 25 °C. Le polymère est également insoluble dans la phase grasse à sa température de ramollissement, à l'inverse d'une cire même d'origine polymérique qui est elle soluble dans la phase organique liquide (ou phase grasse) à sa température de fusion. En ce sens, le polymère n'est pas une cire.

*1) Polymères*

La composition selon l'invention comprend avantageusement au moins une dispersion stable de particules de polymère essentiellement sphériques d'un ou plusieurs polymères, dans une phase grasse physiologiquement acceptable.

Ces dispersions peuvent notamment se présenter sous forme de nanoparticules de polymères en dispersion stable dans ladite phase organique liquide. Les nanoparticules sont de préférence d'une taille moyenne comprise entre 5 et 800 nm, et mieux entre 50 et 500 nm. Il est toutefois possible d'obtenir des tailles de particules de polymère allant jusqu'à 1μm.

En particulier, les particules de polymères en dispersion sont insolubles dans les alcools hydrosolubles tels que, par exemple, l'éthanol.

Les polymères en dispersion utilisables dans la composition de l'invention ont de préférence un poids moléculaire de l'ordre de 2000 à 10 000 000 g/mol, et une Tg de - 100°C à 300°C et mieux de -50° à 100°C, de préférence de -10°C à 50°C.

Il est possible d'utiliser des polymères filmifiables, de préférence ayant une Tg basse, inférieure ou égale à la température de la peau et notamment inférieure ou égale à 40°C.

Parmi les polymères filmogènes, on peut citer des homopolymères ou des copolymères radicalaires, acryliques ou vinyliques, de préférence ayant une Tg inférieure ou égale à 40°C et notamment allant de -10° à 30°C, utilisés seul ou en mélange.

Par polymère radicalaire, on entend un polymère obtenu par polymérisation de monomères à insaturation notamment éthylénique, chaque monomère étant susceptible de s'homopolymériser (à l'inverse des polycondensats). Les polymères radicalaires peuvent être notamment des polymères, ou des copolymères, vinyliques, notamment des polymères acryliques.

Les polymères acryliques peuvent résulter de la polymérisation de monomères à insaturation éthylénique ayant au moins un groupement acide et/ou des esters de ces monomères acides et/ou des amides de ces acides.

Comme monomère porteur de groupement acide, on peut utiliser des acides carboxyliques insaturés α,β-éthyléniques tels que l'acide acrylique, l'acide méthacrylique, l'acide crotonique, l'acide maléique, l'acide itaconique. On utilise en particulier l'acide (méth)acrylique et l'acide crotonique, et plus préférentiellement l'acide (méth)acrylique.

Les esters de monomères acides sont avantageusement choisis parmi les esters de l'acide (méth)acrylique (encore appelé les (méth)acrylates), comme les (méth)acrylates d'alkyle, en particulier d'alkyle en $C_1$-$C_{20}$, de préférence en $C_1$-$C_8$, les (méth)acrylates d'aryle, en particulier d'aryle en $C_6$-$C_{10}$, les (méth)acrylates d'hydroxyalkyle, en particulier d'hydroxyalkyle en $C_2$-$C_6$. Comme (méth)acrylates d'alkyle, on peut citer le (méth)acrylate de méthyle, d'éthyle, de butyle, d'isobutyle, d'éthyl-2 hexyle et de lauryle. Comme (méth)acrylates d'hydroxyalkyle, on peut citer le (méth)acrylate d'hydroxyéthyle, le (méth)acrylate de 2-hydroxypropyle. Comme (méth)acrylates d'aryle, on peut citer l'acrylate de benzyle ou de phényle.

Les esters de l'acide (méth)acrylique qui conviennent particulièrement pour les compositions cosmétiques selon l'invention sont les (méth)acrylates d'alkyle.

Comme polymère radicalaire, on utilise en particulier les copolymères d'acide (méth)acrylique et de (méth)acrylate d'alkyle, notamment d'alkyle en $C_1$-$C_4$. Plus particulièrement, on peut utiliser les acrylates de méthyle éventuellement copolymérisés avec l'acide acrylique.

Comme amides des monomères acides, on peut citer les (méth)acrylamides, et notamment les N-alkyl (méth)acrylamides, en particulier d'alkyle en $C_2$-$C_{12}$ tels que le N-éthyl acrylamide, le N-t-butyl acrylamide, le N-octyl acrylamide ; les N- dialkyl ($C_1$-$C_4$) (méth)acrylamides.

Les polymères acryliques peuvent également résulter de la polymérisation de monomères à insaturation éthylénique ayant au moins un groupe amine, sous forme libre ou bien partiellement ou totalement neutralisée, ou bien encore partiellement ou totalement quaternisée. De tels monomères peuvent être par exemple le (méth)acrylate de diméthylaminoéthyle, le méthacrylamide de diméthylaminoéthyle, la vinylamine, la vinylpyridine, le chlorure de diallyldiméthylammonium.

Les polymères vinyliques peuvent également résulter de l'homopolymérisation ou de la copolymérisation d'au moins un monomère choisi parmi les esters vinyliques et les monomères styrèniques. En particulier, ces monomères peuvent être polymérisés avec des monomères acides et/ou leurs esters et/ou leurs amides, tels que ceux mentionnés précédemment. Comme exemple d'esters vinyliques, on peut citer l'acétate de vinyle, le propionate de vinyle, le néodécanoate de vinyle, le pivalate de vinyle, le benzoate de vinyle et le t-butyl benzoate de vinyle. Comme monomères styréniques, on peut citer le styrène et l'alpha-méthyl styrène.

La liste des monomères donnée n'est pas limitative et il est possible d'utiliser tout monomère connu de l'homme du métier entrant dans les catégories de monomères acryliques et vinyliques (y compris les monomères modifiés par une chaîne siliconée).

Comme autres monomères vinyliques utilisables, on peut encore citer :

- la N-vinylpyrrolidone, la vinylcaprolactame, les vinyl N-alkyl($C_1$-$C_6$) pyrroles, les vinyl-oxazoles, les vinyl-thiazoles, les vinylpyrimidines, les vinylimidazoles,
- les oléfines tels que l'éthylène, le propylène, le butylène, l'isoprène, le butadiène.

Le polymère vinylique peut être réticulé à l'aide d'un ou plusieurs monomères difonctionnels, notamment comprenant au moins deux insaturations éthyléniques, tel que le diméthacrylate d'éthylène glycol ou le phtalate de diallyle.

De façon non limitative, les polymères en dispersion de l'invention peuvent être choisis parmi, les polymères ou copolymères suivants : polyuréthanes, polyuréthanes-acryliques, polyurées, polyurée-polyuréthanes, polyester-polyuréthanes, polyéther-polyuréthanes, polyesters, polyesters amides, alkydes ; polymères ou copolymères acryliques et/ou vinyliques ; copolymères acryliques-silicone ; polyacrylamides ; polymères siliconés comme les polyuréthanes ou acryliques siliconés, polymères fluorés et leurs mélanges.

Le ou les polymères en dispersion dans la phase grasse peuvent représenter en matière sèche de 5 à 40% du poids de la composition, de préférence de 5 à 35 % et mieux de 8 à 30%.

*2) <u>Stabilisant</u>*

Selon un mode de mise en oeuvre, les particules de polymère en dispersion sont stabilisées en surface par un stabilisant solide à température ambiante. Dans ce cas, la quantité en matière sèche de la dispersion représente la quantité totale de polymère + stabilisant, sachant que la quantité de polymère ne peut être inférieure à 5%.

Les particules de polymère sont en particulier stabilisées en surface grâce à un stabilisant, qui peut être un polymère séquencé, un polymère greffé, et/ou un polymère statistique, seul ou en mélange. La stabilisation peut être effectuée par tout moyen connu, et en particulier par ajout direct du polymère stabilisant lors de la polymérisation.

Le stabilisant peut être également présent dans le mélange avant polymérisation du polymère. Toutefois, il est également possible de l'ajouter en continu, notamment lorsqu'on ajoute également les monomères en continu.

On peut utiliser 2-30% en poids de stabilisant par rapport au mélange initial de monomères, et de préférence 5-20% en poids.

Lorsqu'on utilise un polymère greffé et/ou séquencé en tant que stabilisant, on choisit le solvant de synthèse de telle manière qu'au moins une partie des greffons ou séquences dudit polymère-stabilisant soit soluble dans ledit solvant, l'autre partie des greffons ou séquences n'y étant pas soluble. Le polymère-stabilisant utilisé lors de la polymérisation doit être soluble, ou dispersible, dans le solvant de synthèse. De plus, on choisit de préférence un stabilisant dont les séquences ou greffons insolubles présentent une certaine affinité pour le polymère formé lors de la polymérisation.

Parmi les polymères greffés, on peut citer les polymères siliconés greffés avec une chaîne hydrocarbonée ; les polymères hydrocarbonés greffés avec une chaîne siliconée.

Ainsi on peut utiliser des copolymères blocs greffés ou séquencés comprenant au moins un bloc de type polyorganosiloxane et au moins un bloc d'un polymère radicalaire, comme les copolymères greffés de type acrylique/silicone qui peuvent être employés notamment lorsque le milieu non aqueux est siliconé.

On peut aussi utiliser des copolymères blocs greffés ou séquencés comprenant au moins un bloc de type polyorganosiloxane et au moins d'un polyéther. Le bloc polyorganopolysiloxane peut être notamment un polydiméthylsiloxane ou bien encore un poly alkyl($C_2$-$C_{18}$) méthyl siloxane ; le bloc polyéther peut être un poly alkylène en $C_2$-$C_{18}$, en particulier polyoxyéthylène et/ou polyoxypropylène. En particulier, on peut utiliser les diméthicones copolyol ou des alkyl ($C_2$-$C_{18}$) diméthicones copolyol tels que ceux vendu sous la dénomination "DOW CORNING 3225C" par la société DOW CORNING, les lauryl méthicones tels que ceux vendu sous la dénomination "DOW CORNING Q2-5200 par la société "DOW CORNING".

Comme copolymères blocs greffés ou séquencés, on peut citer aussi ceux comprenant au moins un bloc résultant de la polymérisation d'au moins un monomère éthylénique, à une ou plusieurs liaisons éthyléniques éventuellement conjuguées, comme l'éthylène ou les diènes tels que le butadiène et l'isoprène, et d'au moins un bloc d'un polymère vinylique et mieux styrénique. Lorsque le monomère éthylénique comporte plusieurs liaisons éthyléniques éventuellement conjuguées, les insaturations éthyléniques résiduelles après la polymérisation sont généralement hydrogénées. Ainsi, de façon connue, la polymérisation de l'isoprène conduit, après hydrogénation, à la formation de bloc éthylène-propylène, et la polymérisation de butadiène conduit, après hydrogénation, à la formation de bloc éthylène-butylène. Parmi ces polymères, on peut citer les copolymères séquencés, notamment de type "dibloc" ou "tribloc" du type

polystyrène/polyisoprène (SI), polystyrène/polybutadiène (SB) tels que ceux vendus sous le nom de 'LUVITOL HSB' par BASF, du type polystyrène/copoly(éthylène-propylène) (SEP) tels que ceux vendus sous le nom de "KRATON" par SHELL CHEMICAL Co ou encore du type polystyrène/copoly(éthylène-butylène) (SEB). En particulier, on peut utiliser le KRATON G1650 (SEBS), le KRATON G1651 (SEBS), le KRATON G1652 (SEBS), le KRATON G1657X (SEBS), le KRATON G1701X (SEP), le KRATON G1702X (SEP), le KRATON G1726X (SEB), le KRATON D-1101 (SBS), le KRATON D-1102 (SBS), le KRATON D-1107 (SIS). Les polymères sont généralement appelés des copolymères de diènes hydrogénés ou non.

On peut aussi utiliser les GELLED PERMETHYL 99A-750, 99A-753-59 et 99A-753-58 (mélange de tribloc et de polymère en étoile), VERSAGEL 5960 de chez PENRECO (tribloc + polymère en étoile) ; OS129880, OS129881 et OS84383 de chez LUBRIZOL (copolymère styrène/méthacrylate).

Comme copolymères blocs greffés ou séquencés comprenant au moins un bloc résultant de la polymérisation d'au moins un monomère éthylénique à une ou plusieurs liaisons éthyléniques et d'au moins un bloc d'un polymère acrylique, on peut citer les copolymères bi- ou triséquencés poly(méthylacrylate de méthyle)/polyisobutylène ou les copolymères greffés à squelette poly(méthylacrylate de méthyle) et à greffons polyisobutylène.

Comme copolymères blocs greffés ou séquencés comprenant au moins un bloc résultant de la polymérisation d'au moins un monomère éthylénique à une ou plusieurs liaisons éthyléniques et d'au moins un bloc d'un polyéther tel qu'un polylkylène en $C_2$-$C_{18}$ (polyéthyléné et/ou polyoxypropyléné notamment), on peut citer les copolymères bi- ou triséquencés polyoxyéthylène/polybutadiène ou polyoxyéthylène/polyisobutylène.

Lorsqu'on utilise un polymère statistique en tant que stabilisant, on le choisit de manière à ce qu'il possède une quantité suffisante de groupements le rendant soluble dans le solvant de synthèse envisagé.

On peut ainsi employer des copolymères à base d'acrylates ou de méthacrylates d'alkyle issus d'alcools en $C_1$-$C_4$, et d'acrylates ou de méthacrylates d'alkyle issus d'alcools en $C_8$-$C_{30}$. On peut en particulier citer le copolymère méthacrylate de stéaryle/méthacrylate de méthyle.

Lorsque le solvant de synthèse du polymère est apolaire, il est avantageux de choisir en tant que stabilisant, un polymère apportant une couverture des particules la plus complète possible, plusieurs chaînes de polymères-stabilisants venant alors s'adsorber sur une particule de polymère obtenu par polymérisation.

Dans ce cas, on préfère alors utiliser comme stabilisant, soit un polymère greffé, soit un polymère séquencé, de manière à avoir une meilleure activité interfaciale. En effet, les séquences ou greffons insolubles dans le solvant de synthèse apportent une couverture plus volumineuse à la surface des particules.

Lorsque le solvant de synthèse comprend au moins une huile de silicone, l'agent stabilisant est de préférence choisi dans le groupe constitué par les copolymères blocs greffés ou séquencés comprenant au moins un bloc de type polyorganosiloxane et au moins un bloc d'un polymère radicalaire ou d'un polyéther ou d'un polyester comme les blocs polyoxypropyléné et/ou oxyéthyléné.

Lorsque le solvant de synthèse ne comprend pas d'huile de silicone, l'agent stabilisant est de préférence choisi dans le groupe constitué par :

a) les copolymères blocs greffés ou séquencés comprenant au moins un bloc de type polyorganosiloxane et au moins un bloc d'un polymère radicalaire ou d'un polyéther ou d'un polyester,

b) les copolymères d'acrylates ou de méthacrylates d'alkyle issus d'alcools en $C_1$-$C_4$, et d'acrylates ou de méthacrylates d'alkyle issus d'alcools en $C_8$-$C_{30}$,

c) les copolymères blocs greffés ou séquencés comprenant au moins un bloc résultant de la polymérisation d'au moins un monomère éthylénique, à liaisons éthyléniques conjuguées,

et au moins un bloc d'un polymère vinylique ou acrylique ou d'un polyéther ou d'un polyester, ou leurs mélanges.

De préférence, on utilise des polymères dibloc comme agent stabilisant.

Le polymère filmogène liposoluble ou en dispersion dans une phase grasse peut également être utilisé en une quantité allant de 0,01% à 20% (en matière active) par rapport au poids total de la composition, tel que par exemple de 1 % à 10%, le cas échéant.

## IV. Filmogène dispersible dans la phase aqueuse

Selon un autre mode de réalisation, le polymère filmogène peut être choisi parmi les dispersions aqueuses de particules polymères, dans le cas où la composition selon l'invention comprend une phase aqueuse.

La dispersion aqueuse comprenant un ou plusieurs polymères filmogènes peut être préparée par l'homme de l'art sur la base de ses connaissances générales, en particulier par une polymérisation en émulsion ou par une mise en dispersion du polymère précédemment formé.

Parmi les polymères filmogènes pouvant être utilisés dans la composition selon la présente invention, on peut citer les polymères synthétiques du type polycondensat ou du type radical, les polymères d'origine naturelle, et des

mélanges de ceux-ci.

*1) Polycondensats*

Parmi les polycondensats, on peut également citer les polyuréthanes anioniques, cationiques, non ioniques ou amphotères, les polyuréthane-acryliques, les polyuréthanes-polyvinylpyrrolidones, les polyester-polyuréthanes, les polyéther-polyuréthanes, les polyurées, les polyurée/polyuréthanes, et des mélanges de ceux-ci.

Les polyuréthanes peuvent être par exemple un copolymère de polyuréthane aliphatique, cycloaliphatique ou aromatique, de polyurée/polyuréthane ou de polyurée comprenant, seul ou en tant que mélange :

- au moins une séquence d'origine polyester linéaire ou ramifiée, aliphatique et/ou cycloaliphatique et/ou aromatique, et/ou
- au moins une séquence d'origine polyéther aliphatique et/ou cycloaliphatique et/ou aromatique, et/ou
- au moins une séquence siliconée, substituée ou non substituée, ramifiée ou non ramifiée, par exemple de polydiméthylsiloxane ou de polyméthylphénylsiloxane, et/ou
- au moins une séquence comprenant des groupements fluorés.

Les polyuréthanes tels que définis dans l'invention peuvent également être obtenus à partir de polyesters ramifiés ou non ramifiés ou à partir d'alkydes comprenant des hydrogènes mobiles qui sont modifiés au moyen d'une polyaddition avec un diisocyanate et un composé co-réactif bifonctionnel organique (par exemple dihydro, diamino ou hydroxy-amino), comprenant en outre soit un groupement carboxylate ou acide carboxylique, soit un groupement sulfonate ou acide sulfonique, voire un groupement amine tertiaire neutralisable ou un groupement ammonium quaternaire.

On peut également citer les polyesters, les polyesteramides, les polyesters à chaîne grasse, les polyamides et les résines d'époxyester.

Les polyesters peuvent être obtenus de manière connue au moyen de la polycondensation de diacides aliphatiques ou aromatiques avec des diols aliphatiques ou aromatiques ou avec des polyols. L'acide succinique, l'acide glutarique, l'acide adipique, l'acide pimélique, l'acide subérique ou l'acide sébacique peuvent être utilisés comme diacides aliphatiques. L'acide téréphtalique ou l'acide isophtalique, voire un dérivé tel que l'anhydride phtalique, peuvent être utilisés comme diacides aromatiques. L'éthylène glycol, le propylène glycol, le diéthylène glycol, le néopentyl glycol, le cyclohexanediméthanol et le 4,4-N-(1-méthylpropylidène)bisphénol, peuvent être utilisés comme diols aliphatiques. Le glycérol, le pentaérythritol, le sorbitol et le triméthylolpropane peuvent être utilisés comme polyols.

Les polyesteramides peuvent être obtenus de manière analogue aux polyesters, au moyen de la polycondensation de diacides avec des diamines ou des aminoalcools. L'éthylènediamine, l'hexaméthylènediamine, et la méta- ou para-phénylènediamine peuvent être utilisées comme diamine. La monoéthanolamine peut être utilisée comme aminoalcool.

Comme monomère portant un groupement anionique pouvant être utilisé pendant la polycondensation, on peut citer par exemple, l'acide diméthylolpropionique, l'acide trimellitique ou un dérivé tel que l'anhydride trimellitique, le sel de sodium de l'acide 3-sulfopentanediol et le sel de sodium de l'acide 5-sulfo-1,3-benzènedicarboxylique. Les polyesters ayant une chaîne grasse peuvent être obtenus par l'intermédiaire de l'utilisation de diols ayant une chaîne grasse lors de la polycondensation. Les résines d'époxyester peuvent être obtenues par la polycondensation d'acides gras avec un condensat au niveau des extrémités $\alpha,\omega$-diépoxy.

Les polymères radicalaires peuvent être en particulier les polymères ou les copolymères acryliques et/ou vinyliques. Les polymères à radical anionique sont préférés. Comme monomère portant un groupement anionique pouvant être utilisé lors de la polymérisation radicalaire, on peut citer l'acide acrylique, l'acide méthacrylique, l'acide crotonique, l'anhydride maléique et l'acide 2-acrylamido-2-méthylpropanesulfonique.

Les polymères acryliques peuvent résulter de la copolymérisation de monomères choisis parmi les esters et/ou les amides de l'acide acrylique ou de l'acide méthacrylique. Comme exemples de monomères du type ester, on peut citer le méthacrylate de méthyle, le méthacrylate d'éthyle, le méthacrylate de butyle, le méthacrylate d'isobutyle, le méthacrylate de 2-éthylhexyle et le méthacrylate de lauryle. Comme exemples de monomères du type amide, on peut citer le N-t-butylacrylamide et le N-t-octylacrylamide.

On utilise en particulier les polymères acryliques obtenus par la copolymérisation de monomères à insaturation éthylénique contenant des groupements hydrophiles, préférablement de nature non ionique, tels que l'acrylate d'hydroxyéthyle, l'acrylate de 2-hydroxypropyle, le méthacrylate d'hydroxyéthyle et le méthacrylate de 2-hydroxypropyle.

Les polymères vinyliques peuvent résulter de l'homopolymérisation ou de la copolymérisation de monomères choisis parmi les esters vinyliques, le styrène ou le butadiène. Comme exemples d'esters vinyliques, on peut citer l'acétate de vinyle, le néodécanoate de vinyle, le pivalate de vinyle, le benzoate de vinyle et le t-butylbenzoate de vinyle.

On peut également utiliser des copolymères d'acrylique/silicone, voire des copolymères de nitrocellulose/acrylique.

2) *Polymère type radical*

On peut également citer les polymères résultant de la polymérisation radicalaire d'un ou plusieurs monomères radicalaires, à l'intérieur et/ou partiellement à la surface de particules préexistantes d'au moins un polymère choisi parmi le groupe constitué par les polyuréthanes, les polyurées, les polyesters, les polyesteramides et/ou les alkydes. Ces polymères sont généralement appelés « polymères hybrides ».
Lorsqu'une dispersion aqueuse de particules polymères est utilisée, la teneur en matière sèche de ladite dispersion aqueuse peut être de l'ordre de 3 à 60% en poids, et préférablement de 10 à 50%.
La taille des particules polymères en dispersion aqueuse peut être comprise entre 10 et 500 nm, et elle est préférablement comprise entre 20 et 150 nm, permettant l'obtention d'un film ayant un brillant notable. Cependant, on peut utiliser des tailles de particules allant jusqu'à un micron.
Comme dispersion aqueuse de polymère filmogène, on peut utiliser les dispersions acryliques vendues sous les dénominations «NEOCRYL XK-90®», «NEOCRYL A-1070®», «NEOCRYL A-1090® », «NEOCRYL BT-62®», «NEOCRYL A-1079®» et «NEOCRYL A-523®» par la société AVECIA-NEORESINS, «DOW LATEX 432®» par la société DOW CHEMICAL, «DAITOSOL 5000 AD®» ou «DAITOSOL 5000 SJ» par la société DAITO KASEY KOGYO; «SYNTRAN 5760» par la société INTERPOLYMER ou encore les dispersions aqueuses de polyuréthane vendues sous les dénominations «NEOREZ R-981®» et «NEOREZ R-974®» par la société AVECIA-NEORESINS, les «AVALURE UR-405® », «AVALURE UR-410® », «AVALURE UR-425®», «AVALURE UR-450® », «SANCURE 875®», «SANCURE 861®», «SANCURE 878®» et «SANCURE 2060®» par la société GOODRICH, «IMPRANIL 85®» par la société BAYER, «AQUAMERE H-1511®» par la société HYDROMER ; les sulfopolyesters vendus sous le nom de marque «EASTMAN AQ® » par la société EASTMAN CHEMICAL PRODUCTS, les dispersions vinyliques comme le «MEXOMERE PAM», les dispersions aqueuses de polyvinyl acétate comme le «VINYBRAN®» de la société NISSHIN CHEMICAL ou celles commercialisées par la société UNION CARBIDE, les dispersions aqueuses de terpolymère vinyl pyrrolidone, diméthylaminopropyl méthacrylamide et chlorure de lauryldiméthylpropylmethacrylamidoammonium telles que le STYLEZE W-d'ISP, les dispersion aqueuse de polymère hybrides polyuréthane/polyacryliques telles que celles commercialisées sous les références «HYBRIDUR ®» par la société AIR PRODUCTS ou « DUROMER® » de NATIONAL STARCH, les dispersions type core/shell : par exemple celles commercialisées par la société ATOFINA sous la référence KYNAR (core : fluoré - shell : acrylique) ou encore ceux décrits dans le document US 5 188 899 (core : silice - shell : silicone) et leurs mélanges.
Dans le cas où la composition comprend une phase aqueuse, le polymère filmogène peut être un polymère hydrosoluble. Le polymère hydrosoluble est donc solubilisé dans la phase aqueuse de la composition.
Parmi les polymères filmogènes hydrosolubles, on peut citer les polymères cationiques suivants :

1) les polymères ou copolymères acryliques, tels que les polyacrylates ou les polyméthacrylates ; les copolymères de la famille (1) peuvent contenir en outre un ou plusieurs motifs dérivant de comonomères pouvant être choisis parmi la famille des acrylamides, des méthacrylamides, des diacétoneacrylamides, des acrylamides et méthacrylamides substitués sur l'azote par des alkyles inférieurs, des acides acrylique ou méthacrylique ou des esters de ceux-ci, des vinyllactames tels que la vinylpyrrolidone ou le vinylcaprolactame, des esters vinyliques.
Ainsi, parmi ces copolymères de la famille (1), on peut citer :

- les copolymères d'acrylamide et de méthacrylate de diméthylaminoéthyle quaternisés par le sulfate de diméthyle, ou par un halogénure de diméthyle tel que celui commercialisé sous la dénomination HERCOFLOC par la société HERCULES,
- le copolymère d'acrylamide et de chlorure de méthacryloyloxyéthyltriméthylammonium décrit, par exemple, dans la demande de brevet EP-A-0 809 76 et commercialisé sous la dénomination BINA QUAT P 100 par la société CIBA GEIGY,
- le copolymère d'acrylamide et de méthosulfate de méthacryloyloxyéthyltriméthylammonium commercialisé sous la dénomination RETEN par la société HERCULES,
- les copolymères de vinylpyrrolidone/acrylate ou méthacrylate de dialkylaminoalkyle, quaternisés ou non quaternisés, tels que les produits commercialisés sous la dénomination « GAFQUAT » par la société ISP, tels que par exemple « GAFQUAT 734 » ou « GAFQUAT 755 », ou bien les produits désignés par « COPOLYMER 845, 958 et 937 ». Ces polymères sont décrits en détail dans les brevets français 2 077 143 et 2 393 573,
- les terpolymères de méthacrylate de diméthyl-aminoéthyle/vinylcaprolactame/vinylpyrrolidone tels que le produit commercialisé sous la dénomination GAFFIX VC 713 par la société ISP, et
- le copolymère de vinylpyrrolidone/diméthylaminopropyl-méthacrylamide quaternisé tel que le produit commercialisé sous la dénomination « GAFQUAT HS 100 » par la société ISP.

2) les polysaccharides quaternisés décrits plus particulièrement dans les brevets US 3 589 578 et US 4 031 307,

tels que les gommes de guar contenant des groupements trialkylammonium cationiques. De tels produits sont commercialisés en particulier sous les dénominations commerciales JAGUAR C13 S, JAGUAR C 15 et JAGUAR C 17 par la société MEYHALL.

3) les copolymères de vinylpyrrolidone et de vinylimidazole quaternaires ;

4) les chitosanes ou les sels de ceux-ci ;

5) les dérivés de cellulose cationiques, tels que les copolymères de cellulose ou de dérivés de cellulose greffés par un monomère hydrosoluble comprenant un ammonium quaternaire et décrits en particulier dans le brevet US 4 131 576, tels que les hydroalkyl celluloses, comme les hydroxyméthyl, hydroxyéthyl ou hydroxypropyl celluloses greffées en particulier par un sel de méthacryloyloxyéthyltriméthylammonium, de méthacrylamidopropyltriméthylammonium ou de diméthyldiallylammonium. Les produits commercialisés correspondant à cette définition sont plus particulièrement les produits commercialisés sous la dénomination « CELQUAT L 200 » et « CELQUAT H 100 » par la NATIONAL STARCH COMPANY.

Parmi les polymères hydrosolubles filmogènes, on peut citer les polymères amphotères suivants :

1) les polymères résultant de la copolymérisation d'un monomère dérivé d'un composé vinylique portant un groupement carboxylique tel que plus particulièrement l'acide acrylique, l'acide méthacrylique, l'acide maléique, l'acide alpha-chloroacrylique, et d'un monomère basique dérivé d'un composé vinylique substitué contenant au moins un atome de base tel que plus particulièrement un méthacrylate et acrylate de dialkylaminoalkyle, et un dialkylaminoalkylméthacrylamide et acrylamide. De tels composés sont décrits dans le brevet américain n° 3 836 537,

2) les polymères comprenant des motifs dérivant :

a) d'au moins un monomère choisi parmi les acrylamides ou les méthacrylamides substitués sur l'azote par un radical alkyle,

b) d'au moins un comonomère acide contenant un ou plusieurs groupements carboxyliques réactifs, et

c) d'au moins un comonomère basique tel que les esters, ayant des substituants amine primaire, secondaire, tertiaire et quaternaire, d'acides acrylique et méthacrylique, et le produit de la quaternisation du méthacrylate de diméthylaminoéthyle par du sulfate de diméthyle ou de diéthyle.

d) les alkoylpolyaminoamides réticulés dérivés totalement ou en partie de polyaminoamides,

3) les polymères comprenant des motifs zwitterioniques,

4) le polymère dérivé du chitosane,

5) les polymères dérivés de la N-carboxyalkylation du chitosane, tels que le N-carboxyméthyl chitosane ou le N-carboxybutyl chitosane commercialisé sous la dénomination « EVALSAN » par la société JAN DEKKER,

6) les copolymères du ($C_1$-$C_5$)alkylvinyléther/ anhydride maléique partiellement modifié par une semi-amidification par une N,N-dialkylaminoalkylamine, telle que la N,N-diméthylaminopropylamine ou par une semi-estérification par une N,N-dialcanolamine. Ces copolymères peuvent également comporter d'autres comonomères vinyliques tels que le vinylcaprolactame.

Les polymères filmogènes hydrosolubles sont préférablement choisis parmi le groupe constitué par :

- les protéines telles que les protéines d'origine végétale, telles que les protéines de blé ou de soja ; les protéines d'origine animale, telles que la kératine, par exemple les hydrolysats de kératine et les kératines sulfoniques ;
- les polymères anioniques, cationiques, amphotères ou non ioniques de la chitine ou du chitosane ;
- les polymères cellulosiques, tels que l'hydroxyéthyl cellulose, l'hydroxypropyl cellulose, la méthylcellulose, l'éthylhydroxyéthyl cellulose, la carboxyméthyl cellulose, et les dérivés quaternisés de la cellulose ;
- les polymères ou copolymères acryliques tels que les polyacrylates ou les polyméthacrylates ;
- les polymères vinyliques, tels que les polyvinylpyrrolidones, les copolymères du méthylvinyléther et de l'anhydride maléique, le copolymère de l'acétate de vinyle et de l'acide crotonique, les copolymères de la vinylpyrrolidone et de l'acétate de vinyle ;
- les copolymères de la vinylpyrrolidone et du caprolactame ; les alcools polyvinyliques ;
- les polymères éventuellement modifiés d'origine naturelle, tels que :
  - la gomme arabique, la gomme de guar, les dérivés du xanthane, la gomme de karaya ;
  - les alginates et les carraghénanes ;
  - les glycoaminoglycanes, l'acide hyaluronique et ses dérivés ;
  - la gomme laque, la gomme sandaraque, les dammars, l'élémis, les copals ;
  - l'acide désoxyribonucléique ;

- les mucopolysaccharides, tels que l'acide hyaluronique, le sulfate de chondroïtine, et des mélanges de ceux-ci.

Ces polymères seront utilisés en particulier si l'on désire une élimination plus ou moins appréciable du film par de l'eau.

Afm d'améliorer la nature filmogène d'un polymère huileux ou aqueux, il est possible d'ajouter au système polymère un agent de coalescence qui sera choisi parmi les agents de coalescence connus.

**V. Filmogène siliconé**

*1) Polymère à squelette*

Selon un mode de réalisation de l'invention, le polymère filmogène peu être choisi parmi les polymères ayant un squelette organique non siliconé greffé par des monomères contenant un polysiloxane. Ces polymères peuvent être liposolubles, lipodispersibles, hydrosolubles ou dispersibles en milieu aqueux, le cas échéant.

Les polymères ayant un squelette organique non siliconé greffé par des monomères contenant un polysiloxane sont constitués d'une chaîne organique principale formée de monomères organiques ne comprenant pas la silicone, sur laquelle on greffe, au sein de ladite chaîne ainsi qu'éventuellement sur au moins l'une des extrémités de celle-ci, au moins un macromère de polysiloxane.

Dans ce qui suit, on doit comprendre que l'expression « macromère de polysiloxane » désigne, ainsi qu'il est généralement accepté, tout monomère contenant une chaîne polymère du type polysiloxane dans sa structure.

Les monomères organiques non siliconés constituant la chaîne principale du polymère siliconé greffé peuvent être choisis parmi les monomères à insaturation éthylénique pouvant être polymérisés par la méthode radicalaire, les monomères polymérisables par polycondensation tels que ceux formant les polyamides, les polyesters, les polyuréthanes, les monomères à cycle ouvrant tels que ceux du type oxazoline ou caprolactone.

Les polymères ayant un squelette organique non siliconé greffé par des monomères contenant un polysiloxane selon la présente invention peuvent être obtenus conformément à toute méthode connue de l'homme de l'art, en particulier par la réaction entre (i) un macromère de polysiloxane de départ correctement fonctionnalisé sur la chaîne de polysiloxane et (ii) un ou plusieurs composés organiques non siliconés, eux même correctement fonctionnalisés par une fonction qui est capable de réagir avec le groupement ou les groupements fonctionnel(s) porté(s) par ladite silicone en formant une liaison covalente ; un exemple classique d'une telle réaction est la réaction radicalaire entre un groupement vinyle porté à l'une des extrémités de la silicone avec une double liaison d'un monomère à insaturation éthylénique de la chaîne principale.

Les polymères ayant un squelette organique non siliconé greffé par des monomères contenant un polysiloxane selon l'invention sont choisis de préférence parmi ceux décrits dans les brevets US 4 693 935, US 4 728 571 et US 4 972 037 et les demandes de brevet EP-A-0 412 704, EP-A-0 412 707, EP-A-0 640 105 et WO 95/00578. Il concerne des copolymères obtenus par la polymérisation radicalaire à partir de monomères à insaturation éthylénique et de monomères ayant un groupement terminal vinyle, ou bien des copolymères obtenus par la réaction d'une polyoléfine contenant des groupements fonctionnalisés et un macromère de polysiloxane ayant une fonction terminale réactive avec lesdits groupements fonctionnalisés.

Une famille particulière de polymères siliconés greffés convenables pour la mise en oeuvre de la présente invention est constituée par les polymères siliconés greffés contenant :

a) de 0 à 98% en poids d'au moins un monomère lipophile (A) de faible polarité lipophile à insaturation éthylénique, polymérisable par la méthode radicalaire ;
b) de 0 à 98% en poids d'au moins un monomère polaire hydrophile (B) à insaturation éthylénique, copolymérisable avec le monomère ou les monomères du type (A) ;
c) de 0,01 à 50% en poids d'au moins un macromère de polysiloxane (C) de formule générale (XXVII) :

$$X(Y)_nSi(R)_{3-m}Z_m \qquad \text{(XXVII)}$$

dans laquelle :

- X désigne un groupement vinyle copolymérisable avec les monomères (A) et (B) ;
- Y désigne un groupement ayant une liaison divalente ;
- R désigne hydrogène, alkyle ou alkoxy en $C_1$-$C_6$, aryle en $C_6$-$C_{12}$ ;
- Z désigne un motif polysiloxane monovalent ayant un poids moléculaire moyen en nombre d'au moins 500 ;
- n vaut 0 ou 1 et m est un nombre entier allant de 1 à 3 ; les pourcentages étant calculés par rapport au poids total des monomères (A), (B) et (C).

Ces polymères ont un poids moléculaire moyen en nombre allant de 10 000 à 2 000 000, et préférablement une température de transition vitreuse Tg ou une température de fusion cristalline Tm d'au moins -20°C.

Comme exemples de monomères lipophiles (A), on peut citer les esters d'alcool en $C_1$-$C_{18}$ et de l'acide acrylique ou méthacrylique ; les esters d'alcool en $C_{12}$-$C_{30}$ et de l'acide méthacrylique, le styrène ; les macromères de polystyrène ; l'acétate de vinyle ; le propionate de vinyle ; l'alpha-méthylstyrène ; le tertio-butylstyrène ; le butadiène ; le cyclohexadiène; l'éthylène ; le propylène ; le vinyltoluène, les esters de l'acide acrylique ou méthacrylique et de 1,1-dihydroperfluoroalcanols ou d'homologues de ceux-ci ; les esters de l'acide acrylique ou méthacrylique et d'omega-hydrofluoroalcanols ; les esters de l'acide acrylique ou méthacrylique et de fluoroalkylsulfonamidoalcools ; les esters de l'acide acrylique ou méthacrylique et de fluoroalkylalcools ; les esters de l'acide acrylique ou méthacrylique et d'alcoolfluoroéthers ; ou des mélanges de ceux-ci. Les monomères (A) préférés sont choisis au sein du groupe constitué par le méthacrylate de n-butyle, le méthacrylate d'isobutyle, l'acrylate de tertio-butyle, le méthacrylate de tertio-butyle, le méthacrylate de 2-éthylhexyle, le méthacrylate de méthyle, l'acrylate de 2-(N-méthylperfluorooctanesulfonamido)éthyle, l'acrylate de 2-(N-butylperfluorooctanesulfonamido)éthyle, ou des mélanges de ceux-ci.

Comme exemples de monomères (B) polaires, on peut citer l'acide acrylique, l'acide méthacrylique, le N,N-diméthylacrylamide, le méthacrylate de diméthylaminoéthyle, le méthacrylate de diméthylaminoéthyle quaternisé, le (méth)acrylamide, le N-t-butylacrylamide, l'acide maléique, l'anhydride maléique et les hemi-esters de ceux-ci, les (méth)acrylates d'hydroxyalkyle, le chlorure de diallyldiméthylammonium, la vinylpyrrolidone, les éthers vinyliques, les maléimides, la vinylpyridine, le vinylimidazole, les composés polaires vinyliques et hétérocycliques, le sulfonate de styrène, l'alcool allylique, l'alcool vinylique, le vinylcaprolactame ou des mélanges de ceux-ci. Les monomères (B) sont choisis de préférence au sein du groupe constitué par l'acide acrylique, le N,N-diméthylacrylamide, le méthacrylate de diméthylaminoéthyle, le méthacrylate de diméthylaminoéthyle quaternisé, la vinylpyrrolidone, et des mélanges de ceux-ci.

On cite notamment le produit KP 561 ou le KP 562 commercialisé par SHIN ETSU tel que le monomère (A) et choisi parmi les esters d'alcool en $C_{18}$-$C_{22}$ et de l'acide méthacrylique.

Les macromères de polysiloxane (C) de formule (XXVII) sont choisis de préférence parmi ceux correspondant à la formule générale (XXVIII) suivante :

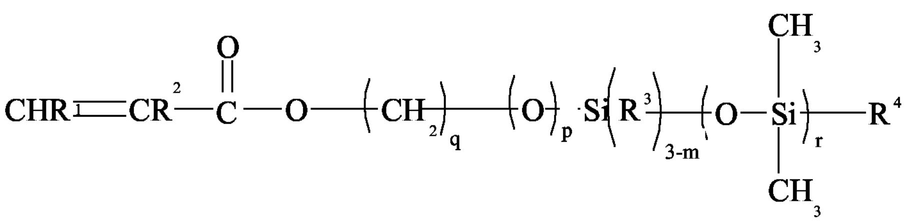
(XXVIII)

dans laquelle :

- $R^1$ est hydrogène ou -COOH (préférablement hydrogène) ;
- $R^2$ est hydrogène, méthyle ou $-CH_2COOH$ (préférablement méthyle) ;
- $R^3$ est alkyle, alkoxy ou alkylamino en $C_1$-$C_6$, aryle en $C_6$-$C_{12}$ ou hydroxyle (préférablement méthyle) ;
- $R^4$ est alkyle, alkoxy ou alkylamino en $C_1$-$C_6$, aryle en $C_6$-$C_{12}$ ou hydroxyle (préférablement méthyle) ;
- q est un nombre entier allant de 2 à 6 (préférablement 3) ;
- p vaut 0 ou 1 ;
- r est un nombre entier allant de 5 à 700 ;
- m est un nombre entier allant de 1 à 3 (préférablement 1).

On utilise de préférence les macromères de polysiloxane de formule (XXIX) :

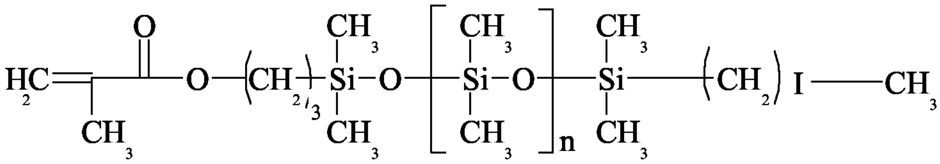
(XXIX)

n étant un nombre allant de 5 à 700 et 1 étant un nombre entier compris entre 0 et 3.

Un mode de réalisation de l'invention consiste en l'utilisation d'un copolymère capable d'être obtenu par une

polymérisation radicalaire à partir du mélange de monomères constitué par :

a) 60% en poids d'acrylate de tertio-butyle ;
b) 20% en poids d'acide acrylique ;
c) 20% en poids de macromère siliconé de formule (XXX) :

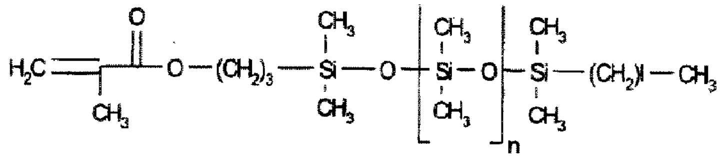

(XXX)

n étant un nombre allant de 5 à 700 et l étant un nombre entier compris entre 0 et 3, les pourcentages en poids étant calculés par rapport au poids total des monomères.

Un autre mode de réalisation particulier de l'invention consiste en l'utilisation d'un copolymère capable d'être obtenu par une polymérisation radicalaire à partir du mélange de monomères constitué par :

a) 80% en poids d'acrylate de tertio-butyle ;
b) 20% en poids de macromère siliconé de formule (XXXI) :

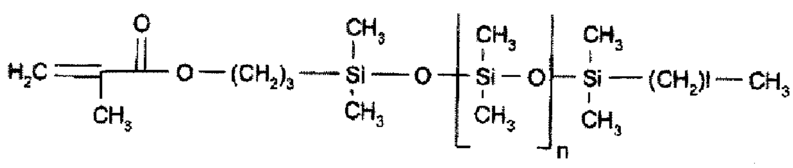

(XXXI)

n étant un nombre allant de 5 à 700 et l étant un nombre entier compris entre 0 et 3, les pourcentages en poids étant calculés par rapport au poids total des monomères.

Une autre famille particulière de polymères siliconés greffés ayant un squelette organique non siliconé convenable pour une mise en oeuvre de la présente invention est constituée par les copolymères siliconés greffés capables d'être obtenus par l'extrusion réactive d'un macromère de polysiloxane à fonction terminale réactive sur un polymère du type polyoléfine comprenant des groupements réactifs capables de réagir avec la fonction terminale du macromère de polysiloxane pour former une liaison covalente permettant le greffage de la silicone sur la chaîne principale de la polyoléfine. Ces polymères, ainsi que leur procédé de préparation, sont décrits dans la demande de brevet WO 95/00578.

Les polyoléfines réactives sont choisies de préférence parmi les polyéthylènes ou les polymères de monomères dérivés de l'éthylène, tels que le propylène, le styrène, l'alkylstyrène, le butylène, le butadiène, les (méth)acrylates, les esters vinyliques ou équivalents, comprenant des fonctions réactives capables de réagir avec la fonction terminale du macromère de polysiloxane. Ils sont choisis plus particulièrement parmi les copolymères d'éthylène ou de dérivés d'éthylène et les monomères choisis parmi ceux comprenant une fonction carboxylique tels que l'acide (méth)acrylique ; ceux comprenant une fonction anhydride d'acide tels que l'anhydride de l'acide maléique ; ceux comprenant une fonction chlorure d'acide tels que le chlorure de l'acide (méth)acrylique ; ceux comprenant une fonction ester tels que les esters de l'acide (méth)acrylique ; et ceux comportant une fonction isocyanate.

Les macromères siliconés sont choisis de préférence parmi les polysiloxanes comprenant un groupement fonctionnalisé, à l'extrémité de la chaîne de polysiloxane ou à proximité de l'extrémité de ladite chaîne, choisis au sein du groupe constitué par les alcools, les thiols, les époxy, les amines primaires et secondaires, et tout particulièrement parmi ceux correspondant à la formule générale (XXXII):

$$T\text{-}(CH_2)_6\text{-}Si\text{-}[\text{-}(OSiR^5R^6)_t\text{-}R^7]_y \qquad \text{(XXXII)}$$

dans laquelle T est choisi parmi le groupe constitué par $NH_2$, NHRN, une fonction époxy, OH, SH ; $R^5$, $R^6$, $R^7$ et RN, indépendamment, désignent alkyle en $C_1$-$C_6$, phényle, benzyle ou alkylphényle en $C_6$-$C_{12}$, hydrogène ; s est un nombre allant de 2 à 00, t est un nombre allant de 0 à 1000 et y est un nombre allant de 1 à 3. Ils ont un poids moléculaire

moyen en nombre allant préférablement de 5000 à 300 000, plus préférablement de 8000 à 200 000, et plus particulièrement de 9000 à 40 000.

Selon un mode de réalisation particulier, le polymère filmogène peut être obtenu auprès de la MINNESOTA MINING AND MANUFACTURING COMPANY sous les dénominations commerciales de polymères « SILICONE PLUS ». Par exemple, le poly(méthacrylate d'isobutyle-co-FOSEA de méthyle)-g-poly(diméthylsiloxane) est commercialisé sous la dénomination commerciale SA 70-5 IBMMF.

*2) Polymère à squelette siliconé*

Selon une autre forme préférée de l'invention, le polymère filmogène est choisi parmi les polymères siliconés greffés par des monomères organiques non siliconés. Ces polymères peuvent être liposolubles, lipodispersables, hydrosolubles ou dispersables en milieu aqueux, le cas échéant.

Ledit polymère ou lesdits polymères siliconé(s) greffé(s) ayant un squelette de polysiloxane greffé par des monomères organiques non siliconés contenant une chaîne principale de silicone (ou de polysiloxane $(/SiO-)_n$) sur laquelle se trouve greffé, à l'intérieur de ladite chaîne ainsi qu'éventuellement à l'une au moins de ses extrémités, au moins un groupement organique ne comprenant pas de silicone.

Les polymères ayant un squelette de polysiloxane greffé par des monomères organiques non siliconés selon l'invention peuvent être des produits commerciaux existants ou bien ils peuvent être obtenus par tout moyen connu de l'homme de l'art, en particulier par une réaction entre (i) une silicone de départ correctement fonctionnalisée sur un ou plusieurs de ces atomes de silicium et (ii) un composé organique non siliconé lui-même correctement fonctionnalisé par une fonction qui est capable de réagir avec le groupement ou les groupements fonctionnel(s) porté(s) par ladite silicone en formant une liaison covalente ; un exemple classique d'une telle réaction est la réaction d'hydrosylilation entre des groupements /Si-H et des groupements vinyliques $CH_2$=CH-, voire la réaction entre des groupements thio-fonctionnels -SH avec ces mêmes groupements vinyle.

Des exemples de polymères ayant un squelette de polysiloxane greffé par des monomères organiques non siliconés convenables pour une mise en oeuvre de la présente invention, ainsi que leur méthode spécifique de préparation, sont décrits en particulier dans les demandes de brevet EP-A-0 582 152, WO 93/23009 et WO 95/03776, dont les enseignements sont totalement inclus dans la présente description à titre de références non limitatives.

Selon un mode de réalisation particulièrement préféré de la présente invention, le polymère siliconé, ayant un squelette de polysiloxane greffé par des monomères organiques non siliconés, mis en oeuvre, est constitué du résultat d'une copolymérisation radicalaire entre, d'une part, au moins un monomère organique anionique non siliconé à insaturation éthylénique et/ou un monomère organique hydrophobe non siliconé à insaturation éthylénique et, d'autre part, une silicone présentant dans sa chaîne au moins un groupement fonctionnel, et préférablement plusieurs, capable de réagir avec lesdites insaturations éthyléniques desdits monomères non siliconés en formant une liaison covalente, en particulier des groupements thio-fonctionnels.

Selon la présente invention, lesdits monomères anioniques à insaturation éthylénique sont de préférence choisis, seuls ou comme mélanges, parmi les acides carboxyliques insaturés, linéaires ou ramifiés, éventuellement neutralisés partiellement ou totalement sous forme d'un sel, ce ou ces acide(s) carboxylique(s) insaturé(s) pouvant être plus particulièrement l'acide acrylique, l'acide méthacrylique, l'acide maléique, l'acide itaconique, l'acide fumarique et l'acide crotonique. Les sels convenables sont en particulier les sels alcalins, alcalino-terreux et d'ammonium. On notera que, de même, dans le polymère siliconé greffé final, le groupement organique de nature anionique qui est constitué du résultat de l'(homo)polymérisation radicalaire d'au moins un monomère anionique du type acide carboxylique insaturé peut être, après réaction, post-neutralisé par une base (soude, ammoniaque, etc.) pour l'amener sous la forme d'un sel.

Selon la présente invention, les monomères hydrophobes à insaturation éthylénique sont de préférence choisis, seuls ou comme mélange, parmi les esters de l'acide acrylique d'alcanols et/ou les esters de l'acide méthacrylique d'alcanols. Les alcanols sont de préférence en $C_1$ à $C_{30}$ et plus particulièrement en $C_1$ à $C_{22}$. Les monomères préférés sont choisis parmi le groupe constitué par le (méth)acrylate d'isooctyle, le (méth)acrylate d'isononyle, le (méth)acrylate de 2-éthylhexyle, le (méth)acrylate de lauryle, le (méth)acrylate d'isopentyle, le (méth)acrylate de n-butyle, le (méth)acrylate d'isobutyle, le (méth)acrylate de méthyle, le (méth)acrylate de tertio-butyle, le (méth)-acrylate de tridécyle et le (méth)acrylate de stéaryle, ou des mélanges de ceux-ci.

Une famille de polymères siliconés ayant un squelette de polysiloxane greffé par des monomères organiques non siliconés, convenant particulièrement bien à la mise en oeuvre de la présente invention, est constituée par les polymères siliconés comprenant dans leur structure le motif de formule (XXXIII) ci-dessous :

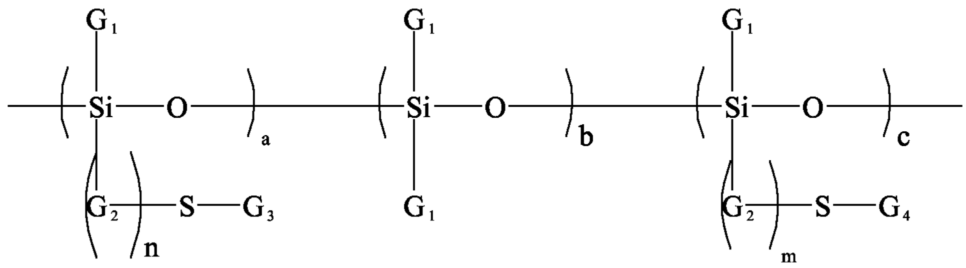

(XXXIII)

dans laquelle les radicaux $G_1$, identiques ou différents, représentent hydrogène ou un radical alkyle en $C_1$-$C_{10}$, voire un radical phényle ; les radicaux $G_2$, identiques ou différents, représentent un groupement alkylène en $C_1$-$C_{10}$; $G_3$ représente un reste polymère résultant de l'(homo)polymérisation d'au moins un monomère anionique à insaturation éthylénique ; $G_4$ représente un reste polymère résultant de l'(homo)polymérisation d'au moins un monomère d'au moins un monomère hydrophobe [sic] à insaturation éthylénique ; m et n sont égaux à 0 ou 1 ; a est un nombre entier allant de 0 à 50 ; b est un nombre entier pouvant être compris entre 10 et 350, c est un nombre entier allant de 0 à 50 ; à condition que l'un des paramètres a et c soit différent de 0.

Le motif de formule (XXXIII) du texte ci-dessus possède de préférence au moins une, et encore plus préférablement l'ensemble, des caractéristiques suivantes :

- les radicaux $G_1$ désignent un radical alkyle, préférablement un radical méthyle ;
- n ne vaut pas zéro, et les radicaux $G_2$ représentent un radical divalent en $C_1$-$C_3$, préférablement un radical propylène ;
- $G_3$ représente un radical polymère résultant de l'(homo)polymérisation d'au moins un monomère du type acide carboxylique à insaturation éthylénique, préférablement l'acide acrylique et/ou l'acide méthacrylique ;
- $G_4$ représente un radical polymère résultant de l'(homo)polymérisation d'au moins un monomère du type (méth)acrylate d'alkyle en $C_1$-$C_{10}$, préférablement le (méth)acrylate d'isobutyle ou de méthyle.

Des exemples de polymères siliconés correspondant à la formule (XXXIII) sont en particulier des polydiméthylsiloxanes (PDMS) sur lesquels sont greffés, par l'intermédiaire d'une liaison secondaire du type thiopropylène, des motifs polymères mixtes du type acide poly(méth)acrylique et du type poly(méth)acrylate d'alkyle.

D'autres exemples de polymères siliconés correspondant à la formule (XXXIII) sont en particulier des polydiméthylsiloxanes (PDMS) sur lesquels sont greffés, par l'intermédiaire d'une liaison secondaire du type thiopropylène, des motifs polymères du type poly(méth)acrylate d'isobutyle.

De tels polymères comportent les polymères comprenant au moins un groupement de formule (XXXIV) :

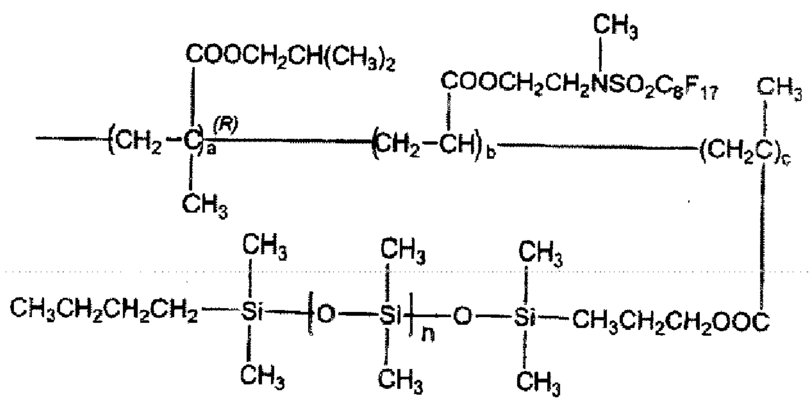

(XXXIV)

dans laquelle :

a, b et c, pouvant être identiques ou différents, sont chacun un nombre allant de 1 à 100 000 ; et les groupements terminaux, pouvant être identiques ou différents, sont choisis chacun parmi les groupements alkyle linéaires en $C_1$ à $C_{20}$, les groupements alkyle à chaîne ramifiée en $C_3$ à $C_{20}$, les groupements aryle en $C_3$ à $C_{20}$, les groupements alkoxy linéaires en $C_1$ à $C_{20}$ et les groupements alkoxy ramifiés en $C_3$ à $C_{20}$.

De tels polymères sont divulgués dans les brevets US n° 4 972 037, 5 061 481, 5 209 924, 5 849 275 et 6 033

650, et WO 93/23446 et WO 95/06078.

Une autre famille de polymères siliconés ayant un squelette de polysiloxane greffé par des monomères organiques non siliconés, convenant particulièrement bien à la mise en oeuvre de la présente invention, est constituée par les polymères siliconés comprenant dans leur structure le motif de formule (XXXV) ci-dessous :

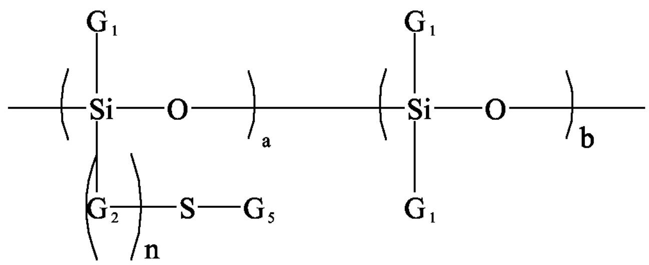 (XXXV)

dans laquelle les radicaux $G_1$ et $G_2$ ont la même signification que ci-dessus ; $G_5$ représente un reste polymère résultant de l'(homo)polymérisation d'au moins un monomère hydrophobe à insaturation éthylénique ou de la copolymérisation d'au moins un monomère anionique à insaturation éthylénique et d'au moins un monomère hydrophobe à insaturation éthylénique ; n est égal à 0 ou 1 ; a est un nombre entier allant de 0 à 50 ; b est un nombre entier pouvant être compris entre 10 et 350 ; à condition que a soit différent de 0.

Le motif de formule (XXXV) du texte ci-dessus possède de préférence au moins une, et encore plus préférablement l'ensemble, des caractéristiques suivantes :

- les radicaux $G_1$ désignent un radical alkyle, préférablement un radical méthyle ;
- n ne vaut pas zéro, et les radicaux $G_2$ représentent un radical divalent en $C_1$-$C_3$, préférablement un radical propylène.

La masse moléculaire en nombre des polymères siliconés ayant un squelette de polysiloxane greffé par des monomères organiques non siliconés de l'invention varie préférablement d'environ 10 000 à 1 000 000, et encore plus préférablement d'environ 10 000 à 100 000.

Selon un mode de réalisation particulier, un polymère filmogène siliconé convenant particulièrement à la mise en oeuvre de la présente invention, peut être un copolymère comportant des groupements carboxylates et des groupements polydiméthylsiloxanes.

Par « copolymère comportant des groupements carboxylates et des groupements polydiméthylsiloxanes », on entend dans la présente demande, un copolymère obtenu à partir de (a) un ou plusieurs monomères carboxyliques (acide ou ester), et (b) une ou plusieurs chaînes polydiméthylsiloxane (PDMS).

On entend dans la présente demande par « monomère carboxylique » aussi bien les monomères d'acide carboxylique que les monomères d'ester d'acide carboxylique. Ainsi, le monomère (a) peut être choisi par exemple parmi l'acide acrylique, l'acide méthacrylique, l'acide maléique, l'acide fumarique, l'acide itaconique, l'acide crotonique, leurs esters et les mélanges de ces monomères. Comme esters, on peut citer les monomères suivants : acrylate, méthacrylate, maléate, fumarate, itaconoate et/ou crotonoate. Les monomères sous forme d'esters sont plus particulièrement choisis parmi les acrylates et méthacrylates d'alkyle linéaire ou ramifié de préférence en $C_1$-$C_{24}$ et mieux en $C_1$-$C_{22}$, le radical alkyle étant préférentiellement choisi parmi les radicaux méthyle, éthyle, stéaryle, butyle, éthyl-2-hexyle, et leurs mélanges.

Le copolymère peut comprendre comme groupements carboxylates, au moins un groupement choisi parmi l'acide acrylique, l'acide méthacrylique, les acrylates ou méthacrylates de méthyle, d'éthyle, de stéaryle, de butyle, d'éthyl-2-hexyle, et leurs mélanges.

On entend désigner par « polydiméthylsiloxanes » (appelé aussi organopolysiloxanes ou, en abréviation, PDMS), en conformité avec l'acceptation générale, tout polymère ou oligomère organosilicié à structure linéaire, de poids moléculaire variable, obtenus par polymérisation et/ou polycondensation de silanes convenablement fonctionnalisés, et constitués pour l'essentiel par une répétition de motifs principaux dans lesquels les atomes de silicium sont reliés entre eux par des atomes d'oxygène (liaison siloxane ≡Si-O-Si≡), comportant des radicaux triméthyle directement liés par l'intermédiaire d'un atome de carbone sur lesdits atomes de silicium. Les chaînes PDMS pouvant être utilisées pour obtenir le copolymère comportent au moins un groupe radical polymérisable, de préférence situé sur au moins l'une des extrémités de la chaîne, c'est-à-dire que le PDMS peut avoir par exemple un groupe radical polymérisable sur les deux extrémités de la chaîne ou avoir un groupe radical polymérisable sur une extrémité de la chaîne et un groupement terminal triméthylsilyle sur l'autre extrémité de la chaîne. Le groupe radical polymérisable peut être notamment un groupe acrylique ou méthacrylique, en particulier un groupe $CH_2 = CR_1 - CO - O - R_2$, où $R_1$ représente un hydrogène ou un groupe méthyle, et $R_2$ représente $-CH_2-$, $-(CH_2)_n-$ avec n = 3, 5, 8 ou 10, $-CH_2-CH(CH_3)-CH_2-$ ,

$-CH_2-CH_2-O-CH_2-CH_2-$, $-CH_2-CH_2-O-CH_2-CH_2-CH(CH_3)-CH_2-$, $-CH_2-CH_2-O-CH_2\ CH_2-O-CH_2-CH_2-CH_2-$.

Les copolymères utilisés sont généralement obtenus selon les méthodes usuelles de polymérisation et de greffage, par exemple par polymérisation radicalaire (A) d'un PDMS comportant au moins un groupe radical polymérisable (par exemple sur l'une des extrémités de la chaîne ou sur les deux) et (B) d'au moins un monomère carboxylique, comme décrit par exemple dans les documents US-A-5,061,481 et US-A-5,219,560.

Les copolymères obtenus ont généralement un poids molécule allant d'environ 3000 à 200 000 et de préférence d'environ 5000 à 100 000.

Le copolymère peut se présenter tel quel ou sous forme dispersée dans un solvant tel que les alcools inférieurs comportant de 2 à 8 atomes de carbone, comme l'alcool isopropylique, ou les huiles comme les huiles de silicone volatiles (par exemple cyclopentasiloxane).

Comme copolymères utilisables, on peut citer par exemple les copolymères d'acide acrylique et d'acrylate de stéaryle à greffons polydiméthylsiloxane, les copolymères de méthacrylate de stéaryle à greffons polydiméthylsiloxane, les copolymères d'acide acrylique et de méthacrylate de stéaryle à greffons polydiméthylsiloxane, les copolymères de méthacrylate de méthyle, méthacrylate de butyle, d'acrylate d'éthyl-2-hexyle et de méthacrylate de stéaryle à greffons polydiméthylsiloxane. On peut citer en particulier comme copolymère utilisable, les copolymères commercialisés par la société SHIN-ETSU sous les dénominations KP-561 (nom CTFA : acrylates/dimethicone), KP-541 où le copolymère est dispersé à 60 % en poids dans de l'alcool isopropylique (nom CTFA : acrylates/dimethicone and Isopropyl alcohol), KP-545 où le copolymère est dispersé à 30 % dans du cyclopentasiloxane (nom CTFA : acrylates/dimethicone and Cyclopentasiloxane). Selon un mode préféré de réalisation de l'invention, on utilise de préférence le KP561 ; ce copolymère n'est pas dispersé dans un solvant, mais se présente sous forme cireuse, son point de fusion étant d'environ 30°C.

Plus généralement, la quantité totale de polymère doit être en quantité suffisante pour former sur la peau et/ou les lèvres un film cohésif capable de suivre les mouvements de la peau et/ou des lèvres sans se décoller ou craquer.

Lorsque le polymère a une température de transition vitreuse trop élevée pour l'utilisation désirée, on peut y associer un plastifiant de façon à abaisser cette température du mélange utilisé. Le plastifiant peut être choisi parmi les plastifiants utilisés habituellement dans le domaine d'application, et notamment parmi les composés pouvant être des solvants pour le polymère.

Dans les compositions cosmétiques conformes à l'invention, la teneur en agents filmogènes peut varier de 0,1 à 20 % en poids, notamment de 0,5 à 20 % en poids, et en particulier de 1 à 20 % en poids, par rapport au poids total de la composition.

Avantageusement, l'agent filmogène est présent, dans les compositions cosmétiques conformes à l'invention, dans un rapport pondéral par rapport au poids des polymères de silicone de formule générale (I), inférieur à 5:1, en particulier inférieur à 1:1, et plus particulièrement inférieur ou égal à 1:2.

## COMPOSES SILICONES

On entend par « composé siliconé », au sens de la présente invention, des composés comprenant au moins un atome de silicium et distinct du polymère de silicone de formule générale (I).

La composition selon l'invention comprend avantageusement, outre le polymère de formule générale (I), un composé siliconé choisi parmi les huiles de silicone citées précédemment, les gommes de silicone, les résines de silicone citées précédemment, les élastomères de silicone, et leurs mélanges.

Selon un mode de réalisation particulier, les compositions selon l'invention comprennent avantageusement un composé siliconé fluide non volatil.

Lorsque le composé siliconé est une huile siliconée, il peut s'agir des huiles siliconées telles que défmies précédemment.

Lorsque le composé siliconé est une résine de silicone, il peut s'agir des résines de silicone telles que définies précédemment dans la section relative aux agents filmogènes.

Les compositions cosmétiques conformes à l'invention peuvent également comprendre une gomme siliconée.

On entend par gomme siliconée un composé fluide ou solide de masse moléculaire en poids supérieure ou égale à 200 000 g/mol, en particulier variant de 200 000 à 4 000 000, et plus particulièrement variant de 200 000 à 2 000 000 g/mol.

La viscosité de la gomme siliconée fluide peut varier dans la plage allant de 1 000 à 10 000 000 cSt, en particulier de 100 000 à 1 000 000 cSt, plus particulièrement de 300 000 à 700 000 cSt, mesurée selon la norme ASTM D-445.

La gomme siliconée est avantageusement un polymère non greffé, c'est à dire un polymère obtenu par polymérisation d'au moins un monomère, sans réaction subséquente des chaînes latérales avec un autre composé chimique. La gomme siliconée est de préférence choisie parmi les diméthiconols, les fluorosilicones, les diméthicones et leurs mélanges. La gomme siliconée est de préférence un homopolymère.

En particulier, on peut utiliser une gomme répondant à la formule suivante :

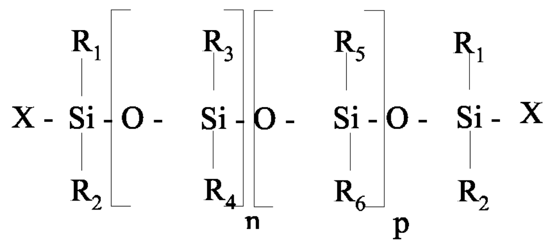

dans laquelle :

- $R_1$, $R_2$, $R_5$ et $R_6$ sont, indépendamment les uns des autres, un radical alkyle ayant 1 à 6 atomes de carbone, éventuellement substitué par au moins un atome de fluor,
- $R_3$ et $R_4$ sont, ensemble ou séparément, un radical alkyle ayant de 1 à 6 atomes de carbone ou un radical aryle,
- X est un radical alkyle ayant de 1 à 6 atomes de carbone, un radical hydroxyle, un radical vinyle, allyle ou un radical alkoxyle ayant de 1 à 6 atomes de carbone,
- n et p étant choisis de manière à ce que le composé siliconé ait une masse moléculaire en poids supérieure ou égale à 200 000 g/mol.
- p est avantageusement égal à 0.

Les polymères de la formule précédente tels $R_1$ à $R_6$ représentent un groupement méthyle et le substituant X représente un groupement hydroxyle sont des diméthiconols. On cite par exemple les polymères de formule (IX) tels que p=0 et n est compris entre 2 000 et 40 000, de préférence entre 3 000 et 30 000. On peut également citer les polymères ayant une masse moléculaire comprise entre 1 500 000 et 2 000 000 g/mol.

Selon un mode de mise en oeuvre, la gomme siliconée est la diméthiconol commercialisée par DOW CORNING dans une polydiméthylsiloxane (5 cSt) sous la référence D2-9085, la viscosité du mélange étant égale à 1 550 cSt, ou la diméthiconol commercialisée par DOW CORNING dans une polydiméthylsiloxane (5cS) sous la référence DC 1503. On préfère également la diméthiconol (de poids moléculaire égal à 1 770 000 g/mol) commercialisée par DOW CORNING sous la référence Q2-1403 ou Q2-1401, la viscosité du mélange étant égale à 4 000 cSt.

Comme gomme siliconée utilisable selon l'invention, on peut citer également celles pour lesquelles :

- les substituants $R_1$ à $R_6$ et X représentent un groupement méthyle, comme celle vendue sous la dénomination SE30 par la société GENERAL ELECTRIC, et celle vendue sous la dénomination AK 500000 par la société WAKER,
- les substituants $R_1$ à $R_6$ et X représentent un groupement méthyle, p et n sont tels que le poids moléculaire est de 250 000 g/mol, comme celle vendue sous la dénomination SILBIONE 70047 V par la société RHODIA,
- les substituants $R_1$ à $R_6$ représentent un groupement méthyle, le substituant X représente un groupement hydroxyle, comme celle vendue sous la dénomination Q2-1401 ou Q2-1403 par la société DOW CORNING,
- les substituants $R_1$, $R_2$, $R_5$, $R_6$ et X représentent un groupement méthyle, les substituants $R_3$ et $R_4$ représentent un groupement aryle, n et p sont tels que le poids moléculaire du polymère soit de 600 000 g/mol, comme celle vendue sous la dénomination 761 par la société RHODIA-CHIMIE.

La gomme siliconée peut être introduite dans la composition sous la forme d'un mélange avec une huile siliconée, la viscosité de ladite huile variant de 0,5 à 10 000 cSt, en particulier de 0,5 à 500 cSt, et plus particulièrement de 1 à 10 cSt.

L'huile siliconée en mélange avec la gomme siliconée peut être choisie parmi les polyalkylsiloxanes, les polyarylsiloxanes, les polyalkylarylsiloxanes et leurs mélanges. La silicone liquide peut être une silicone volatile telle qu'une polydiméthylsiloxane cyclique comprenant 3 à 7 motifs $-(CH_3)_2SiO-$.

L'huile siliconée peut également être une silicone non volatile polydiméthylsiloxane, notamment de viscosité comprise entre 8 et 10 000 cSt, de préférence encore de l'ordre de 10 cSt, par exemple la silicone commercialisée sous la référence DC 200 par DOW CORNING.

La proportion de la gomme siliconée dans le mélange gomme/huile est de préférence comprise entre 10/90 et 20/80. La viscosité du mélange gomme/huile est de préférence comprise entre 1 000 et 10 000 cSt.

Les diméthicones de haut poids moléculaire selon l'invention incluent les diméthicones décrites dans le brevet US 4 152 416. Elles sont par exemple commercialisées sous les références SE30, SE33, SE 54 et SE 76.

Les diméthicones selon l'invention peuvent être également des composés comformes à la formule précédente telle que $R_1$ à $R_6$ et X sont des méthyles et p=0. Le poids moléculaire de ces composés est de préférence compris entre 200 000 et 300 000, de préférence entre 240 000 et 260 000 g/mol.

Les diméthicones selon l'invention incluent également les polydiméthylsiloxanes, les copolymères (polydiméthylsiloxane)(méthylvinylsiloxane), les copolymères poly(diméthylsiloxane)(diphényl)(méthylvinylsiloxane), et leurs mélanges

Les gommes fluorosilicones de haut poids moléculaire selon l'invention ont en particulier un poids moléculaire variant de 200 000 à 300 000, et en particulier de 240 000 à 260 000 g/mol.

Le composé siliconé utilisable dans les compositions cosmétiques selon la présente invention peut également être un élastomère de silicone. L'élastomère de silicone est avantageusement un élastomère de silicone polyglycérolé. Ce dernier est distinct du polymère de silicone de formule générale (I).

L'élastomère de silicone polyglycérolé pouvant être utilisé pour la formulation des compositions selon l'invention est un organopolysiloxane réticulé élastomère susceptible d'être obtenu par réaction d'addition réticulation de diorganopolysiloxane contenant au moins un hydrogène lié au silicium et de composés polyglycérolés ayant des groupements à insaturation éthylénique, notamment en présence de catalyseur platine.

En particulier, l'organopolysiloxane réticulé élastomère est obtenu par réaction d'addition réticulation (A) de diorganopolysiloxane contenant au moins deux hydrogènes liés chacun à un silicium, et (B) de composés glycérolés ayant au moins deux groupements à insaturation éthylénique, notamment en présence (C) de catalyseur platine.

En particulier, l'organopolysiloxane peut être obtenu par réaction de composé polyglycérolé à terminaisons diméthylvinylsiloxy et de méthylhydrogénopolysiloxane à terminaisons triméthylsiloxy, en présence de catalyseur platine.

Le composé (A) est le réactif de base pour la formation d'organopolysiloxane élastomère et la réticulation s'effectue par réaction d'addition du composé (A) avec le composé (B) en présence du catalyseur (C).

Le composé (A) est en particulier un organopolysiloxane ayant au moins 2 atomes d'hydrogène liés à des atomes de silicium distincts dans chaque molécule.

Le composé (A) peut présenter toute structure moléculaire, notamment une structure chaîne linéaire ou chaîne ramifiée ou une structure cyclique.

Le composé (A) peut avoir une viscosité à 25 °C allant de 1 à 50 000 centistokes, notamment pour être bien miscible avec le composé (B).

Les groupes organiques liés aux atomes de silicium du composé (A) peuvent être des groupes alkyles ayant de 1 à 18 atomes de carbone, tels que méthyle, éthyle, propyle, butyle, octyle, décyle, dodécyle (ou lauryle), myristyle, cétyle, stéaryle ; des groupes alkyles substitués tels que 2-phényléthyle, 2-phénylpropyle, 3,3,3-trifluoropropyle ; des groupes aryle tels que phényle, tolyle, xylyle ; des groupes aryle substitués tels que phényléthyle ; et des groupes hydrocarbonés monovalents substitués tels qu'un groupe époxy, un groupe ester carboxylate, ou un groupe mercapto. De préférence, ledit groupe organique est choisi parmi les groupes méthyle , phényle, lauryle.

Le composé (A) peut ainsi être choisi parmi les méthylhydrogénopolysiloxanes à terminaisons triméthylsiloxy, les copolymères diméthylsiloxane-méthylhydrogénosiloxane à terminaisons triméthylsiloxy, les copolymères cycliques diméthylsiloxane-méthylhydrogénosiloxane, les copolymères diméthylsiloxane-méthylhydrogénosiloxane-laurylméthylsiloxane à terminaisons triméthylsiloxy.

Le composé (B) peut être un composé polyglycérolé répondant à la formule (B') suivante :

$$C_mH_{2m-1}\text{-O-[Gly]n-}C_mH_{2m-1} \qquad (B')$$

dans laquelle m est un entier allant de 2 à 6, n est un entier allant de 2 à 200, de préférence allant de 2 à 100, de préférence allant de 2 à 50, de préférence n allant de 2 à 20, de préférence allant de 2 à 10, et préférentiellement allant de 2 à 5, et en particulier égal à 3 ; Gly désigne :

$$-CH_2\text{-}CH(OH)\text{-}CH_2\text{-O- ou -}CH_2\text{-}CH(CH_2OH)\text{-O-}$$

Avantageusement, la somme du nombre de groupements éthyléniques par molécule du composé (B) et du nombre d'atomes d'hydrogène liés à des atomes de silicium par molécule du composé (A) est d'au moins 4.

Il est avantageux que le composé (A) soit ajouté en une quantité telle que le rapport moléculaire entre la quantité totale d'atomes d'hydrogène liés à des atomes de silicium dans le composé (A) et la quantité totale de tous les groupements à insaturation éthylénique dans le composé (B) soit compris dans la gamme de 1/1 à 20/1.

Le composé (C) est le catalyseur de la réaction de réticulation, et est notamment l'acide chloroplatinique, les complexes acide chloroplatinique-oléfine, les complexes acide chloroplatinique-alkenylsiloxane, les complexes acide chloroplatinique-dicétone, le platine noir, et le platine sur support.

Le catalyseur (C) est de préférence ajouté de 0,1 à 1000 parts en poids, mieux de 1 à 100 parts en poids, en tant que métal platine propre pour 1000 parts en poids de la quantité totale des composés (A) et (B).

L'élastomère de silicone polyglycérolé peut être véhiculé sous forme de gel dans au moins une huile hydrocarbonée et/ou une huile siliconée. Dans ces gels, l'élastomère polyglycérolé est souvent à base de particules non-sphériques.

Comme élastomères de silicone polyglycérolés, on peut utiliser ceux vendus sous les dénominations "KSG-710" , "KSG-810", "KSG-820" , "KSG-830" "KSG-840" par la société SHIN-ETSU.

L'élastomère de silicone polyglycérolé peut être présent dans la composition en une teneur allant de 0,1 % à 50 % en poids, par rapport au poids total de la composition, en particulier allant de 0,1 % à 40 % en poids, en particulier de 0,5 % à 30 % en poids, en particulier de 0,5 % à 20 % en poids, et plus particulièrement de 1 % à 10 % en poids.

L'élastomère de silicone utilisable dans les compositions cosmétiques conformes à l'invention peut également être choisi parmi les élastomères siliconés émulsionnants.

L'élastomère de silicone utilisable dans les compositions cosmétiques conformes à l'invention peut également être choisi parmi les élastomères siliconés non émulsionnants.

Le terme "non émulsionnant" définit des élastomères organopolysiloxane ne contenant pas de chaîne hydrophile telle que motifs polyoxyalkylènes ou polyglycérolés.

L'élastomère de silicone non émulsionnant sphérique est un organopolysiloxane réticulé élastomère pouvant être obtenu par réaction d'addition réticulation de diorganopolysiloxane contenant au moins un hydrogène lié au silicium et de diorganopolysiloxane ayant des groupements à insaturation éthylénique liés au silicium, notamment en présence de catalyseur platine ; ou par réaction de condensation réticulation déhydrogénation entre un diorganopolysiloxane à terminaisons hydroxyle et un diorganopolysiloxane contenant au moins un hydrogène lié au silicium, notamment en présence d'un organoétain ; ou par réaction de condensation réticulation d'un diorganopolysiloxane à terminaisons hydroxyle et d'un organopolysilane hydrolysable ; ou par réticulation thermique d'organopolysiloxane, notamment en présence de catalyseur organopéroxyde ; ou par réticulation d'organopolysiloxane par radiations de haute énergie telles que rayons gamma, rayons ultraviolet, faisceau électronique.

De préférence, l'organopolysiloxane réticulé élastomère est obtenu par réaction d'addition réticulation (A2) de diorganopolysiloxane contenant au moins deux hydrogènes liés chacun à un silicium, et (B2) de diorganopolysiloxane ayant au moins deux groupements à insaturation éthylénique liés au silicium, notamment en présence (C2) de catalyseur platine, comme par exemple décrit dans la demande EP-A-295886.

En particulier, l'organopolysiloxane peut être obtenu par réaction de diméthylpolysiloxane à terminaisons diméthylvinylsiloxy et de méthylhydrogénopolysiloxane à terminaisons triméthylsiloxy, en présence de catalyseur platine.

Le composé (A2) est le réactif de base pour la formation d'organopolysiloxane élastomère et la réticulation s'effectue par réaction d'addition du composé (A2) avec le composé (B2) en présence du catalyseur (C2).

Le composé (A2) est avantageusement un diorganopolysiloxane ayant au moins deux groupes alkényles inférieur (par exemple en C2-C4) ; le groupe alkényle inférieur peut être choisi parmi les groupes vinyl, allyl, et propényl. Ces groupements alkényles inférieurs peuvent être situés en toute position de la molécule organopolysiloxane mais sont avantageusement situés aux extrémités de la molécule organopolysiloxane. L'organopolysiloxane (A2) peut avoir une structure chaîne ramifiée, chaîne linéaire, cyclique ou réseau mais la structure chaîne linéaire convient particulièrement. Le composé (A2) peut avoir une viscosité allant de l'état liquide à l'état de gomme. De préférence, le composé (A2) a une viscosité d'au moins 100 centistokes à 25 °C.

Les organopolysiloxanes (A2) peuvent être choisi parmi les méthylvinylsiloxanes, les copolymères méthylvinylsiloxane-diméthylsiloxane, les diméthylpolysiloxanes à terminaisons diméthylvinylsiloxy, les copolymères diméthylsiloxane-méthylphénylsiloxane à terminaisons diméthylvinylsiloxy, les copolymères diméthylsiloxane-diphénylsiloxane-méthylvinylsiloxane à terminaisons diméthylvinylsiloxy, les copolymères diméthylsiloxane-méthylvinylsiloxane à terminaisons triméthylsiloxy, les copolymères diméthylsiloxane-méthylphénylsiloxane-méthylvinylsiloxane à terminaisons triméthylsiloxy, les méthyl(3,3,3-trifluoropropyl)polysiloxane à terminaisons diméthylvinylsiloxy, et les copolymères diméthylsiloxane-méthyl(3,3,3-trifluoropropyl)siloxane à terminaisons diméthylvinylsiloxy.

Le composé (B2) est en particulier un organopolysiloxane ayant au moins 2 hydrogènes liés au silicium dans chaque molécule et est donc le réticulant du composé (A2).

Avantageusement, la somme du nombre de groupements éthyléniques par molécule du composé (A2) et le nombre d'atomes d'hydrogène liés au silicum par molécule du composé (B2) est d'au moins 4.

Le composé (B2) peut être sous toute structure moléculaire, notamment de structure chaîne linéaire, chaîne ramifiée, structure cyclique.

Le composé (B2) peut avoir une viscosité à 25 °C allant de 1 à 50 000 centistokes, notamment pour être bien miscible avec le composé (A).

Il est avantageux que le composé (B2) soit ajouté en une quantité telle que le rapport moléculaire entre la quantité totale d'atomes d'hydrogène liés au silicium dans le composé (B2) et la quantité totale de tous les goupements à insaturation éthylénique dans le composé (A2) aille dans la gamme de 1/1 à 20/1.

Le composé (B2) peut être choisi parmi les méthylhydrogénopolysiloxanes à terminaisons triméthylsiloxy, les copolymères diméthylsiloxane-méthylhydrogénosiloxane à terminaisons triméthylsiloxy, les copolymères cycliques diméthylsiloxane-méthylhydrogénosiloxane.

Le composé (C2) est le catalyseur de la réaction de réticulation, et est notamment l'acide chloroplatinique, les complexes acide chloroplatinique-oléfine, les complexes acide chloroplatinique-alkenylsiloxane, les complexes acide

chloroplatinique-dicétone, le platine noir, et le platine sur support.

Le catalyseur (C2) est de préférence ajouté de 0,1 à 1000 parts en poids, mieux de 1 à 100 parts en poids, en tant que métal platine propre pour 1000 parts en poids de la quantité totale des composés (A2) et (B2).

D'autres groupes organiques peuvent être liés au silicium dans les organopolysiloxane (A2) et (B2) décrits précédemment, comme par exemple des groupes alkyles tels que méthyl, éthyl, propyl, butyl, octyl ; des groupes alkyles substitués tels que 2-phényléthyl, 2-phénylpropyl, 3,3,3-trifluoropropyl ; des groupes aryles tels que phényl, tolyl, xylyl ; des groupes aryles substitutés tel que phényléthyl ; et des groupes hydrocarbonés monovalents substitués tels que un groupe époxy, un gropue ester carboxylate, un groupe mercapto.

Les particules d'organopolysiloxane réticulés élastomères peuvent être véhiculées sous forme de gel constitué d'un organopolysiloxane élastomérique inclus dans au moins une huile hydrocarbonée et/ou une huile siliconée. Dans ces gels, les particules d'organopolysiloxanes sont souvent des particules non-sphériques.

Les particules d'organopolysiloxane réticulés élastomères peuvent également se présenter sous forme de poudre, notamment sous forme de poudre sphérique.

Des élastomères non-émulsionnants sphériques sont notamment décrits dans les demandes JP-A-61-194009, EP-A-242219, EP-A-285886, EP-A-765656, dont le contenu est incorporé à titre de référence.

Comme élastomères non-émulsionnants sphériques, on peut utiliser ceux vendus sous les dénominations "DC 9040" , "DC9041" , "DC 9509" , "DC9505" , "DC 9506" par la société DOW CORNING.

L'élastomère de silicone non émulsionnant sphérique peut se présenter également sous forme de poudre d'organopolysiloxane réticulé élastomère enrobée de résine de silicone, notamment de résine silsesquioxane, comme décrit par exemple dans le brevet US5538793 dont le contenu est incorporé à titre de référence. De tels élastomères sont vendus sous les dénomination "KSP-100" , "KSP-101" "KSP-102" "KSP-103" KSP-104", "KSP-105" par la société SHIN ETSU.

D'autres organopolysiloxane réticulé élastomère sous forme de poudres sphériques peuvent être des poudres de silicone hybride fonctionnalisé par des groupes fluoroalkyle, notamment vendues sous la dénomination "KSP-200" par la société SHIN ETSU ; des poudres de silicones hybride fonctionnalisé par des groupes phényl, notamment vendues sous la dénomination "KSP-300" par la société SHIN ETSU.

L'élastomère de silicone sphérique non émulsionnant peut être présent dans la composition en une teneur allant de 0,1 % à 95 % en poids, par rapport au poids total de la composition, en particulier allant de 0,5 % à 75 % en poids, en particulier allant de 1 % à 50 % en poids, en particulier allant de 1 % à 40 % en poids, et plus particulièrement allant de 1 % à 30 % en poids.

## ESTER HYDROCARBONE COURT

La composition selon l'invention peut contenir outre le polymère de silicone et l'agent filmogène, un ester court.

Au sens de la présente invention, on entend par « ester hydrocarboné court », un ester hydrocarboné comprenant moins de 40 atomes de carbone.

Les compositions cosmétiques peuvent comprendre au moins un ester hydrocarboné court.

Les esters conformes à l'invention peuvent être des monoesters, des diesters ou des polyesters et sont plus particulièrement des monoesters c'est-à-dire portant une unique fonction ester. Ces esters peuvent être linéaires, ramifiés ou cycliques, saturés ou insaturés. En particulier, ils sont ramifiés et saturés. Ils peuvent également être volatils ou non volatils.

En particulier, les esters hydrocarbonés peuvent répondre à la formule RCOOR' dans laquelle RCOO représente un reste d'acide gras comportant de 2 à 28 atomes de carbone, et R' représente une chaîne hydrocarbonée contenant de 1 à 28 atomes de carbone. Plus particulièrement les groupements R et R' sont tels que l'ester correspondant est non volatil.

De manière avantageuse, les esters hydrocarbonés mono- di- ou polyesters, utilisables dans les compositions cosmétiques conformes à l'invention, comprennent moins de 40 atomes de carbone,et plus de 10 atomes de carbone.

Ces esters non volatils peuvent notamment être en $C_{10}$ à $C_{25}$ et en particulier en $C_{14}$ à $C_{22}$. Ils peuvent être choisis parmi des esters des acides en $C_2$ à $C_{18}$ et notamment, des alcools en $C_2$ à $C_{20}$ ou de polyols en $C_2$ à $C_8$ ou de leurs mélanges.

Ils comportent avantageusement moins de 22 atomes de carbone.

Selon un mode de réalisation particulier, lorsque l'ester hydrocarboné comprend moins de 22 atomes de carbone, il n'est pas une huile volatile.

Ainsi, les esters peuvent être choisis parmi une liste non limitative comprenant les esters de l'acide néopentanoïque comme le néopentanoate d'isodécyle, le néopentanoate d'isotridécyle, le néopentanoate d'isostéaryle, le néopentanoate d'octyldocécyle, les esters de l'acide isononanoïque comme l'isononanoate d'isononyle, l'isononanoate d'octyle, l'isononanoate d'isodécyle, l'isononanoate d'isotridécyle, l'isononanoate d'isostéaryle, mais aussi les esters de l'alcool isopropylique, tels que le myristate d'isopropyle, le palmitate d'isopropyle, le stéarate ou l'isostéarate d'iso-

propyle, l'octanoate de cétyle, l'octanoate de tridécyle, le 4-diheptanoate et le palmitate d'éthyle 2-hexyle, le benzoate d'alkyle, le diheptanoate de polyéthylène glycol, le diéthyl 2-d'hexanoate de propylèneglycol et leurs mélanges. Ledit ester peut être également choisi parmi les esters de synthèse notamment d'acide gras comme l'huile de purcellin, le myristate d'isopropyle, le palmitate d'éthyle, le stéarate d'octyle ; les esters hydroxylés comme le lactate d'isostéaryle, l'hydroxystéarate d'octyle, l'adipate de diisopropyle, les heptanoates, octanoates, décanoates d'alcool gras et leurs mélanges.

L'isononanoate d'isononyle, l'isononanoate d'isotridécyle, l'heptanoate de stéaryle, et leurs mélanges conviennent tout particulièrement à la réalisation de l'invention.

Selon un mode de réalisation particulier, l'ester hydrocarboné court utilisé dans la composition cosmétique conforme à la présente invention est un mélange d'isononanoate d'isononyle, d'isononanoate d'isotridécyle et d'héptanoate de stéaryle.

Cet ou ces ester(s) hydrocarboné(s) peut(peuvent) être utilisé(s) dans la composition à raison de 5 à 90 %, notamment de 10 à 60 % et en particulier de 20 à 50 % en poids par rapport au poids total de la composition.

## PHASE GRASSE

Les compositions cosmétiques conformes à la présente invention peuvent comprendre une phase grasse comprenant notamment des huiles et des corps gras solides à température ambiante (20 - 25 °C) et pression atmosphérique.

On entend par huile, tout corps gras sous forme liquide à température ambiante (20 - 25 °C) et à pression atmosphérique. La phase grasse liquide peut, également, contenir outre des huiles, d'autres composés solubilisés dans les huiles tels que des agents gélifiants et/ou structurants.

La composition cosmétique selon la présente invention peut comprendre au moins une, et en particulier au moins deux huiles.

La ou les huiles peuvent être présentes à raison de 0,1 à 99 % en poids, en particulier d'au moins 1 à 90 % en poids, plus particulièrement de 5 à 70 % en poids, notamment de 10 à 60 % en poids, voire de 20 à 50 % en poids, par rapport au poids total de la composition cosmétique selon l'invention.

Les huiles convenant à la préparation des compositions cosmétiques selon l'invention peuvent être des huiles volatiles ou non, siliconées ou non.

Au sens de la présente invention, on entend par « huile volatile », une huile (ou milieu non aqueux) susceptible de s'évaporer au contact de la peau en moins d'une heure, à température ambiante et à pression atmosphérique. L'huile volatile est une huile cosmétique volatile, liquide à température ambiante, ayant notamment une pression de vapeur non nulle, à température ambiante et pression atmosphérique, en particulier ayant une pression de vapeur allant de 0,13 Pa à 40 000 Pa (10-3 à 300 mm Hg), et de préférence allant de 1,3 Pa à 13 000 Pa (0,01 à 100 mm Hg), et préférentiellement allant de 1,3 Pa à 1300 Pa (0,01 à 10 mm Hg).

Au sens de la présente invention, on entend par « huile non-volatile », une huile ayant une pression de vapeur inférieure à 0,13 Pa. Les huiles volatiles ou non volatiles peuvent être des huiles hydrocarbonées notamment d'origine animale ou végétale, des huiles synthétiques, des huiles siliconées, des huiles fluorées, ou leurs mélanges.

Au sens de la présente invention, on entend par « huile siliconé », une huile comprenant au moins un atome de silicium, et notamment au moins un groupe Si-O.

On entend par "huile hydrocarbonée", une huile contenant principalement des atomes d'hydrogène et de carbone et éventuellement des atomes d'oxygène, d'azote, de soufre et/ou de phosphore.

Les huiles hydrocarbonées volatiles peuvent être choisies parmi les huiles hydrocarbonées ayant de 8 à 16 atomes de carbone, et notamment les alcanes ramifiés en $C_8$-$C_{16}$ (appelées aussi isoparaffines) comme l'isododécane (encore appelé 2,2,4,4,6-pentaméthylheptane), l'isodécane, l'isohexadécane, et par exemple les huiles vendues sous les noms commerciaux d'ISOPARS® ou de PERMETHYLS®.

Comme huiles volatiles, on peut aussi utiliser les silicones volatiles, comme par exemple les huiles de silicones linéaires ou cycliques volatiles, notamment celles ayant une viscosité $\leq$ 8 centistokes ($8 \times 10^{-6}$ $m^2/s$), et ayant notamment de 2 à 10 atomes de silicium, et en particulier de 2 à 7 atomes de silicium, ces silicones comportant éventuellement des groupes alkyle ou alkoxy ayant de 1 à 10 atomes de carbone. Comme huile de silicone volatile utilisable dans l'invention, on peut citer notamment les diméthicones de viscosité 5 et 6 cSt, l'octaméthyl cyclotétrasiloxane, le décaméthyl cyclopentasiloxane, le dodécaméthyl cyclohexasiloxane, l'heptaméthyl hexyltrisiloxane, l'heptaméthyloctyl trisiloxane, l'hexaméthyl disiloxane, l'octaméthyl trisiloxane, le décaméthyl tétrasiloxane, le dodécaméthyl pentasiloxane, et leurs mélanges.

On peut également utiliser des huiles volatiles fluorées tels que le nonafluorométhoxybutane ou le perfluorométhylcyclopentane, et leurs mélanges.

La phase grasse des compositions cosmétiques selon la présente l'invention peut également comprendre au moins une huile volatile.

Selon un mode particulier de réalisation, les compositions cosmétiques selon l'invention comprennent moins de 30 % en poids, et en particulier moins de 15 %, en particulier moins de 10 %, et plus particulièrement moins de 5 %, en poids d'huile volatile, par rapport au poids total de la composition.

Selon un autre mode de réalisation, les compositions cosmétiques selon la présente invention sont exemptes d'huiles volatiles.

La phase grasse des compositions cosmétiques selon la présente invention peut également comprendre au moins une huile non volatile.

Les huiles non volatiles peuvent notamment être choisies parmi les huiles hydrocarbonées le cas échéant fluorées et/ou les huiles siliconées non volatiles.

Comme huile hydrocarbonée non volatile, on peut notamment citer :

- les huiles hydrocarbonées d'origine animale,
- les huiles hydrocarbonées d'origine végétale telles que les esters de phytostéaryle, tels que l'oléate de phytostéaryle, l'isostéarate de physostéaryle et le glutanate de lauroyl/octyldodécyle/phytostéaryle (AJINOMOTO, ELDEW PS203), les triglycérides constitués d'esters d'acides gras et de glycérol dont les acides gras peuvent avoir des longueurs de chaînes variées de $C_4$ à $C_{24}$, ces dernières pouvant être linéaires ou ramifiées, saturées ou insaturées ; ces huiles sont notamment des triglycérides héptanoïques ou octanoïques, les huiles de germe de blé, de tournesol, de pépins de raisin, de sésame, de maïs, d'abricot, de ricin, de karité, d'avocat, d'olive, de soja, d'amande douce, de palme, de colza, de coton, de noisette, de macadamia, de jojoba, de luzerne, de pavot, de potimarron, de courge, de cassis, d'onagre, de millet, d'orge, de quinoa, de seigle, de carthame, de bancoulier, de passiflore, de rosier muscat ; le beurre de karité ; ou encore les triglycérides des acides caprylique/caprique comme ceux vendus par la société STEARINERIES DUBOIS ou ceux vendus sous les dénominations MIGLYOL 810®, 812® et 818® par la société DYNAMIT NOBEL,
- les éthers de synthèse ayant de 10 à 40 atomes de carbone ;
- les hydrocarbures linéaires ou ramifiés, d'origine minérale ou synthétique tels que la vaseline, les polydécènes, le polyisobutène hydrogéné tel que le parléam, le squalane et leurs mélanges, et en particulier le polyisobutène hydrogéné,
- les esters de synthèse comme ceux décrits précédemment,
- les esters de polyols et les esters du pentaérythritol, comme le tétrahydroxystéarate/tétraisostéarate de dipentaérythritol,
- les esters de dimères diols et dimères diacides tels que les Lusplan DD-DA5® et Lusplan DD-DA7®, commercialisés par la société NIPPON FINE CHEMICAL et décrits dans la demande FR0302809 déposée le 6 mars 2003, dont le contenu est incorporé dans la présente demande par référence.
- les alcools gras liquides à température ambiante à chaîne carbonée ramifiée et/ou insaturée ayant de 12 à 26 atomes de carbone comme le 2-octyldodécanol, l'alcool isostéarylique, l'alcool oléique, le 2-hexyldécanol, le 2-butyloctanol, et le 2-undécylpentadécanol,
- les acides gras supérieurs tels que l'acide oléique, l'acide linoléique, l'acide linolénique et leurs mélanges, et
- les carbonates de di-alkyle, les 2 chaînes alkyles pouvant être identiques ou différentes, tel que le dicaprylyl carbonate commercialisé sous la dénomination CETIOL CC®, par COGNIS.

Les huiles de silicone non volatiles utilisables dans la composition selon l'invention peuvent être les polydiméthylsiloxanes (PDMS) non volatiles, les polydiméthylsiloxanes comportant des groupements alkyle ou alcoxy pendants et/ou en bouts de chaîne siliconée, groupements ayant chacun de 2 à 24 atomes de carbone, les silicones phénylées comme les phényl triméthicones, les phényl diméthicones, les phényl triméthylsiloxy diphénylsiloxanes, les diphényl diméthicones, les diphényl méthyldiphényl trisiloxanes, et les 2-phényléthyl triméthylsiloxysilicates, et leurs mélanges.

De manière avantageuse, l'huile présente dans les compositions cosmétiques conformes à la présente invention, est notamment choisie parmi le polyisobutène hydrogéné, l'heptanoate d'isostéaryle, l'isononanoate d'isononyle, l'isononanoate d'isotridécyle, le malate de di-isostéaryle, le tétrahydroxystéarate/tétraisostéarate de dipentaérythritol, le 2-octyldodécanol, et leurs mélanges.

Selon un mode de réalisation particulière, l'huile présente dans la composition cosmétique conforme à la présente invention est un mélange de polyisobutène hydrogéné, d'heptanoate d'isostéaryle, d'isononanoate d'isononyle, d'isononanoate d'isotridécyle, de malate de di-isostéaryle, de tétrahydroxystéarate/tétraisostéarate de dipentaérythritol et de 2-octyldodécanol.

Les huiles non volatiles peuvent être présentes dans les compositions selon l'invention en une teneur allant de 20 à 99 % en poids, notamment de 30 % à 80 % en poids, et en particulier de 40 % à 80 % en poids, par rapport au poids total de la composition.

Selon un mode particulier de réalisation, lorsque la phase grasse liquide des compositions cosmétiques selon la présente invention est une huile de silicone, celle-ci est présente en une teneur allant de 0 à 90 % en poids, notamment

de 0,1 à 80 % en poids, et en particulier de 2 à 80 % en poids, par rapport au poids total de la composition.

Selon un autre mode de réalisation, l'huile de silicone est présente dans les compositions cosmétiques conformes à la présente invention, selon un rapport pondéral, par rapport au polymère de silicone de formule générale (I), variant de 80:1 , et en particulier de 60:1, et plus particulièrement de 40:1.

La phase grasse liquide peut, le cas échéant, être épaissie, gélifiée ou structurée en y incorporant un agent gélifiant de phase grasse, tel que décrit dans la demande WO 2004/55080 publiée le 1$^{er}$ juillet 2004, dont le contenu est incorporé dans la présente demande par référence.

Les compositions selon l'invention peuvent également comprendre au moins un composé choisi parmi les cires , les corps gras pâteux, et leurs mélanges.

La cire est solide à température ambiante (25 ˚C), à changement d'état solide/liquide réversible, ayant une température de fusion supérieure à 30 ˚C pouvant aller jusqu'à 200 ˚C, une dureté supérieure à 0,5 MPa et présentant à l'état solide une organisation cristalline anisotrope. Elle peut être hydrocarbonée, fluorée et/ou siliconée et être d'origine animale, végétale, minérale ou synthétique. Elle peut être choisie par exemple parmi la cire d'abeille, la cire de Carnauba, la cire de Candelilla, les cires de paraffine, l'huile de ricin hydrogénée, les cires synthétiques comme les cires de polyéthylène (de préférence de poids moléculaire compris entre 400 et 600) ou de Fischer-Tropsch, les cires de silicone comme les alkyl- ou alkoxy-diméthicone ayant de 16 à 45 atomes de carbone, les cérésines ou les ozokérites, comme par exemple les isoparaffines dont le point de fusion est inférieur à 40 ˚C, tel que l'EMW-0003, commercialisé par la société NIPPON SEIROU, les oligomères d'$\alpha$-oléfine, tel que les polymères PERFORMA V® 825, 103 et 260, commercialisés par la société NEW PHASE TECHNOLOGIES ; les copolymères éthylène-propylène, tel que le PERFORMALENE® EP 700, et les cires microcristallines dont le point de fusion est supérieur à 85 ˚C, tel que les HI-MIC® 1070, 1080, 1090 et 3080, commercialisées par NIPPON SEIROU, et leurs mélanges.

De manière avantageuse, la cire utilisée dans les compositions cosmétiques conformes à l'invention, est choisie parmi les cires de polyéthylène, la cire de Candelilla, et leurs mélanges.

Selon un mode particulier de réalisation, les compositions cosmétiques selon la présente invention comprennent un mélange de cire de polyéthylène, et de cire de Candelilla.

Selon un mode particulier de mise en oeuvre, la ou les cires utilisées dans les compositions cosmétique conformes à la présente invention est ou sont présentes en une teneur variant d'environ 1,5 à environ 20 %, en particulier d'environ 3 à environ 15 %, en particulier d'environ 5 à environ 10 %, et plus particulièrement d'environ 6,5 à environ 8,5 % en poids par rapport au poids total de la composition.

Les compositions cosmétiques conformes à la présente invention peuvent également comprendre au moins un composé pâteux.

Par "pâteux" au sens de la présente invention, on entend un composé gras à changement d'état solide/liquide réversible, et comportant à la température de 23˚C une fraction liquide et une fraction solide. On entend également par pâteux, le polylaurate de vinyle.

Le composé pâteux au sens de l'invention présente avantageusement une dureté à 20 ˚C allant de 0,001 à 0,5 MPa, de préférence de 0,002 à 0,4 MPa.

Parmi les composés pâteux susceptibles d'être utilisés dans la composition selon l'invention, on peut citer les lanolines et les dérivés de lanoline comme les lanolines acétylées, les lanolines oxypropylénées ou le lanolate d'isopropyle, et leurs mélanges. On peut également utiliser des esters d'acides ou d'alcools gras, notamment ceux ayant 20 à 65 atomes de carbone comme le citrate de tri-isostéaryle ou de cétyle ; le propionate d'arachidyle ; le polylaurate de vinyle ; les esters du cholestérol comme les triglycérides d'origine végétale tels que les huiles végétales hydrogénées, les polyesters visqueux et leurs mélanges. Comme triglycéride d'origine végétale, on peut utiliser les dérivés d'huile de ricin hydrogénée, tels que le « THIXINR®» de Rheox.

On peut également citer les polyesters résultant de l'estérification d'un acide carboxylique et d'un ester acide hydroxycarboxylique aliphatique. Par exemple, le RISOCAST® DA-L (ester issu de la réaction d'estérification de l'huile de ricin hydrogéné avec de l'acide dilinoléïque dans des proportions de 2 pour 1) et le RISOCAST® DA-H (ester résultant de l'estérification de l'huile de ricin hydrogénée avec de l'acide isostéarique dans des proportions de 4 pour 3) commercialisés par la société japonaise KOKYU ALCOHOL KOGYO.

Comme composé pâteux convenant avantageusement à la formulation des compositions cosmétiques conformes à la présente invention, on peut faire mention des coco-glycérides hydrogénés.

On peut aussi citer les composés pâteux siliconés tels que les polydiméthylsiloxanes (PDMS) de hauts poids moléculaires et en particulier ceux ayant des chaînes pendantes du type alkyle ou alcoxy ayant de 8 à 24 atomes de carbone, et un point de fusion de 20-55˚C, comme les stéaryl diméthicones notamment ceux vendus par la société DOW CORNING sous les noms commerciaux de DC2503® et DC25514® et leurs mélanges.

## PHASE AQUEUSE

Selon certains aspects de la présente invention, la composition selon l'invention peut comprendre au moins

un milieu aqueux, constituant une phase aqueuse, qui peut former la phase continue de la composition.

La phase aqueuse peut être constituée essentiellement d'eau.

Elle peut également comprendre un mélange d'eau et de solvant organique miscible à l'eau (miscibilité dans l'eau supérieure à 50 % en poids à 25 °C) comme les monoalcools inférieurs ayant de 1 à 5 atomes de carbone tels que l'éthanol, l'isopropanol, les glycols ayant de 2 à 8 atomes de carbone tels que le propylène glycol, l'éthylène glycol, le 1,3-butylène glycol, le dipropylène glycol, les cétones en $C_3$-$C_4$, et les aldéhydes en $C_2$-$C_4$.

La phase aqueuse (eau et éventuellement le solvant organique miscible à l'eau) peut être présente à une teneur allant de 0,1 à 40 % en poids, notamment allant de 0,1 à 20 % en poids, et en particulier 0,1 à 10 % en poids, par rapport au poids total de la composition.

**MATIERES COLORANTES**

La composition cosmétique conforme à l'invention peut, avantageusement, incorporer un ou plusieurs agents de coloration, au moins une matière colorante, organique ou inorganique, notamment de type pigments ou nacres classiquement utilisée dans les compositions cosmétiques.

Par pigments, il faut comprendre des particules blanches ou colorées, minérales ou organiques, insolubles dans une solution aqueuse, destinées à colorer et/ou opacifier le film résultant.

Les pigments peuvent être présents à raison de 0,01 à 15 % en poids, notamment de 0,01 à 10 % en poids, et en particulier de 0,02 à 5 % en poids, par rapport au poids total de la composition cosmétique. Comme pigments minéraux utilisables dans l'invention, on peut citer les oxydes de titane, de zirconium ou de cérium ainsi que les oxydes de zinc, de fer ou de chrome, le bleu ferrique, le violet de manganèse, le bleu outremer et l'hydrate de chrome.

Il peut également s'agir de pigment ayant une structure qui peut être par exemple de type séricite/oxyde de fer brun/dioxyde de titane/silice. Un tel pigment est commercialisé par exemple sous la référence COVERLEAF NS ou JS par la société CHEMICALS AND CATALYSTS et présente un rapport de contraste voisin de 30.

La matière colorante peut encore comporter un pigment ayant une structure qui peut être par exemple de type microsphères de silice contenant de l'oxyde de fer. Un exemple de pigment présentant cette structure est celui commercialisé par la société MIYOSHI sous la référence PC BALL PC-LL-100 P, ce pigment étant constitué de microsphères de silice contenant de l'oxyde de fer jaune.

Parmi les pigments organiques utilisables dans l'invention, on peut citer le noir de carbone, les pigments de type D & C, les laques à base de carmin de cochenille, de baryum, strontium, calcium, aluminium ou encore les dicéto pyrrolopyrrole (DPP) décrits dans les documents EP-A-542669, EP-A-787730, EP-A-787731 et WO-A- 96/08537.

Par « nacres », il faut comprendre des particules colorées de toute forme, irisées ou non, notamment produites par certains mollusques dans leur coquille ou bien synthétisées et qui présentent un effet de couleur par interférence optique.

Les nacres peuvent être choisies parmi les pigments nacrés tels que le mica titane recouvert avec un oxyde de fer, le mica recouvert d'oxychlorure de bismuth, le mica titane recouvert avec de l'oxyde de chrome, le mica titane recouvert avec un colorant organique ainsi que les pigments nacrés à base d'oxychlorure de bismuth Il peut également s'agir de particules de mica à la surface desquelles sont superposées au moins deux couches successives d'oxydes métalliques et/ou de matières colorantes organiques.

On peut également citer, à titre d'exemple de nacres, le mica naturel recouvert d'oxyde de titane, d'oxyde de fer, de pigment naturel ou d'oxychlorure de bismuth.

Parmi les nacres disponibles sur le marché, on peut citer les nacres TIMICA, FLAMENCO et DUOCHROME (sur base de mica) commercialisées par la société ENGELHARD, les nacres TIMIRON commercialisées par la société MERCK, les nacres sur base de mica PRESTIGE commercialisées par la société ECKART et les nacres sur base de mica synthétique SUNSHINE commercialisées par la société SUN CHEMICAL.

Les nacres peuvent plus particulièrement posséder une couleur ou un reflet jaune, rose, rouge, bronze, orangé, brun, or et/ou cuivré.

A titre illustratif des nacres pouvant être mises en oeuvre dans le cadre de la présente invention, on peut notamment citer les nacres de couleur or notamment commercialisées par la société ENGELHARD sous le nom de Brillant gold 212G (Timica), Gold 222C (Cloisonne), Sparkle gold (Timica), Gold 4504 (Chromalite) et Monarch gold 233X (Cloisonne) ; les nacres bronzes notamment commercialisées par la société MERCK sous la dénomination Bronze fine (17384) (Colorona) et Bronze (17353) (Colorona) et par la société ENGELHARD sous la dénomination Super bronze (Cloisonne) ; les nacres oranges notamment commercialisées par la société ENGELHARD sous la dénomination Orange 363C (Cloisonne) et Orange MCR 101 (Cosmica) et par la société MERCK sous la dénomination Passion orange (Colorona) et Matte orange (17449) (Microna) ; les nacres de teinte brune notamment commercialisées par la société ENGELHARD sous la dénomination Nu-antique copper 340XB (Cloisonne) et Brown CL4509 (Chromalite) ; les nacres à reflet cuivre notamment commercialisées par la société ENGELHARD sous la dénomination Copper 340A (Timica) ; les nacres à reflet rouge notamment commercialisées par la société MERCK sous la dénomination Sienna fine (17386)

(Colorona) ; les nacres à reflet jaune notamment commercialisées par la société ENGELHARD sous la dénomination Yellow (4502) (Chromalite) ; les nacres de teinte rouge à reflet or notamment commercialisées par la société ENGELHARD sous la dénomination Sunstone G012 (Gemtone) ; les nacres roses notamment commercialisées par la société ENGELHARD sous la dénomination Tan opale G005 (Gemtone) ; les nacres noires à reflet or notamment commercialisées par la société ENGELHARD sous la dénomination Nu antique bronze 240 AB (Timica), les nacres bleues notamment commercialisées par la société MERCK sous la dénomination Matte blue (17433) (Microna), les nacres blanches à reflet argenté notamment commercialisées par la société MERCK sous la dénomination Xirona Silver et les nacres orangées rosées vert doré notamment commercialisées par la société MERCK sous la dénomination Indian summer (Xirona) et leurs mélanges.

La composition cosmétique selon l'invention peut comprendre également des colorants hydrosolubles ou liposolubles en une teneur allant de 0,01 à 10 % en poids, notamment allant de 0,01 à 5 % en poids par rapport au poids total de la composition cosmétique. Les colorants liposolubles sont par exemple le rouge Soudan, le DC Red 17, le DC Green 6, le β-carotène, l'huile de soja, le brun Soudan, le DC Yellow 11, le DC Violet 2, le DC orange 5, le jaune quinoléine Les colorants hydrosolubles sont par exemple le jus de betterave, le bleu de méthylène.

La composition cosmétique selon l'invention peut également contenir au moins un matériau à effet optique spécifique.

Cet effet est différent d'un simple effet de teinte conventionnel, c'est-à-dire unifié et stabilisé tel que produit par les matières colorantes classiques comme par exemple les pigments monochromatiques. Au sens de l'invention, « stabilisé » signifie dénué d'effet de variabilité de la couleur avec l'angle d'observation ou encore en réponse à un changement de température.

Par exemple, ce matériau peut être choisi parmi les particules à reflet métallique, les agents de coloration goniochromatiques, les pigments diffractants, les agents thermochromes, les agents azurants optiques, ainsi que les fibres, notamment interférentielles. Bien entendu, ces différents matériaux peuvent être associés de manière à procurer la manifestation simultanée de deux effets, voire d'un nouvel effet conforme à l'invention.

Les particules à reflet métallique utilisables dans l'invention sont en particulier choisies parmi :

- les particules d'au moins un métal et/ou d'au moins un dérivé métallique,
- les particules comportant un substrat, organique ou minéral, monomatière ou multimatériaux, recouvert au moins partiellement par au moins une couche à reflet métallique comprenant au moins un métal et/ou au moins un dérivé métallique, et
- les mélanges desdites particules.

Parmi les métaux pouvant être présents dans lesdites particules, on peut citer par exemple Ag, Au, Cu, Al, Ni, Sn, Mg, Cr, Mo, Ti, Zr, Pt, Va, Rb, W, Zn, Ge, Te, Se et leurs mélanges ou alliages. Ag, Au, Cu, Al, Zn, Ni, Mo, Cr, et leurs mélanges ou alliages (par exemple les bronzes et les laitons) sont des métaux préférés.

Par « dérivés métalliques », on désigne des composés dérivés de métaux notamment des oxydes, des fluorures, des chlorures et des sulfures

A titre illustratif de ces particules, on peut citer des particules d'aluminium, telles que celles commercialisées sous les dénominations STARBRITE 1200 EAC® par la société SIBERLINE et METALURE® par la société ECKART.

On peut également citer les poudres métalliques de cuivre ou des mélanges d'alliage telles les références 2844 commercialisées par la société RADIUM BRONZE, les pigments métalliques comme l'aluminium ou le bronze, telles que celles commercialisées sous les dénominations ROTOSAFE 700 de la société ECKART, les particules d'aluminium enrobé de silice commercialisées sous la dénomination VISIONAIRE BRIGHT SILVER de la société ECKART et les particules d'alliage métallique comme des poudres de bronze (alliage cuivre et zinc) enrobé de silice commercialisées sous la dénomination de Visionaire Bright Natural Gold de la société Eckart.

Il peut s'agir de particules comportant un substrat de verre comme celles commercialisées par la société NIPPON SHEET GLASS sous les dénominations MICROGLASS METASHINE.

L'agent de coloration goniochromatique peut être choisi par exemple parmi les structures multicouches interférentielles et les agents de coloration à cristaux liquides.

Des exemples de structures multicouche interférentielles symétriques utilisables dans des compositions réalisées conformément à l'invention sont par exemple les structures suivantes : $Al/SiO_2/Al/SiO_2/Al$, des pigments ayant cette structure étant commercialisés par la société DUPONT DE NEMOURS ; $Cr/MgF_2/Al/MgF_2/Cr$, des pigments ayant cette structure étant commercialisés sous la dénomination CHROMAFLAIR par la société FLEX ; $MoS_2/SiO_2/Al/SiO_2/MoS_2$ ; $Fe_2O_3/SiO_2/Al/SiO_2/Fe_2O_3$, et $Fe_2O_3/SiO_2/Fe_2O_3/SiO_2/Fe_2O_3$, des pigments ayant ces structures étant commercialisés sous la dénomination SICOPEARL par la société BASF ; $MoS_2/SiO_2$/mica-oxyde/$SiO_2/MoS_2$ ; $Fe_2O_3/SiO_2$/mica-oxyde/$SiO_2/Fe_2O_3$ ; $TiO_2/SiO_2/TiO_2$ et $TiO_2/Al_2O_3/TiO_2$ ; $SnO/TiO_2/SiO_2/TiO_2/SnO$ ; $Fe_2O_3/SiO_2/Fe_2O_3$ ; $SnO$/mica/$TiO_2/SiO_2/TiO_2$/mica/$SnO$, des pigments ayant ces structures étant commercialisés sous la dénomination XIRONA par la société MERCK (Darmstadt). A titre d'exemple, ces

pigments peuvent être les pigments de structure silice/oxyde de titane/oxyde d'étain commercialisés sous le nom XIRONA MAGIC par la société MERCK, les pigments de structure silice/oxyde de fer brun commercialisés sous le nom XIRONA INDIAN SUMMER par la société MERCK et les pigments de structure silice/oxyde de titane/mica/oxyde d'étain commercialisés sous le nom XIRONA CARRIBEAN BLUE par la société MERCK. On peut encore citer les pigments INFINITE COLORS de la société SHISEIDO. Selon l'épaisseur et la nature des différentes couches, on obtient différents effets. Ainsi, avec la structure $Fe_2O_3/SiO_2/Al/\ SiO_2/Fe_2O_3$ on passe du doré-vert au gris-rouge pour des couches de $SiO_2$ de 320 à 350 nm ; du rouge au doré pour des couches de $SiO_2$ de 380 à 400 nm ; du violet au vert pour des couches de $SiO_2$ de 410 à 420 nm ; du cuivre au rouge pour des couches de $SiO_2$ de 430 à 440 nm.

On peut citer, à titre d'exemple de pigments à structure multicouche polymérique, ceux commercialisés par la société 3M sous la dénomination COLOR GLITTER.

Comme particules goniochromatiques à cristaux liquides, on peut utiliser par exemple celles vendues par la société CHENIX ainsi que celle commercialisées sous la dénomination HELICONE® HC par la société WACKER.

## POLYOLS

Selon un mode de réalisation, les compositions cosmétiques conformes à l'invention peuvent également comprendre en outre au moins un polyol ou alcool polyhydrique.

Par "alcool polyhydrique" ou "polyol", il faut comprendre, au sens de la présente invention, toute molécule organique comportant au moins deux groupements hydroxyle libres.

Les alcools polyhydriques convenant avantageusement pour la formulation des compositions cosmétiques selon la présente invention sont ceux présentant notamment de 2 à 20 atomes de carbone, en particulier de 2 à 10 atomes de carbone, et plus particulièrement de 2 à 6 atomes de carbones.

Avantageusement, le polyol peut être par exemple choisi parmi la glycérine, le propylène glycol, le butylène glycol, le pentylène glycol, l'hexylène glycol, le dipropylène glycol, le diéthylène glycol, le sorbitol, l'hydroxypropyl sorbitol, le 1,2,6-hexanetriol ;les éthers de glycol (ayant notamment de 3 à 16 atomes de carbone) tels que les alkyl($C_1$-$C_4$) éther de mono, di- ou tripropylène glycol, les alkyl($C_1$-$C_4$)éthers de mono, di- ou triéthylène glycol ; et leurs mélanges.

## CHARGES

De manière avantageuse, les compositions cosmétiques conformes à l'invention peuvent également comprendre au moins une charge, de nature organique ou minérale, permettant notamment de leur conférer une stabilité améliorée au regard de l'exsudation.

Par « charge », il faut comprendre les particules incolores ou blanches, solides de toutes formes, qui se présentent sous une forme insoluble et dispersée dans le milieu de la composition. De nature minérale ou organique, elles permettent de conférer du corps ou de la rigidité à la composition, et/ou de la douceur, de la matité et de l'uniformité au maquillage.

Les charges utilisées dans les compositions selon la présente invention peuvent être de formes lamellaires, globulaires, sphériques, de fibres ou de toute autre forme intermédiaire entre ces formes définies.

Les charges selon l'invention peuvent être ou non enrobées superficiellement, en particulier elles peuvent être traitées en surface par des silicones, des acides aminés, des dérivés fluorés ou toute autre substance favorisant la dispersion et la compatibilité de la charge dans la composition.

Au sens de la présente invention, les termes « charges minérales » et « charges inorganiques » sont utilisés de manière interchangeable.

Parmi les charges minérales utilisables dans les compositions selon l'invention, on peut citer le talc, le mica, la silice, le siloxysilicate de triméthyle, le kaolin, la bentone, le carbonate de calcium précipité, le carbonate et l'hydrogéno-carbonate de magnésium, l'hydroxyapatite, le nitrure de bore, les microsphères de silice creuses (Silice Beads de Maprecos), les microcapsules de verre ou de céramique, les charges à base de silice comme l'Aerosil 200, l'Aerosil 300 ; le Sunsphare L-31, le Sunphare H-31 commercialisés par Asahi Glass ; le Chemicelen commercialisé par Asahi Chemical ; les composites de silice et de dioxyde de titane comme la série TSG commercialisée par Nippon Sheet Glass, et leurs mélanges.

Parmi les charges organiques utilisables dans les compositions selon l'invention on peut citer les poudres de polyamide (Nylon® Orgasol de chez Atochem), de poly-b-alanine et polyéthylène, les poudres de polytétrafluoroéthylène (Téflon®), la lauroy-lysine, l'amidon, les poudres de polymères de tétrafluoroéthylène, les microsphères creuses de polymères telles l'EXPANCEL (NOBEL INDUSTRIE), le carbonate de calcium précipité, le carbonate et l'hydrogéno-carbonate de magnésium, les savons métalliques dérivés d'acides organiques carboxyliques ayant de 8 à 22 atomes de carbone, de préférence de 12 à 18 atomes de carbone, par exemple le stéarate de zinc, de magnésium ou de lithium, le laurate de zinc, le myristate de magnésium, le Polypore® L 200 (Chemdal Corporation), les microbilles de résine de silicone (Tospearl ® de Toshiba, par exemple), les poudres de poyuréthanne, en particulier les poudres de polyuréthanne

réticulé comprenant un copolymère, ledit copolymère comprenant du triméthylol hexyllactone. En particulier, il peut s'agir d'un polymère d'hexaméthylène di-isocyanate/triméthylol hexyllactone. De telles particules sont notamment disponibles dans le commerce, par exemple sous la dénomination de PLASTIC POWDER D-400® ou PLASTIC POWDER D-800® de la société TOSHIKI, et leurs mélanges.

Les charges peuvent être présentes dans les compositions cosmétiques conformes à l'invention à raison de 0,001 à 35 % du poids total de la composition, de préférence 0,5 à 15 %.

La charge peut être par exemple une charge dont la granulométrie moyenne est inférieure à 100 μm, notamment comprise entre 1 et 50 μm, par exemple entre 4 et 20 μm.

Selon un mode de réalisation particulier, la composition selon l'invention comprend au moins une charge qui présente à raison de 0,01 % à 60 % du poids total de la composition, en particulier de 0,5 à 20 %, plus particulièrement de 1 à 10 % en poids par rapport au poids total de la composition.

## ADDITIFS

Les compositions cosmétiques selon l'invention peuvent également comprendre tout additif usuellement utilisé dans le domaine concerné, choisi parmi des gélifiants, des tensioactifs tels que ceux décrits dans la demande FR 2834452 publiée le 7 juillet 2003, dont le contenu est incorporé dans la présente demande par référence, des antioxydants, des huiles essentielles, des conservateurs, des parfums, des neutralisants, des agents hydratants, des agents antiseptiques, des vitamines telles que la vitamine B3, la vitamine E et leurs dérivés, et des agents protecteurs contre les UV.

Selon un mode particulier de réalisation, les tensioactifs siliconés convenant avantageusement à la mise en oeuvre des compositions conformes à la présente invention sont de type non réticulé.

Selon un mode particulier de réalisation, les compositions conformes à la présente invention sont avantageusement dénuées de tensioactifs appartenant à la famille des tensioactifs de type ammonium.

Bien entendu, l'homme de l'art veillera à choisir le ou les éventuel(s) additif(s) ajoutés à la composition cosmétique selon l'invention, de manière telle que les propriétés avantageuses attachées intrinsèquement à la composition conforme à l'invention ne soit pas, ou substantiellement pas, altérées par l'addition envisagée.

Selon une variante particulière, le polymère de silicone de formule générale (I) utilisé dans les compositions cosmétiques conformes à la présente invention, est choisi parmi la diméthicone de polyglycéryle-3 polydiméthylsiloxyéthyle, la diméthicone de lauryle polyglycéryle-3 polydiméthylsiloxyéthyle, la diméthicone de polyglycéryle-3 disiloxane, et leurs mélanges.

Selon une autre variante, le polymère de silicone de formule générale (I) utilisé dans les compositions cosmétiques conformes à la présente invention, est choisi parmi les polymères de silicone commercialisés par la société SHIN-ETSU sous les références KF 6100®, KF 6104®, KF 6105®, et leurs mélanges.

Selon une autre variante, la composition cosmétique selon l'invention combine la diméthicone de polyglycéryle-3 polydiméthylsiloxyéthyle et, en tant qu'agent filmogène, le copolymère d'acrylate/acrylate de stéaryle/méthacrylate de diméthicone, notamment celui commercialisé sous la référence KP 561® par la société SHIN-ETSU.

Selon encore une autre variante, la composition cosmétique selon l'invention combine la diméthicone de polyglycéryle-3 polydiméthylsiloxyéthyle et au moins une cire, notamment choisie parmi les cires de polyéthylène, la cire de Candelilla, la cire de coco-glycéride hydrogénée, et leurs mélanges.

Selon une variante supplémentaire, la composition cosmétique selon l'invention combine la diméthicone de polyglycéryle-3 polydiméthylsiloxyéthyle et au moins un ester hydrocarboné comprenant moins de 40 atomes de carbone, notamment choisi parmi l'heptanoate de stéaryle, l'isononanoate d'isononyle et l'isononanoate d'isotridécyle, et leurs mélanges.

Il apparaît de manière évidente que certains composants utilisables dans les compositions cosmétiques selon la présente invention, peuvent appartenir simultanément à différentes classes de composés. Par exemple, l'isononanoate d'isononyle est classé, à la fois en tant qu'huile non volatile, et en tant qu'ester hydrocarboné comprenant moins de 40 atomes de carbone.

Ainsi, il ne sort pas du cadre de travail habituel de l'homme du métier d'ajuster la quantité d'un composé appartenant à différentes classes de produit, de manière à ce que sa présence dans la formulation se traduise par l'effet désiré, et correspond, le cas échéant, à l'effet susceptible d'être obtenu par la présence de produits appartenant auxdites différentes classes.

La composition cosmétique selon l'invention peut notamment se présenter sous la forme d'un produit de maquillage des lèvres, en particulier un rouge à lèvres, ou un baume à lèvres.

Les exemples de compositions ci-après sont donnés à titre illustratif et sans caractère limitatif de l'invention.

## Exemple 1 : Rouge à lèvres

On prépare un rouge à lèvres comprenant la diméthicone de polyglycéryl-3 polydiméthylsiloxyéthyle commercialisée sous la référence KF6104® par SHIN-ETSU, et dont la composition est précisée en tableau I.

Tableau I

| | Pourcentages massiques |
|---|---|
| Polyisobutène hydrogéné (Parleam HV de NOF) | 4 |
| Isononanoate d'isononyle | 12 |
| 2-Octyldodécanol | 4,5 |
| Malate de diisostéaryle | 33,9 |
| Triglycérides d'acides lauriques/palmitique/cétylique/stéarique 50/20/10/10 (Softisan 100 ®de Sasol) | 4 |
| N-lauroyl L-lysine | 1 |
| Copolymère d'acrylates/acrylate de stéaryle/méthacrylate de diméthicone (KP 561 P ® de Shin Etsu) | 4 |
| Diméthicone 6 cSt (KF 96) | 4 |
| Diméthicone de polyglycéryl-3 polydiméthylsiloxyéthyle (KF6104 ® de Shin Etsu) | 13 |
| Conservateur | qs |
| Cire de Polyéthylène (PM 500) | 6,6 |
| Cire de polyéthylène (PM 400) | 3,8 |
| Silice pyrogénée hydrophobe traitée en surface par diméthylsilane (Aerosil R 972 ® de Degussa) | 2 |
| Pigments | 7,0 |
| Siméthicone (Antifoam C ®de Dow Corning) | 0,2 |
| Total | 100 |

*Mode opératoire*

Une phase huileuse est préparée en mélangeant à chaud (environ 95 °C) l'isononanoate d'isononyle, le 2-octyldodécanol, le malate de diisostéaryle, avec la diméthicone de polyglycéryl-3 polydiméthylsiloxyéthyle et l'huile diméthicone. La phase huileuse ainsi préparée est maintenue sous agitation à environ 95 °C, et les charges (N-lauroyl L-lysine et silice pyrogénée) sont ajoutées au mélange.

Puis sont rajoutés au mélange les cires, les pigments sous la forme d'une pâte pigmentaire, le polyisobutène hydrogénée et la siméthicone.

Le mélange ainsi obtenu est ensuite coulé dans un moule de rouge à lèvres et laissé refroidir jusqu'à l'obtention d'une composition solide.

Puis on mesure la tenue, le confort et la brillance de cette composition selon les protocoles décrits plus haut dans la description.

On mesure également, selon les mêmes méthodes, la tenue et la brillance de deux produits commerciaux :

- Témoin A : Jelly Plumpy commercialisé par Maybelline contenant de la phényltriméthicone à titre de polymère de silicone,
- Témoin B : Aube Rouge Glacé commercialisé par KAO et contenant la silicone glycérolé de formule suivante, à titre de polymère de silicone

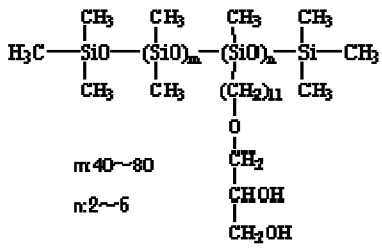

Les résultats obtenus sont reportés dans le tableau II ci-dessous.

Tableau II

| Formule | Tenue à l'eau | Tenue à l'huile | Transfert | Indice de confort | Brillance moyenne |
|---|---|---|---|---|---|
| Témoin A | 14,4 | 23,4 | 47,3 | | 42,5 |
| Témoin B | 7,0 | 8,5 | 42,0 | 85,6 | 48,2 |
| Invention | 5,9 | 7,9 | 32,1 | 99,5 | 48,3 |

Dans ces mesures, la couleur de la face interne de l'avant-bras est telle que L* = 63,9, a* = 8,4, b* = 13,3, et la couleur du mouchoir en papier est L* = 97,9, a* = 0,6 et b* = 3,3.

Le rouge à lèvres conforme à l'invention présente une meilleure tenue et un meilleur confort que les produits commerciaux A et B de l'art antérieur , pour une brillance équivalente voir égale.

En outre, le rouge à lèvres conforme à l'invention migre trois à quatre fois moins que le témoin B de l'art antérieur.

## Exemples 2 et 3: Rouge à lèvres

Les pourcentages sont en poids

| | Exemple 2 | Exemple 3 |
|---|---|---|
| Polyisobutène hydrogéné (Parleam HV de NOF) | | 4 |
| Polyisobutène hydrogéné (Parleam Lite de NOF) | 6 | |
| Isononanoate d'isononyle | 8 | 12 |
| 2-Octyldodécanol | 4,5 | 4,5 |
| Malate de diisostéaryle | 33,9 | 23,9 |
| Polyglycéryl-2-diisostéarate (Cosmol 42 V de Nishin Oil) | | 5 |
| Polyglycéryl-2 triisostéarate (Cosmol 43 N de Nishin Oil) | | 4 |
| Sorbitan Sesquiloléate (Cosmol 82 de Nishin Oil) | | 1 |
| Triglycérides d'acides lauriques/palmitique/cétylique/stéarique 50/20/10/10 (Softisan 100 ®de Sasol) | 3 | 4 |
| N-lauroyl L-lysine | 1 | 1 |
| Polylaurate de vinyle | 3 | |
| Copolymère d'acrylates/acrylate de stéaryle/méthacrylate de diméthicone (KP 561 P ® de Shin Etsu) | 2 | 4 |
| Diméthicone 6 cSt (KF 96) | 5 | 4 |
| Diméthicone de polyglycéryl-3 polydiméthylsiloxyéthyle (KF6104 ® de Shin Etsu) | 13 | 13 |
| Conservateur | qs | |
| Cire de Polyéthylène (PM 500) | 5,5 | 6,6 |
| Cire de polyéthylène (PM 400) | 1,1 | |
| Cire de Candellila | 4,8 | |
| Cire microcristalline | | 3,8 |
| Silice pyrogénée hydrophobe traitée en surface par di-méthylsilane (Aerosil R 972 ® de Degussa) | 2 | 2 |
| Pigments | 7,0 | 7 |
| Siméthicone (Antifoam C ®de Dow Corning) | 0,2 | 0,2 |
| Total | 100 | 100 |

## Revendications

**1.** Composition cosmétique anhydre, **caractérisée en ce qu'**elle comprend, dans un milieu physiologiquement acceptable, au moins un agent filmogène et au moins un polymère de silicone de formule générale (I) :

$$R^1_aR^2_bR^3_c\ SiO_{(4-a-b-c)/2} \qquad (I)$$

dans laquelle :

- a, b et c sont tels que a varie de 1 à 2,5 ; b et c, indépendamment l'un de l'autre, varient de 0,001 à 1,5 ;
- $R^1$, identique ou différent, est choisi parmi :
  - les radicaux alkyle en $C_1$ à $C_{30}$, le cas échéant substitués par un ou plusieurs atomes de fluor, groupements amino et/ou carboxy,
  - les radicaux aryle, aralkyle,
  - les radicaux de formule générale (II) :

    $$-C_dH_{2d}-O-(C_2H_4O)_e(C_3H_6O)_fR^4 \qquad (II)$$

    avec :

    - $R^4$ étant un radical hydrocarboné en $C_1$ à $C_{30}$ ou un radical $R^5$-(CO)- avec $R^5$ étant un radical hydrocarboné en $C_1$ à $C_{30}$, et
    - d, e et f étant des entiers tels que d varie de 0 à 15, e et f, indépendamment l'un de l'autre, varient de 0 à 50, et

  - leurs combinaisons,

- $R^2$ est un radical représenté par la formule générale (III) :

  $$-Q-O-X \qquad (III)$$

  avec :

  - Q étant un radical hydrocarboné bivalent en $C_2$ à $C_{20}$ pouvant inclure au moins une liaison éther et/ou au moins une liaison ester, et
  - X étant un radical hydrocarboné polyhydroxylé,

  sous réserve que lorsque $R^2$ est un groupement de formule générale (IIIA) :

  $$-\ C_3H_6-O[CH_2CH(OH)CH_2O]_nH \qquad (IIIA)$$

  dans laquelle n est un entier variant de 1 à 5, alors $R^1$ n'est pas un radical alkyl en $C_{12}$,
- $R^3$ est un groupement organosiloxane de formule générale (IV) :

  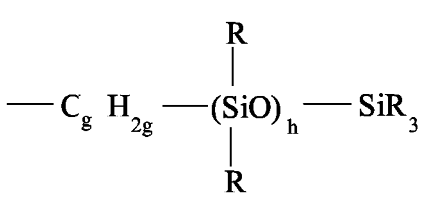 (IV)

  avec :

  - chacun des radicaux R représentant, indépendamment l'un de l'autre, un radical choisi parmi les radicaux alkyle en $C_1$ à $C_{30}$, le cas échéant substitués par un ou plusieurs atomes de fluor ; et les radicaux aryle et aralkyle,

- g et h étant des entiers tels que g varie de 1 à 5 et h varie de 0 à 500.

**2.** Composition cosmétique anhydre, **caractérisée en ce qu'**elle comprend, dans un milieu physiologiquement acceptable, au moins un agent filmogène et au moins un polymère de silicone de formule générale (I) :

$$R^1_a\ R^2_b\ R^3_c\ SiO_{(4-a-b-c)/2} \qquad (I)$$

dans laquelle :

- a, b et c sont tels que a varie de 1 à 2,5, b et c, indépendamment l'un de l'autre, varient de 0,001 à 1,5,
- $R^1$ identique ou différent, est choisi parmi :

  - les radicaux alkyle en $C_1$ à $C_{30}$, le cas échéant substitués par un ou plusieurs atomes de fluor, groupements amino et/ou carboxy,
  - les radicaux aryle, aralkyle,
  - les radicaux de formule générale (II) :

$$—C_dH_{2d}\text{-O-}(C_2H_4O)_e(C_3H_6O)_fR^4 \qquad (II)$$

  avec :

    - $R^4$ étant un radical hydrocarboné en $C_1$ à $C_{30}$ ou un radical $R^5$-(CO)- avec $R^5$ étant un radical hydrocarboné en $C_1$-$C_{30}$, et
    - d, e et f sont des entiers tels que d varie de 0 à 15, e et f, indépendamment l'un de l'autre, varient de 0 à 50, et

  - leurs combinaisons,

- $R^2$ est un radical représenté par la formule générale (III) :

$$\text{-Q-O-X} \qquad (III)$$

avec :

  - Q étant un radical hydrocarboné bivalent en $C_2$ à $C_{20}$ pouvant inclure au moins une liaison éther et/ou au moins une liaison ester et
  - X étant un radical hydrocarboné polyhydroxylé,

- $R^3$ est un organosiloxane de formule générale (IV) :

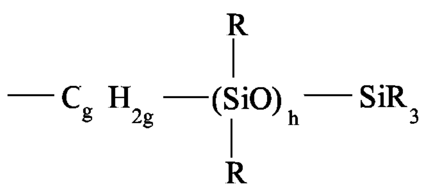

(IV)

avec :

  - chacun des radicaux R représentant, indépendamment l'un de l'autre, un radical choisi parmi les radicaux alkyle en $C_1$ à $C_{30}$, le cas échéant substitué par un ou plusieurs atomes de fluor ; et les radicaux aryle, aralkyle,
  - g et h étant des entiers tels que g varie de 1 à 5 et h varie de 0 à 500,

ledit agent filmogène étant présent dans un rapport pondéral, par rapport au poids du polymère de silicone de formule générale (I), inférieur à 5:1, en particulier inférieur à 1:1, et plus particulièrement inférieur ou égal à 1:2.

**3.** Composition cosmétique anhydre, **caractérisée en ce qu'**elle comprend, dans un milieu physiologiquement acceptable au moins un agent filmogène non cristallin, au moins un composé siliconé distinct de l'agent filmogène et au moins un polymère de silicone de formule générale (I) :

$$R^1_aR^2_bR^3_c\ SiO_{(4-a-b-c)/2} \qquad (I)$$

dans laquelle :

- a, b et c sont tels que a varie de 1 à 2,5, b et c, indépendamment l'un de l'autre, varient de 0,001 à 1,5,
- $R^1$ identique ou différent, est choisi parmi :
  - les radicaux alkyle en $C_1$ à $C_{30}$, le cas échéant substitués par un ou plusieurs atomes de fluor, groupements amino et/ou carboxylique,
  - les radicaux aryle, aralkyle,
  - les radicaux de formule générale (II) :

$$—C_dH_{2d}\text{-O-}(C_2H_4O)_e(C_3H_6O)_fR^4 \qquad (II)$$

  avec :

    - $R^4$ étant un radical hydrocarboné en $C_1$ à $C_{30}$ ou un radical $R^5$-(CO)- avec $R^5$ étant un radical hydrocarboné en $C_1$ à $C_{30}$, et
    - d, e et f sont des entiers tels que d varie de 0 à 15, e et f, indépendamment l'un de l'autre, varient de 0 à 50, et

  - leurs combinaisons,

- $R^2$ est un radical de formule générale (III) :

$$\text{-Q-O-X} \qquad (III)$$

avec :

  - Q étant un radical hydrocarboné bivalent en $C_2$ à $C_{20}$ pouvant inclure au moins une liaison éther et/ou au moins une liaison ester,
  - X étant un radical hydrocarboné polyhydroxylé, et

- $R^3$ est un organosiloxane de formule générale (IV) :

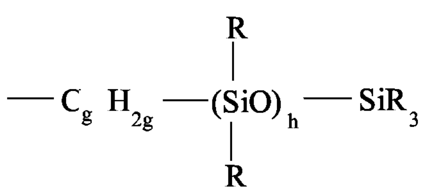

(IV)

avec :

  - chacun des radicaux R représentant, indépendamment l'un de l'autre, un radical choisi parmi les radicaux alkyle en $C_1$-$C_{30}$, le cas échéant substitué par un ou plusieurs atomes de fluor ; et les radicaux aryle, aralkyle,
  - g et h étant des entiers tels que g varie de 1 à 5 et h varie de 0 à 500.

**4.** Composition cosmétique de maquillage des lèvres, **caractérisée en ce qu'**elle comprend, dans un milieu physiologiquement acceptable, au moins un agent filmogène et au moins un polymère de silicone de formule générale (I) :

$$R^1_aR^2_bR^3_c\ SiO_{(4-a-b-c)/2} \qquad (I)$$

avec :

- a, b et c sont tels que a varie de 1 à 2,5, b et c, indépendamment l'un de l'autre, varient de 0,001 à 1,5,
- $R^1$ identique ou différent, est choisi parmi :

  - les radicaux alkyle en $C_1$ à $C_{30}$, le cas échéant substitués par un ou plusieurs atomes de fluor, groupements amino et/ou carboxylique,
  - les radicaux aryle, aralkyle,
  - les radicaux de formule générale (II) :

$$— C_dH_{2d}\text{-O-}(C_2H_4O)_e(C_3H_6O)_fR^4 \qquad \text{(II)}$$

  avec :

    - $R^4$ étant un radical hydrocarboné en $C_1$ à $C_{30}$ ou $R^5$-(CO)-avec $R^5$ étant un radical en $C_1$ à $C_{30}$,
    - d, e et f étant des entiers tels que d varie de 0 à 15, e et f, indépendamment l'un de l'autre, varient de 0 à 50, et

  - leurs combinaisons,

- $R^2$ étant un radical de formule générale (III) :

$$\text{-Q-O-X} \qquad \text{(III)}$$

avec :

  - Q étant un radical hydrocarboné bivalent de $C_2$ à $C_{20}$ pouvant inclure au moins une liaison éther et/ou au moins une liaison ester, et
  - X étant un radical hydrocarboné polyhydroxylé,

- $R^3$ est un organosiloxane de formule générale (IV) :

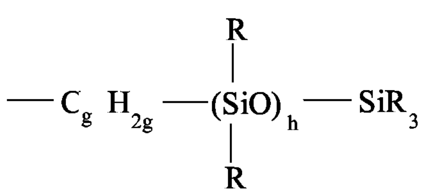

(IV)

avec :

  - chacun des groupes R représentant, indépendamment l'un de l'autre, un radical choisi parmi les radicaux alkyle en $C_1$ à $C_{30}$, le cas échéant substitué par un ou plusieurs atomes de fluor, les radicaux aryle, aralkyle,
  - g et h étant des entiers tels que g varie de 1 à 5 et h varie de 0 à 500,

ledit agent filmogène étant choisi parmi les agents filmogènes non siliconés et les agents filmogènes siliconés dépourvus de motif acrylique.

**5.** Composition cosmétique, **caractérisée en ce qu'**elle comprend, dans un milieu physiologiquement acceptable, au moins un ester hydrocarboné comprenant moins de 40 atomes de carbone, au moins un agent filmogène et au moins un polymère de silicone de formule générale (I) :

$$R^1_aR^2_bR^3_c\ SiO_{(4-a-b-c)/2} \qquad \text{(I)}$$

avec :

- a, b et c sont tels que a varie de 1 à 2,5, b et c, indépendamment l'un de l'autre, varient de 0,001 à 1,5,

- $R^1$ identique ou différent, est choisi parmi :
    - les radicaux alkyle en $C_1$ à $C_{30}$, le cas échéant substitués par un ou plusieurs atomes de fluor, groupements amino et/ou carboxylique,
    - les radicaux aryle, aralkyle,
    - les radicaux de formule générale (II) :

$$—C_dH_{2d}\text{-O-}(C_2H_4O)_e(C_3H_6O)_fR^4 \qquad \text{(II)}$$

    avec :

    - $R^4$ étant un radical hydrocarboné en $C_1$ à $C_{30}$ ou $R^5$-(CO)-avec $R^5$ étant un radical en $C_1$ à $C_{30}$,
    - d et f étant des entiers tels que d varie de 0 à 15, e et f, indépendamment l'un de l'autre, varient de 0 à 50, et

    - leurs combinaisons,

- $R^2$ étant un radical de formule générale (III) :

$$\text{-Q-O-X} \qquad \text{(III)}$$

avec :

- Q étant un radical hydrocarboné bivalent de $C_2$ à $C_{20}$ pouvant inclure au moins une liaison éther et/ou au moins une liaison ester, et
- X étant un radical hydrocarboné polyhydroxylé,

- $R^3$ est un organosiloxane de formule générale (IV) :

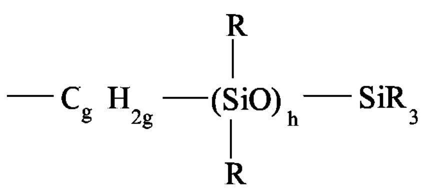

(IV)

avec :

- chacun des groupes R représentant, indépendamment l'un de l'autre, un radical choisi parmi les radicaux alkyle en $C_1$ à $C_{30}$, le cas échéant substitué par un ou plusieurs atomes de fluor ; et les radicaux aryle, aralkyle,
- g et h étant des entiers tels que g varie de 1 à 5 et h varie de 0 à 500,

ledit agent filmogène étant choisi parmi les agents filmogènes non siliconés et les agents filmogènes siliconés dépourvus de motif acrylique.

**6.** Composition selon l'une quelconque des revendications précédentes, **caractérisée en ce que** le polymère de silicone est un composé de formule générale (I) dans laquelle $R^1$ est un radical alkyle en $C_1$ à $C_{10}$, en particulier en $C_1$ à $C_6$, et plus particulièrement en $C_1$ à $C_4$.

**7.** Composition selon l'une quelconque des revendications précédentes, **caractérisée en ce que** le polymère de silicone est un composé de formule générale (I) dans laquelle :

- a varie de 1 à 1,4, b et c, indépendamment l'un de l'autre, varient de 0,02 à 0,04, et
- $R^1$ est un radical alkyle en $C_1$ à $C_{10}$, en particulier en $C_1$ à $C_6$, et plus particulièrement en $C_1$ à $C_4$,
- $R^2$ est représenté par la formule (IIIA) :

$$-C_3H_6O[CH_2CH(OH)CH_2O]_nH \quad \text{(IIIA)}$$

dans laquelle n varie de 1 à 5,
- $R^3$ est représenté par la formule (IVA) :

$$— C_2H_4(CH_3)_2SiO[(CH_3)_2SiO]_mSi(CH_3)_3 \quad \text{(IVA)}$$

dans laquelle m varie de 3 à 9.

**8.** Composition selon la revendication précédente, **caractérisée en ce que** le polymère de silicone est un composé de formule générale (I) :

$$R^1{}_aR^2{}_bR^3{}_c\ SiO_{(4-a-b-c)/2} \quad \text{(I)}$$

dans laquelle :

- a varie de 1 à 1,4, b et c, indépendamment l'un de l'autre, varient de 0,02 à 0,04, et
- $R^1$ est un radical méthyle,
- $R^2$ est représenté par la formule (IIIA), dans laquelle n varie de 1 à 5, et
- $R^3$ est représenté par la formule (IVA), dans laquelle m varie de 3 à 9.

**9.** Composition selon l'une quelconque des revendications précédentes **caractérisée en ce que** ledit agent filmogène est choisi parmi les polymères radicalaires, les polycondensats et les polymères d'origine naturelle, en particulier les polymères acryliques, les polyuréthanes, les polyesters, les polyamides, les polyurées, les polymères cellulosiques comme la nitrocellulose.

**10.** Composition selon l'une quelconque des revendications 1, et 3 à 9, **caractérisée en ce que** ledit agent filmogène est présent dans un rapport pondéral, par rapport au poids du polymère de silicone de formule générale (I), inférieur à 5 : 1, en particulier inférieur à 1 : 1, et plus particulièrement inférieur ou égal à 1 : 2.

**11.** Composition selon l'une quelconque des revendications 1, 2 et 4 à 10, **caractérisée en ce que** ledit agent filmogène est un agent filmogène non cristallin.

**12.** Composition selon l'une quelconque des revendications 1 à 3, 6 et 10 **caractérisée en ce que** ledit agent filmogène est un agent filmogène non siliconé.

**13.** Composition selon l'une quelconque des revendications 1 à 3, 6 et 10, **caractérisée en ce que** ledit agent filmogène est un agent filmogène siliconé dépourvu de motif acrylique.

**14.** Composition selon l'une quelconque des revendications 1 à 4 et 6 à 13, **caractérisée en ce que** ladite composition comprend en outre au moins un ester hydrocarboné comprenant moins de 40 atomes de carbone.

**15.** Composition selon la revendication 5 ou 14, **caractérisée en ce que** ledit ester hydrocarboné est un monoester.

**16.** Composition selon l'une quelconque des revendications 5, 14 ou 15, **caractérisée en ce que** ledit ester hydrocarboné répond à la formule RCOOR' dans laquelle RCOO représente un reste d'acide gras comportant de 2 à 28 atomes de carbone et R' représente une chaîne hydrocarbonée contenant de 1 à 28 atomes de carbone.

**17.** Composition selon l'une quelconque des revendications 5, 14 à 16, **caractérisée en ce que** ledit ester hydrocarboné comprend moins de 22 atomes de carbone.

**18.** Composition selon l'une quelconque des revendications 5, 14 à 17, **caractérisée en ce que** ledit ester hydrocarboné est choisi parmi les esters de synthèse notamment d'acide gras comme l'huile de purcellin, le myristate d'isopropyle, le palmitate d'éthyle, le stéarate d'octyle ; les esters hydroxylés comme le lactate d'isostéaryle, l'hydroxystéarate d'octyle, l'adipate de diisopropyle, les heptanoates, octanoates, décanoates d'alcool gras, les esters de l'acide néopentanoïque comme le néopentanoate d'isodécyle, le néopentanoate d'isotridécyle, le néopentanoate d'isostéaryle, le néopentanoate d'octyldocécyle, les esters de l'acide isononanoïque comme l'isononanoate d'isononyle, l'isononanoate d'octyle, l'isononanoate d'isodécyle, l'isononanoate d'isotridécyle, l'isononanoate d'isostéaryle, les

esters de l'alcool isopropylique, tels que le myristate d'isopropyle, le palmitate d'isopropyle, le stéarate ou l'isostéarate d'isopropyle, l'octanoate de cétyle, l'octanoate de tridécyle, le 4-diheptanoate et le palmitate d'éthyl-2-hexyle, le benzoate d'alkyle, le diheptanoate de polyéthylène glycol, le diéthyl 2-d'hexanoate de propylèneglycol et leurs mélanges.

**19.** Composition selon l'une des revendications précédentes, **caractérisée en ce qu'**elle comprend au moins une huile hydrocarbonée, choisie parmi le polyisobutène hydrogéné, l'heptanoate d'isostéaryle, l'isononanoate d'isononyle, le tétrahydroxystéarate/tétraisostéarate de dipentaérythritole, le 2-octyldodécanol, l'isononanoate d'isotridécyle, le malate de diisostéaryle, et leurs mélanges.

**20.** Composition selon l'une quelconque des revendications 1, 2, et 4 à 19, **caractérisée en ce qu'**elle comprend en outre au moins un composé siliconé.

**21.** Composition selon la revendication 3 ou 20, **caractérisée en ce que** ledit composé siliconé est choisi parmi les huiles de silicone, les gommes siliconées, les résines de silicone les élastomères de silicone, et leurs mélanges.

**22.** Composition selon la revendication précédente, **caractérisée en ce que** ledit composé siliconé est en particulier un copolymère d'acrylate/acrylate de stéaryle/méthacrylate de silicone.

**23.** Composition selon la revendication 21, **caractérisée en ce que** ladite huile siliconée est choisi parmi :

- les huiles de silicones linéaires ou cycliques, notamment celles ayant une viscosité $\leq$ 8 centistokes ($8\ 10^{-6}$ $m^2/s$) et en particulier l'octaméthyl cyclotétrasiloxane, le décaméthyl cyclopentasiloxane, le dodécaméthyl cyclohexasiloxane, l'heptaméthyl hexyltrisiloxane, l'heptaméthyloctyl trisiloxane, l'hexaméthyl disiloxane, l'octaméthyl trisiloxane, le décaméthyl tétrasiloxane, le dodécaméthyl pentasiloxane, et
- les huiles de silicone, telles que les polydiméthylsiloxanes (PDMS) non volatiles, les polydiméthylsiloxanes comportant des groupements alkyle ou alcoxy, pendant et/ou en bout de chaîne siliconée, groupements ayant chacun de 2 à 24 atomes de carbone, les silicones phénylées comme les phényl triméthicones, les phényl diméthicones, les phényl triméthylsiloxy diphénylsiloxanes, les diphényl diméthicones, les diphényl méthyldiphényl trisiloxanes, les 2-phényléthyl triméthylsiloxysilicates, et
- leurs mélanges.

**24.** Composition selon l'une quelconque des revendications précédentes, **caractérisée en ce qu'**elle est sous forme coulée.

**25.** Composition selon l'une quelconque des revendications précédentes, **caractérisée en ce que** ladite composition comprend en outre au moins une matière colorante.

**26.** Composition selon l'une quelconque des revendications précédentes, **caractérisée en ce qu'**elle comprend en outre au moins un composé choisi parmi les cires, les corps gras pâteux, et leurs mélanges.

**27.** Composition selon l'une quelconque des revendications précédentes, **caractérisée en ce qu'**elle comprend en outre au moins une charge.

**28.** Composition selon l'une quelconque des revendications précédentes, **caractérisée en ce qu'**il s'agit d'une composition de maquillage et/ou de soin des lèvres et/ou de la peau, en particulier d'un rouge à lèvres.

**29.** Utilisation d'au moins un polymère de silicone de formule générale (I), telle que définie selon l'une quelconques des revendications 2, 6, 7 ou 8, en association avec au moins un agent filmogène pour la préparation d'une composition cosmétique présentant une tenue améliorée.

**30.** Utilisation selon la revendication précédente **caractérisée en ce que** ledit agent filmogène est tel que défini selon les revendications 9 à 13.

**31.** Utilisation selon la revendication 29 ou 30 **caractérisée en ce que** ledit polymère de silicone de formule générale (I) est associé en outre à au moins un composé siliconé.